# EUROPEAN PATENT SPECIFICATION

(11) **EP 2 396 331 B1**
(45) Date of publication and mention of the grant of the patent: **16.10.2013**
(21) Application number: 10703022.3
(22) Date of filing: 05.02.2010
(51) Int. Cl.: C07D 487/04, A61P 35/00, A61K 31/517

(54) **FUSED PYRIMIDINES AS AKT INHIBITORS**
KONDENSIERTE PYRIMIDINE ALS AKT-INHIBITOREN
PYRIMIDINES CONDENSÉES EN TANT QU'INHIBITEURS D'AKT

(30) Priority: 13.02.2009 EP 09152805
(43) Date of publication of application: 21.12.2011
(73) Proprietor: Bayer Intellectual Property GmbH, 40789 Monheim (DE)
(72) Inventor: VENNEMANN, Matthias, 78462 Konstanz (DE); BÄR, Thomas, 78479 Reichenau (DE); MAIER, Thomas, 78333 Stockach (DE); HÖLDER, Swen, London SE26 6UR (GB); BENEKE, Gerrit, 78476 Allensbach (DE); DEHMEL, Florian, 52066 Aachen (DE); ZÜLCH, Armin, 69198 Schriesheim (DE); STRUB, Andreas, 82152 Planegg (DE); BECKERS, Thomas, 79104 Freiburg i.Br. (DE); INCE, Stuart, 14129 Berlin (DE); REHWINKEL, Hartmut, 10961 Berlin (DE); LIU, Ningshu, 12203 Berlin (DE); BÖMER, Ulf, 16548 Glienicke/Nordbahn (DE)
(74) Representative: BIP Patents
(86) International application number: PCT/EP2010/000717
(87) International publication number: WO 2010/091824

(56) References cited:
- WO-A2-03/089434
- WO-A2-2004/096131
- WO-A2-2006/065601
- WU Z ET AL: "Rapid assembly of diverse and potent allosteric Akt inhibitors" BIOORGANIC & MEDICINAL CHEMISTRY LETTERS, PERGAMON, ELSEVIER SCIENCE, GB LNKD- DOI:10.1016/J.BMCL.2007.10.023, vol. 18, no. 6, 15 March 2008 (2008-03-15) , pages 2211-2214, XP025695048 ISSN: 0960-894X [retrieved on 2007-10-17]

## Description

### Field of application of the invention

The invention relates to fused Pyrimidine compounds, which are used in the pharmaceutical industry for the manufacture of pharmaceutical compositions.

### Known technical background

Cancer is the second most prevalent cause of death in the United States, causing 450,000 deaths per year. While substantial progress has been made in identifying some of the likely environmental and hereditary causes of cancer, there is a need for additional therapeutic modalities that target cancer and related diseases. In particular there is a need for therapeutic methods for treating diseases associated with dysregulated growth / proliferation.

Cancer is a complex disease arising after a selection process for cells with acquired functional capabilities like enhanced survival / resistance towards apoptosis and a limitless proliferative potential. Thus, it is preferred to develop drugs for cancer therapy addressing distinct features of established tumors.

One pathway that has been shown to mediate important survival signals for mammalian cells comprises receptor tyrosine kinases like platelet-derived growth factor receptor (PDGF-R), human epidermal growth factor 2/3 receptor (HER2/3), or the insulin-like growth factor 1 receptor (IGF-1R). After activation the respectives by ligand, these receptors activate the phoshatidylinositol 3-kinase (Pi3K)/Akt pathway. The phoshatidylinositol 3-kinase (Pi3K)/Akt protein kinase pathway is central to the control of cell growth, proliferation and survival, driving progression of tumors. Therefore within the class of serine-threonine specific signalling kinases, Akt (protein kinase B; PKB) with the isoenzmyes Akt1 (PKBα), Akt2 (PKB ß) and Akt3 (PKB γ) is of high interest for therapeutic intervention. Akt is mainly activated in a Pi3-kinase dependent manner and the activation is regulated through the tumor suppressor PTEN (phosphatase and tensin homolog), which works essentially as the functional antagonist of Pi3K.

The Pi3K/Akt pathway regulates fundamental cellular functions (e.g. transcription, translation, growth and survival), and is implicated in human diseases including diabetes and cancer. The pathway is frequently overactivated in a wide range of tumor entities like breast and prostate carcinomas. Upregulation can be due to overexpression or constitutively activation of receptor tyrosine kinases (e.g. EGFR, HER2/3), which are upstream and involved in its direct activation, or gain- or loss-of-function mutants of some of the components like loss of PTEN. The pathway is targeted by genomic alterations including mutation, amplification and rearrangement more frequently than any other pathway in human cancer, with the possible exception of the p53 and retinoblastoma pathways. The alterations of the Pi3K/Akt pathway trigger a cascade of biological events, that drive tumor progression, survival, angiogenesis and metastasis.

Activation of Akt kinases promotes increased nutrient uptake, converting cells to a glucose-dependent metabolism that redirects lipid precursors and amino acids to anabolic processes that support cell growth and proliferation. These metabolic phenotype with overactivated Akt lead to malignancies that display a metabolic conversion to aerobic glycolysis (the Warburg effect). In that respect the Pi3K/Akt pathway is discussed to be central for survival despite unfavourable growth conditions such as glucose depletion or hypoxia.

A further aspect of the activated PI3K/Akt pathway is to protect cells from programmed cell death ("apoptosis") and is hence considered to transduce a survival signal. By acting as a modulator of anti-apoptotic signalling in tumor cells, the Pi3K/Akt pathway, particular Akt itself is a target for cancer therapy. Activated Akt phosphorylates and regulates several targets, e.g. BAD, GSK3 or FKHRL1, that affect different signalling pathways like cell survival, protein synthesis or cell movement. This Pi3K/Akt pathway also plays a major part in resistance of tumor cells to conventional anti-cancer therapies. Blocking the Pi3K/Akt pathway could therefore simultaneously inhibit the proliferation of tumor cells (e.g. via the inhibition of the metabolic effect) and sensitize towards pro-apoptotic agents.

Akt inhibition selectively sensitized tumor cells to apoptotic stimuli like Trail, Campthothecin and Doxorubicin. Dependent on the genetic background / molecular apperations of tumors, Akt inhibitors might induce apoptotic cell death in monotherapy as well.

In the International patent applications WO2004096131, W02005100344, W02006036395, W02006065601, W02006091395 and W02006135627 Akt inhibitors are described. In a recent disclosure, Y. Li et al (Bioorg. Med. Chem. Lett. 2009, 19, 834-836 and cited references therein) detail the difficulty in finding optimal Akt inhibitors. The potential application of Akt inhibitors in multiple disease settings, such as for example, cancer, makes the provision of new, alternative Akt inhibitors to those currently available highly desirable.

### Description of the invention

A solution to the above problem is the provision of alternative Akt inhibitors. It has now been found that the new fused pyrimidine compounds, which are described in detail below, have activity as AKT inhibitors.

In accordance with a first aspect, the invention relates to compounds of formula (I) 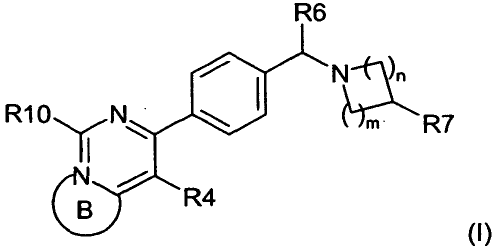 wherein ring B fused with the pyrimidine moiety is selected from 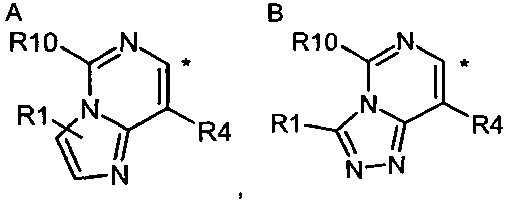
- *: marks the point of attachment
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, 2-4C-alkenyl, 2-4C-alkynyl, C(O)NR11R12, -C(O)OR2, or a monocyclic 5- or 6-membered heteroarylene comprising 1 nitrogen atom and optionally 1, 2 or 3 further heteroatoms independently selected from oxygen, nitrogen and sulfur,
- R2: is hydrogen, 1-4C-alkyl (optionally substituted by halogen, hydroxyl, amino, mono- or di-1-4C-alkylamino) or 3-7C-cycloalkyl,
- R4: is phenyl, thienyl, pyridinyl, oxazolyl or thiazolyl and wherein R4 is option-ally substituted by R5,
- R5: is 1-4C-alkyl, halogen or 1-4C-alkoxy or NR11R12,
- R6: is hydrogen or 1-4C-alkyl,
- n: is 1 or 2,
- m: is 1 or 2,
- R7: is -W-Y,
- W: is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1, 2 or 3 further heteroatoms independently selected from oxygen, nitrogen and sulphur and wherein the bicyclic heteroarylene is optionally substituted by R8,
- R8: hydrogen, is 1-4C-alkyl, 1-4C-haloalkyl, 3-7C-cycloalkyl, 1-4Calkoxy, halogen, cyano or NR11 R12,
- Y: is hydrogen, aryl or a monocyclic 5- or 6-membered heteroaryl comprising 1 nitrogen atom and optionally 1,2 or 3 further heteroatoms independently selected from oxygen, nitrogen, sulphur and wherein the heteroaryl is optionally substituted by R9 and optionally further substituted by R9A,
- R9: is 1-4C-alkyl, 1-4C-alkoxy, halogen, hydroxy, 1-4C-haloalkyl, NR11R12, cyano or C(O)NH2,
- R9A: is 1-4Calkyl or halogen
- R10: is hydrogen or 1-4C-alkyl (optionally substituted by halogen, hydroxy, amino, mono-or di1-4C-alkylamino),
- R11, R12: which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

Another aspect of the invention relates to compounds according to claim 1, wherein
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, C(O)NR11R12, -C(O)OR2,
- R2: is hydrogen or 1-4C-alkyl,
- R4: is phenyl, thienyl, pyridinyl, oxazolyl or thiazolyl and wherein R4 is optionally substituted by R5,
- R5: is 1-4C-alkyl, halogen or 1-4C-alkoxy or NR11R12,
- R6: is hydrogen or 1-4C-alkyl,
- n: is 1 or 2,
- m: is 1 or 2,
- R7: is -W-Y,
- W: is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1, 2 or 3 further heteroatoms independently selected from oxygen, nitrogen and sulphur and wherein the bicyclic heteroarylene is optionally substituted by R8,
- R8: hydrogen, is 1-4C-alkyl, 1-4C-haloalkyl, 3-7C-cycloalkyl, 1-4Calkoxy, halogen, cyano or NR11R12,
- Y: is hydrogen, aryl or a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9 and optionally further substituted by R9A,
- R9: is 1-4C-alkyl, 1-4C-alkoxy, halogen, hydroxy, 1-4C-haloalkyl, NR11R12, cyano or C(O)NH2,
- R9A: is 1-4Calkyl or halogen
- R10: is hydrogen or 1-4C-alkyl (optionally substituted by halogen, hydroxy, amino, mono-or di1-4C-alkylamino),
- R11, R12: which can be same or different is hydrogen or 1-4C-alkyl,
or an N-oxide or a salt, a tautomer, or a stereoisomer of said compound, or a salt, of said N-oxide, tautomer or stereoisomer.

A further aspect of the invention relates to compounds according to claim 1, wherein
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, C(O)NR11R12, -C(O)OR2,
- R2: is hydrogen or 1-4C-alkyl,
- R4: is phenyl, thienyl, pyridinyl, oxazolyl or thiazolyl,
- R6: is hydrogen or 1-4C-alkyl,
- n: is 1 or 2,
- m: is 1 or 2,
- R7: is -W-Y,
- W: is 1,2,4-triazolylene,
- Y: is a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9 and optionally further substituted by R9A,
- R9: is 1-4C-alkyl, 1-4C-alkoxy or halogen, hydroxy, 1-4C-haloalkyl, NR11R12, cyano or C(O)NH2,
- R9A: is 1-4Calkyl
- R10: is hydrogen or 1-4C-alkyl,
- R11, R12: which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

Another aspect of the invention relates to compounds of general formula (I) according to claim 1, wherein
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, 2-4C-alkenyl, 2-4C-alkynyl, C(O)NR11R12, -C(O)OR2, or a monocyclic 5- or 6-membered heteroarylene comprising 1 nitrogen atom and optionally 1, 2 or 3 further heteroatoms independently selected from oxygen, nitrogen and sulfur,
- R2: is hydrogen, 1-4C-alkyl (optionally substituted by halogen, hydroxyl, amino, mono- or di-1-4C-alkylamino) or 3-7C-cycloalkyl,
- R4: is phenyl, thienyl, pyridinyl, oxazolyl or thiazolyl and wherein R4 is optionally substituted by R5,
- R5: is 1-4C-alkyl, halogen or 1-4C-alkoxy or NR11R12,
- R6: is hydrogen or 1-4C-alkyl,
- n: is 1 or 2,
- m: is 1 or 2,
- R7: is -W-Y,
- W: is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1, 2 or 3 further heteroatoms independently selected from oxygen, nitrogen and sulphur and wherein the bicyclic heteroarylene is optionally substituted by R8,
- R8: hydrogen, is 1-4C-alkyl, 1-4C-haloalkyl, 3-7C-cycloalkyl, 1-4Calkoxy, halogen, cyano or NR11R12,
- Y: is hydrogen, aryl or a monocyclic 5- or 6-membered heteroaryl comprising 1 nitrogen atom and optionally 1,2 or 3 further heteroatoms independently selected from oxygen, nitrogen, sulphur and wherein the heteroaryl is optionally substituted by R9,
- R9: is 1-4C-alkyl, 1-4C-alkoxy, halogen, hydroxy, 1-4C-haloalkyl, NR11R12, cyano or C(O)NH2,
- R10: is hydrogen or 1-4C-alkyl (optionally substituted by halogen, hydroxy, amino, mono-or di1-4C-alkylamino),
- R11, R12: which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

A further aspect of the invention relates to compounds of general formula (I) according to claim 1, wherein
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, C(O)NR11R12, -C(O)OR2,
- R2: is hydrogen or 1-4C-alkyl,
- R4: is phenyl, thienyl, pyridinyl, oxazolyl or thiazolyl and wherein R4 is optionally substituted by R5,
- R5: is 1-4C-alkyl, halogen or 1-4C-alkoxy or NR11R12,
- R6: is hydrogen or 1-4C-alkyl,
- n: is 1 or 2,
- m: is 1 or 2,
- R7: is -W-Y,
- W: is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1, 2 or 3 further heteroatoms independently selected from oxygen, nitrogen and sulphur and wherein the bicyclic heteroarylene is optionally substituted by R8,
- R8: hydrogen, is 1-4C-alkyl, 1-4C-haloalkyl, 3-7C-cycloalkyl, 1-4Calkoxy, halogen, cyano or NR11R12,
- Y: is hydrogen, aryl or a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9,
- R9: is 1-4C-alkyl, 1-4C-alkoxy, halogen, hydroxy, 1-4C-haloalkyl, NR11R12, cyano or C(O)NH2,
- R10: is hydrogen or 1-4C-alkyl (optionally substituted by halogen, hydroxy, amino, mono-or di1-4C-alkylamino),
- R11, R12: which can be same or different is hydrogen or 1-4C-alkyl,
or an N-oxide or a salt, a tautomer, or a stereoisomer of said compound, or a salt, of said N-oxide, tautomer or stereoisomer.

Another aspect of the invention relates to compounds of general formula (I) according to claim 1, wherein
- R1: is hydrogen, 1-4C-alkyl (optionally substituted by halogen, hydroxy, amino, mono-or di1-4C-alkylamino), halogen, trifluoromethyl, cyano, 3-7C-cycloalkyl, C(O)NR11R12, -C(O)OR2,
- R2: is hydrogen or 1-4C-alkyl,
- R4: is phenyl, thienyl, pyridinyl, oxazolyl or thiazolyl,
- R6: is hydrogen or 1-4C-alkyl,
- n: is 1 or 2,
- m: is 1 or 2,
- R7: is -W-Y,
- W: is 1,2,4-triazolylene,
- Y: is a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9,
- R9: is 1-4C-alkyl, 1-4C-alkoxy or halogen, hydroxy, 1-4C-haloalkyl, NR11R12, cyano or C(O)NH2,
- R10: is hydrogen or 1-4C-alkyl,
- R11, R12: which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

Another aspect of the invention relates to compounds according to claim 1, wherein
- R1: is hydrogen or 1-4C-alkyl
- R4: is phenyl,
- R6: is hydrogen,
- n: is 1 or 2,
- m: is 1 or 2,
- R7: is -W-Y,
- W: is 1,2,4-triazolylene,
- Y: is pyridin-2-yl, 2-pyrazinyl or 2-pyrimidinyl which is optionally substituted by R9 and optionally further substituted by R9A,
- R9: is hydrogen, 1-4C-alkyl, halogen, 1-4C-haloalkyl
- R9A: is 1-4Calkyl
- R10: is hydrogen or 1-4C-alkyl,
or an N-oxide or a salt, a tautomer, or a stereoisomer of said compound, or a salt, of said N-oxide, tautomer or stereoisomer.

A further aspect of the invention relates to compounds of general formula (I) according to claim 1, wherein
- R1: is hydrogen, 1-4C-alkyl
- R4: is phenyl,
- R6: is hydrogen,
- n: is 1 or 2,
- m: is 1 or 2,
- R7: is -W-Y,
- W: is 1,2,4-triazolylene,
- Y: is pyridin-2-yl,
- R10: is hydrogen or 1-4C-alkyl,
or an N-oxide or a salt, a tautomer, or a stereoisomer of said compound, or a salt, of said N-oxide, tautomer or stereoisomer.

Another aspect of the invention relates to compounds according to claim 1, wherein
- R1: is hydrogen or methyl
- R4: is phenyl,
- R6: is hydrogen,
- n: is 1 or 2,
- m: is 1 or 2,
- R7: is -W-Y,
- W: is 1,2,4-triazolylene,
- Y: is pyridin-2-yl, 2-pyrazinyl or 2-pyrimidinyl which is optionally substituted by R9 and optionally further substituted by R9A,
- R9: is hydrogen, methyl, F, Cl, Br, CF3,
- R9A: is methyl
- R10: is hydrogen or methyl,
or an N-oxide or a salt, a tautomer, or a stereoisomer of said compound, or a salt, of said N-oxide, tautomer or stereoisomer.

Another aspect of the invention relates to compounds of general formula (I) according to claim 1, wherein
- R1: is hydrogen or methyl,
- R4: is phenyl,
- R6: is hydrogen,
- n: is 2,
- m: is 2,
- R7: is -W-Y,
- W: is 1,2,4-triazolylene,
- Y: is pyridin-2-yl,
- R10: is hydrogen or methyl,
or an N-oxide or a salt, a tautomer, or a stereoisomer of said compound, or a salt, of said N-oxide, tautomer or stereoisomer.

A preferred aspect of the invention are compounds according to claim 1 selected from the group consisting of
5-methyl-8-phenyl-7-{4-[4-(5-pyridin-2-yl-1H-[1,2,4]triazol-3-yl)-piperidin-1-ylmethyl]-phenyl}-imidazo[1,2-c]pyrimidine,
8-phenyl-7-{4-[4-(5-pyridin-2-yl-1H-[1,2,4]triazol-3-yl)-piperidin-1-ylmethyl]-phenyl}-imidazo[1,2-c]pyrimidine,
8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine,
3-methyl-8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine,
7-(4-{4-[5-(5,6-dimethyl-pyridin-2-yl)-1H-[1,2,4]triazol-3-yl]-piperidin-1-ylmethyl}-phenyl)-5-methyl-8-phenyl-imidazo[1,2-c]pyrimidine,
5-methyl-8-phenyl-7-{4-[3-(5-pyridin-2-yl-1H-[1,2,4]triazol-3-yl)-azetidin-1-ylmethyl]-phenyl}-imidazo[1,2-c]pyrimidine,
5-methyl-8-phenyl-7-{4-[4-(5-pyrazin-2-yl-1H-[1,2,4]triazol-3-yl)-piperidin-1-ylmethyl]-phenyl}-imidazo[1,2-c]pyrimidine,
5-methyl-8-phenyl-7-{4-[4-(5-pyrimidin-2-yl-1H-[1,2,4]triazol-3-yl)-piperidin-1-ylmethyl]-phenyl}-imidazo[1,2-c]pyrimidine,
7-(4-{4-[5-(5-bromo-pyridin-2-yl)-1H-[1,2,4]triazol-3-yl]-piperidin-1-ylmethyl}-phenyl)-8-phenyl-imidazo[1,2-c]pyrimidine,
7-(4-{4-[5-(5,6-dimethyl-pyridin-2-yl)-1H-[1,2,4]triazol-3-yl]-piperidin-1-ylmethyl}-phenyl)-8-phenyl-imidazo[1,2-c]pyrimidine,
8-phenyl-7-{4-[4-(5-pyrazin-2-yl-1H-[1,2,4]triazol-3-yl)-piperidin-1-ylmethyl]-phenyl}-imidazo[1,2-c]pyrimidine,
7-(4-{4-[5-(5-fluoro-pyridin-2-yl)-1H-[1,2,4]triazol-3-yl]-piperidin-1-ylmethyl}-phenyl)-8-phenyl-imidazo[1,2-c]pyrimidine,
7-(4-{4-[5-(5-methyl-pyridin-2-yl)-1H-[1,2,4]triazol-3-yl]-piperidin-1-ylmethyl}-phenyl)-8-phenyl-imidazo[1,2-c]pyrimidine,
7-(4-{4-[5-(6-chloro-pyridin-2-yl)-1H-[1,2,4]triazol-3-yl]-piperidin-1-ylmethyl}-phenyl)-8-phenyl-imidazo[1,2-c]pyrimidine,
7-(4-{4-[5-(4,6-dimethyl-pyridin-2-yl)-1H-[1,2,4]triazol-3-yl]-piperidin-1-ylmethyl}-phenyl)-8-phenyl-imidazo[1,2-c]pyrimidine,
8-phenyl-7-(4-{4-[5-(4-trifluoromethyl-pyridin-2-yl)-1H-[1,2,4]triazol-3-yl]-piperidin-1-ylmethyl}-phenyl)-imidazo[1,2-c]pyrimidine,
8-phenyl-7-{4-[3-(5-pyridin-2-yl-1H-[1,2,4]triazol-3-yl)-azetidin-1-ylmethyl]-phenyl}-imidazo[1,2-c]pyrimidine,
or an N-oxide, a salt, a tautomer or a stereoisomer of said compound, or a salt of said N-oxide, tautomer or stereoisomer.
aspect of the invention are compounds according to claim 1 selected from the group consisting of
5-methyl-8-phenyl-7-{4-[4-(5-pyridin-2-yl-1H-[1,2,4]triazol-3-yl)-piperidin-1-ylmethyl]-phenyl}-imidazo[1,2-c]pyrimidine,
8-phenyl-7-{4-[4-(5-pyridin-2-yl-1H-[1,2,4]triazol-3-yl)-piperidin-1-ylmethyl]-phenyl}-imidazo[1,2-c]pyrimidine,
8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine,
3-methyl-8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine,
or a N-oxide or a salt, a tautomer, or a stereoisomer of said compound, or a salt, of said N-oxide, tautomer or stereoisomer.

An embodiment of the above-mentioned aspects of the invention relates to compounds of formula (I), wherein ring B fused with the pyrimidine ring is the following ring system 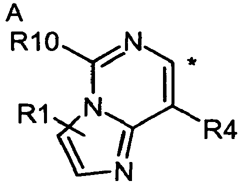 R1, R4 and R10 are defined as described above,
or an N-oxide, a salt, a tautomer or a stereoisomer of said compound, or a salt of said N-oxide, tautomer or stereoisomer.

A further embodiment of the above-mentioned aspects of the invention relates to compounds of formula (I), wherein ring B fused with the pyrimidine ring is the following ring system 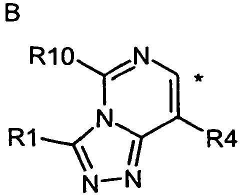 R1, R4 and R10 are defined as described above,
or an N-oxide, a salt, a tautomer or a stereoisomer of said compound, or a salt of said N-oxide, tautomer or stereoisomer.

In a further embodiment of the above-mentioned aspects, the invention relates to compounds of formula (I), wherein m is 1 and n is 1.

In a further embodiment of the above-mentioned aspects, the invention relates to compounds of formula (I), wherein m is 1 and n is 2.

In a further embodiment of the above-mentioned aspects, the invention relates to compounds of formula (I), wherein m is 2 and n is 2.

In a further embodiment of the above-mentioned aspects, the invention relates to compounds of formula (I), wherein m is 1 and n is 1 or m is 2 and n is 2.

In another embodiment the invention relates to compounds of formula (I), wherein R1 is is hydrogen, 1-4C-alkyl (optionally substituted by halogen, hydroxy, amino, mono- or di-1-4C-alkylamino), halogen, trifluoromethyl, cyano, 3-7C-cycloalkyl, 2-4C-alkenyl, 2-4C-alkynyl, -C(O)NR11R12, -C(O)OR2.

In a further embodiment of the above-mentioned aspects, the invention relates to compounds of formula (I), wherein R1 is hydrogen, 1-4C-alkyl (optionally substituted by halogen, hydroxy, amino, mono- or di-1-4C-alkylamino), halogen, trifluoromethyl, cyano or -C(O)NR11R12.

In a further embodiment of the above-mentioned aspects, the invention relates to compounds of formula (I), wherein R1 is hydrogen, 1-4C-alkyl, halogen, trifluoromethyl, cyano or -C(O)NR11R12.

In a further embodiment of the above-mentioned aspects, the invention relates to compounds of formula (I), wherein R1 is hydrogen or 1-4C-alkyl, especially hydrogen or methyl..

In a further embodiment of the above-mentioned aspects, the invention relates to compounds of formula (I), wherein R4 is phenyl.

In a further embodiment of the above-mentioned aspects, the invention relates to compounds of formula (I), wherein R6 is hydrogen.

In a further embodiment of the above-mentioned aspects, the invention relates to compounds of formula (I), wherein R8 is hydrogen, 1-4C-alkyl, 1-4C-haloalkyl, 3-7C-cycloalkyl, 1-4Calkoxy, halogen, cyano or NR11R12.

In a further embodiment of the above-mentioned aspects, the invention relates to compounds of formula (I), wherein R8 is hydrogen, 1-4C-haloalkyl, halogen, cyano or amino, especially R8 is hydrogen.

In a further embodiment of the above-mentioned aspects, the invention relates to compounds of formula (I), wherein W is a monocyclic 5- or -6-membered heteroarylene, especially a 5-membered heteroarylene.

In a further embodiment of the above-mentioned aspects, the invention relates to compounds of formula (I), wherein W is 1,2,4-triazolylene.

In a further embodiment of the above-mentioned aspects, the invention relates to compounds of formula (I), wherein W is a bicyclic heteroarylene comprising 1 nitrogen atom and optionally 1 , 2 or 3 further heteroatoms independently selected from oxygen, nitrogen and sulphur and wherein the bicyclic heteroarylene is optionally substituted by R8 and Y is hydrogen.

In a further embodiment of the above-mentioned aspects, the invention relates to compounds of formula (I), wherein Y is a monocyclic 5- or 6-membered heteroaryl comprising 1 nitrogen atom and optionally 1,2 or 3 further heteroatoms independently selected from oxygen, nitrogen, sulphur, and wherein the heteroaryl is optionally substituted by R9 and R9 is defined as in any of the claims.

In another embodiment Y is a 6-membered heteroaryl comprising 1 nitrogen atom and optionally 1,or 2 further nitrogen atoms, especially pyridyl, pyrimidinyl or pyrazinyl, more specifically 2-pyridyl, 2-pyrimidinyl or 2-pyrazinyl.

In a further embodiment of the above-mentioned aspects, the invention relates to compounds of formula (I), wherein Y is pyridin-2-yl, pyrimidin-2-yl or pyrazin-2-yl optionally substituted by R9 and optionally further substituted by R9A and R9 is 1-4C-alkyl, 1-4C-alkoxy, halogen, hydroxy, 1-4C-haloalkyl, NR11R12, cyano or - C(O)NH2 and R9A is 1-4C-alkyl or halogen.

In a further embodiment of the above-mentioned aspects, the invention relates to compounds of formula (I), wherein Y is pyridin-2-yl optionally substituted by R9 and R9 is 1-4C-alkyl, 1-4C-alkoxy, halogen, hydroxy, 1-4C-haloalkyl, NR11R12, cyano or -C(O)NH2.

In a further embodiment of the above-mentioned aspects, the invention relates to compounds of formula (I), wherein R9 is 1-4C-alkyl, 1-4C-alkoxy, halogen, hydroxy, 1-4C-haloalkyl, NR11R12, cyano or-C(O)NH2, especially 1-4C-alkyl, 1-4C-haloalkyl or halogen, more specifically CH3, F, Cl, Br, CF3.

In a further embodiment of the above-mentioned aspects, the invention relates to compounds of formula (I), wherein R9A is 1-4C-alkyl or halogen, especially 1-4C-alkyl, more specifically CH3.

In a further embodiment of the above-mentioned aspects, the invention relates to compounds of formula (I), wherein R10 is hydrogen or 1-4C-alkyl (optionally substituted by halogen, hydroxy, amino, mono-or di1-4C-alkylamino)., especially hydrogen or 1-4C-alkyl, more specifically hydrogen or CH3.

In a further embodiment of the above-mentioned aspects, the invention relates to compounds of formula (I), wherein R11, R12, which can be same or different, is hydrogen, 1-4C-alkyl (optionally substituted by halogen, hydroxy, amino, mono-or di-1-4C-alkylamino) or 3-7C-cycloalkyl.

In a further embodiment of the above-mentioned aspects, the invention relates to compounds of formula (I), wherein R11, R12, which can be same or different, is hydrogen or 1-4C-alkyl.

### Definitions

1-4C-Alkyl is a straight-chain or branched alkyl group having 1 to 4 carbon atoms. Examples are methyl, ethyl, n-propyl, iso-propyl, n-butyl, iso-butyl, sec-butyl and tert-butyl.

Mono- or di-1-4C-alkylamino radicals contain in addition to the nitrogen atom, one or two of the abovementioned 1-4C-alkyl radicals. Examples are the methyamino, the ethylamino, the isopropylamino, the dimethylamino, the diethylamino and the diisopropylamino radical.

Mono- or di-1-4C-alkylaminocarbonyl radicals contain in addition to the carbonyl group one of the abovementioned mono- or di-1-4C-alkylamino radicals. Examples are the N-methylaminocarbonyl, the N,N-dimethylaminocarbonyl, the N-ethylaminocarbonyl, the N-propylaminocarbonyl, the N,N-diethylaminocarbonyl and the N-isopropylaminocarbonyl.

Halogen within the meaning of the present invention is iodine, bromine, chlorine or fluorine, preferably bromine, chlorine or fluorine, in case of its use as leaving group bromine or iodine are preferred..

1-4C-Haloalkyl is a straight-chain or branched alkyl group having 1 to 4 carbon atoms in which at least one hydrogen is substituted by a halogen atom. Examples are chloromethyl or 2-bromoethyl. For a partially or completely fluorinated C₁-C₄-alkyl group which is included in the term "haloalkyl", the following partially or completely fluorinated groups are considered, for example: fluoromethyl, difluoromethyl, trifluoromethyl, fluoroethyl, 1,1-difluoroethyl, 1,2-difluoroethyl, 1,1,1-trifluoroethyl, tetrafluoroethyl, and pentafluoroethyl, trifluoromethyl is preferred.

1-4C-Alkoxy represents radicals, which in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy, ethoxy and methoxy radicals, methoxy is preferred.

3-7C-Cycloalkyl stands for cyclopropyl, cyclobutyl, cyclopentyl, cyclohexyl or cycloheptyl, preferably cyclopropyl, cyclobutyl, cyclopentyl, cyclohexyl.

3-7C-Cycloalkyloxy stands for cyclopropyloxy, cyclobutyloxy, cyclopentyloxy, cyclohexyloxy or cycloheptyloxy.

2-4C-Alkenyl is a straight chain or branched alkenyl radical having 2 to 4 carbon atoms. Examples are the but-2-enyl, but-3-enyl (homoallyl), prop-1-enyl, prop-2-enyl (allyl) and the ethenyl (vinyl) radicals.

2-4C-Alkynyl is a straight chain or branched alkynyl radical having 2 to 4 carbon atoms. Examples are the but-2-ynyl, but-3-ynyl (homopropargyl), prop-1-ynyl, 1-methylprop-2-ynyl (1-methylpropargyl), prop-2-ynyl (propargyl) and the ethinyl radicals.

The term "monocyclic 5- or 6-membered heteroaryl" comprises without being restricted thereto, the 5-membered heteroaryl radicals furyl, thienyl, pyrrolyl, oxazolyl, isoxazolyl, thiazolyl, isothiazolyl, imidazolyl, pyrazolyl, triazolyl (1,2,4-triazolyl, 1,3,4-triazolyl or 1,2,3-triazolyl), thiadiazolyl (1,3,4-thiadiazolyl, 1,2,5-thiadiazolyl, 1,2,3-thiadiazolyl or 1,2,4-thiadiazolyl) and oxadiazolyl (1,3,4-oxadiazolyl, 1,2,5-oxadiazolyl, 1,2,3-oxadiazolyl or 1,2,4-oxadiazolyl), as well as the 6-membered heteroaryl radicals pyridinyl, pyrimidinyl, pyrazinyl and pyridazinyl. Preferred 5- or 6-membered heteroaryl radicals are furanyl, thienyl, pyrrolyl, thiazolyl, oxazolyl, thiadiazolyl, oxadiazolyl, pyridinyl, pyrimidinyl, pyrazinyl or pyridazinyl. More preferred 5- or 6-membered heteroaryl radicals are furan-2-yl, thien-2-yl, pyrrol-2-yl, thiazolyl, oxazolyl, 1,3,4-thiadiazolyl, 1,3,4-oxadiazolyl, pyridin-2-yl, pyridin-4-yl, pyrimidin-2-yl, pyrimidin-4-yl, pyrazin-2-yl or pyridazin-3-yl.

The term "monocyclic 5-membered heteroarylene" is a divalent radical in which arbitrary one hydrogen atom is eliminated from the above described "heteroaryl" and may include, without being restricted thereto, the 5-membered heteroaryl radicals furylene, thienylene, pyrrolylene, oxazolylene, isoxazolylene, thiazolylene, isothiazolylene, imidazolylene, pyrazolylene, triazolylene (1,2,4-triazolylene, 1,3,4-triazolylene or 1,2,3-triazolylene), thiadiazolylene (1,3,4-thiadiazolylene, 1,2,5-thiadiazolylene, 1,2,3-thiadiazolylene or 1,2,4-thiadiazolylene) and oxadiazolylene (1,3,4-oxadiazolylene, 1,2,5-oxadiazolylene, 1,2,3-oxadiazolylene or 1,2,4-oxadiazolylene). Preferred 5-membered heteroaryl radicals are triazolylene, pyrazolylene or imidazolylene. More prefered 5-membered heteroaryl radicals are 1,2,4-triazolylene, pyrazolylene or imidazolylene, most preferred is 1,2,4-triazolylene.

The NR10R11 group includes amino (NH2), mono-alkyl-amino, di-alkylamino,especially amino (NH2), mono-1-4C-alkyl-amino, di-1-4C-alkylamino, for example, NH, N(H)CH₃, N(CH₃)₂, N(H)CH2CH3 and N(CH3)CH2CH3.

In general and unless otherwise mentioned, the heteroarylic or heteroarylenic radicals include all the possible isomeric forms thereof, e.g. the positional isomers thereof. Thus, for some illustrative non-restricting example, the term pyridinyl or pyridinylene includes pyridin-2-yl, pyridin-2-ylene, pyridin-3-yl, pyridin-3-ylene, pyridin-4-yl and pyridin-4-ylene; or the term thienyl or thienylene includes thien-2-yl, thien-2-ylene, thien-3-yl and thien-3-ylene.

Constituents which are optionally substituted as stated herein, may be substituted, unless otherwise noted, one or more times, independently from one another at any possible position. Analogously it is being understood that it is possible for any heteroaryl group if chemically suitable that said heteroaryl group may be attached to the rest of the molecule via any suitable atom. If not indicated otherwise the following substituents are preferred; methyl, ethyl, trifluoromethyl, fluorine, chlorine, bromine, methoxy, amino, methylamino, dimethylamino.

The heteroarylic or heteroarylenic groups mentioned herein may be substituted by their given substituents or parent molecular groups, unless otherwise noted, at any possible position, such as e.g. at any substitutable ring carbon or ring nitrogen atom.

Unless otherwise noted, rings containing quatemizable amino- or imino-type ring nitrogen atoms (-N=) may be preferably not quatemized on these amino- or imino-type ring nitrogen atoms by the mentioned substituents or parent molecular groups.

Unless otherwise noted, any heteroatom of a heteroarylic or heteroarylenic ring with unsatisfied valences mentioned herein is assumed to have the hydrogen atom(s) to satisfy the valences.

When any variable occurs more than one time in any constituent, each definition is independent.
Unless otherwise noted the term "suitable leaving group" means for example a sulfonate, such as namely methanesulfonate, trifluoromethanesulfonate or para-toluenesulfonate or halogen, e.g. iodine, bromine or chorine.

Salts of the compounds according to the invention include all inorganic and organic acid addition salts and salts with bases, especially all pharmaceutically acceptable inorganic and organic acid addition salts and salts with bases, particularly all pharmaceutically acceptable inorganic and organic acid addition salts and salts with bases customarily used in pharmacy.

Examples of acid addition salts include, but are not limited to, hydrochlorides, hydrobromides, phosphates, nitrates, sulfates, salts of sulfamic acid, formates, acetates, propionates, citrates, D-gluconates, benzoates, 2-(4-hydroxybenzoyl)-benzoates, butyrates, salicylates, sulfosalicylates, lactates, maleates, laurates, malates, fumarates, succinates, oxalates, malonates, pyruvates, acetoacetates, tartarates, stearates, benzensulfontes, toluenesulfonates, methanesulfonates, trifluoromethansulfonates, 3-hydroxy-2-naphthoates, benzenesulfonates, naphthalinedisulfonates, and trifluoroacetates.

Examples of salts with bases include, but are not limited to, lithium, sodium, potassium, calcium, aluminum, magnesium, titanium, meglumine, ammonium salts optionally derived from NH₃ or organic amines having from 1 to 16 C-atoms such as e.g. ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylendiamine, N-methylpiperindine and guanidinium salts.

The salts include water-insoluble and, particularly, water-soluble salts.

According to the person skilled in the art the compounds of formula (I) according to this invention as well as their salts may contain, e.g. when isolated in crystalline form, varying amounts of solvents. Included within the scope of the invention are therefore all solvates and in particular all hydrates of the compounds of formula (I) according to this invention as well as all solvates and in particular all hydrates of the salts of the compounds of formula (I) according to this invention.

The term "combination" in the present invention is used as known to persons skilled in the art and may be present as a fixed combination, a non-fixed combination or kit-of-parts.

A "fixed combination" in the present invention is used as known to persons skilled in the art and is defined as a combination wherein the said first active ingredient and the said second active ingredient are present together in one unit dosage or in a single entity. One example of a "fixed combination" is a pharmaceutical composition wherein the said first active ingredient and the said second active ingredient are present in admixture for simultaneous administration, such as in a formulation. Another example of a "fixed combination" is a pharmaceutical combination wherein the said first active ingredient and the said second active ingredient are present in one unit without being in admixture.

A non-fixed combination or "kit-of-parts" in the present invention is used as known to persons skilled in the art and is defined as a combination wherein the said first active ingredient and the said second active ingredient are present in more than one unit. One example of a non-fixed combination or kit-of-parts is a combination wherein the said first active ingredient and the said second active ingredient are present separately. The components of the non-fixed combination or kit-of-parts may be administered separately, sequentially, simultaneously, concurrently or chronologically staggered.

The term "(chemotherapeutic) anti-cancer agents", includes but is not limited to (i) alkylating/carbamylating agents such as Cyclophosphamid (Endoxan®), Ifosfamid (Holoxan®), Thiotepa (Thiotepa Lederle®), Melphalan (Alkeran®), or chloroethylnitrosourea (BCNU); (ii) platinum derivatives like cis-platin (Platinex® BMS), oxaliplatin (Eloxatin®), satraplatin or carboplatin (Cabroplat® BMS); (iii) antimitotic agents / tubulin inhibitors such as vinca alkaloids (vincristine, vinblastine, vinorelbine), taxanes such as Paclitaxel (Taxol®), Docetaxel (Taxotere®) and analogs as well as new formulations and conjugates thereof (like the nanoparticle formulation Abraxane® with paclitaxel bound to albumin), epothilones such as Epothilone B (Patupilone®), Azaepothilone (Ixabepilone®) or ZK-EPO, a fully synthetic epothilone B analog; (iv) topoisomerase inhibitors such as anthracyclines (exemplified by Doxorubicin / Adriblastin®), epipodophyllotoxines (examplified by Etoposide / Etopophos®) and camptothecin and camptothecin analogs (exemplified by Irinotecan / Camptosar® or Topotecan / Hycamtin®); (v) pyrimidine antagonists such as 5-fluorouracil (5-FU), Capecitabine (Xeloda®), Arabinosylcytosine / Cytarabin (Alexan®) or Gemcitabine (Gemzar®); (vi) purin antagonists such as 6-mercaptopurine (Puri-Nethol®), 6-thioguanine or fludarabine (Fludara®) and (vii) folic acid antagonists such as methotrexate (Farmitrexat®) or premetrexed (Alimta®).

The term "target specific anti-cancer agent", includes but is not limited to (i) kinase inhibitors such as e.g. Imatinib (Glivec®), ZD-1839 / Gefitinib (Iressa®), Bay43-9006 (Sorafenib, Nexavar®), SU11248 / Sunitinib (Sutent®), OSI-774 / Erlotinib (Tarceva®), Dasatinib (Sprycel®), Lapatinib (Tykerb®), or, see also below, Vatalanib, Vandetanib (Zactima®) or Pazopanib; (ii) proteasome inhibitors such as PS-341 / Bortezumib (Velcade®); (iii) histone deacetylase inhibitors like SAHA (Zolinza®), PXD101, MS275, MGCD0103, Depsipeptide / FK228, NVP-LBH589, Valproic acid (VPA), CRA / PCI 24781, ITF2357, SB939 and butyrates (iv) heat shock protein 90 inhibitors like 17-allylaminogeldanamycin (17-AAG) or 17-dimethylaminogeldanamycin (17-DMAG); (v) vascular targeting agents (VTAs) like combretastin A4 phosphate or AVE8062 / AC7700 and anti-angiogenic drugs like the VEGF antibodies, such as Bevacizumab (Avastin®), or KDR tyrosine kinase inhibitors such as PTK787 / ZK222584 (Vatalanib®) or Vandetanib (Zactima®) or Pazopanib; (vi) monoclonal antibodies such as Trastuzumab (Herceptin®), Rituximab (MabThera / Rituxan®), Alemtuzumab (Campath®), Tositumomab (Bexxar®), C225/ Cetuximab (Erbitux®), Avastin (see above) or Panitumumab (Vectibix®) as well as mutants and conjugates of monoclonal antibodies, e.g. Gemtuzumab ozogamicin (Mylotarg®) or Ibriturnomab tiuxetan (Zevalin®), and antibody fragments; (vii) oligonucleotide based therapeutics like G-3139 / Oblimersen (Genasense®) or the DNMT1 inhibitor MG98; (viii) Toll-like receptor / TLR 9 agonists like Promune®, TLR 7 agonists like Imiquimod (Aldara®) or Isatoribine and analogues thereof, or TLR 7/8 agonists like Resiquimod as well as immunostimulatory RNA as TLR 7/8 agonists; (ix) protease inhibitors; (x) hormonal therapeutics such as anti-estrogens (e.g. Tamoxifen or Raloxifen), antiandrogens (e.g. Flutamide or Casodex), LHRH analogs (e.g. Leuprolide, Goserelin or Triptorelin) and aromatase inhibitors (e.g. Femara, Arimedex or Aromasin).

Other "target specific anti-cancer agents" includes bleomycin, retinoids such as all-trans retinoic acid (ATRA), DNA methyltransferase inhibitors such as 5-Aza-2'-deoxycytidine (Decitabine, Dacogen®) and 5-azacytidine (Vidaza®), alanosine, cytokines such as interleukin-2, interferons such as interferon α2 or interferon-γ, bcl2 antagonists (e.g. ABT-737 or analogs), death receptor agonists, such as TRAIL, DR4/5 agonistic antibodies, FasL and TNF-R agonists (e.g. TRAIL receptor agonists like mapatumumab or lexatumumab).

Specific examples include, but is not limited to 5 FU, actinomycin D, ABARELIX, ABCIXIMAB, ACLARUBICIN, ADAPALENE, ALEMTUZUMAB, ALTRETAMINE, AMINOGLUTETHIMIDE, AMIPRILOSE, AMRUBICIN, ANASTROZOLE, ANCITABINE, ARTEMISININ, AZATHIOPRINE, BASILIXIMAB, BENDAMUSTINE, BEVACIZUMAB, BEXXAR, BICALUTAMIDE, BLEOMYCIN, BORTEZOMIB, BROXURIDINE, BUSULFAN, CAMPATH, CAPECITABINE, CARBOPLATIN, CARBOQUONE, CARMUSTINE, CETRORELIX, CHLORAMBUCIL, CHLORME-THINE, CISPLATIN, CLADRIBINE, CLOMIFENE, CYCLOPHOSPHAMIDE, DACARBAZINE, DACLIZUMAB, DACTINOMYCIN, DASATINIB, DAUNORUBI-CIN, DECITABINE, DESLORELIN, DEXRAZOXANE, DOCETAXEL, DOXIFLURIDINE, DOXORUBICIN, DROLOXIFENE, DROSTANOLONE, EDELFOSINE, EFLORNITHINE, EMITEFUR, EPIRUBICIN, EPITIOSTANOL, EPTAPLATIN, ERBITUX, ERLOTINIB, ESTRAMUSTINE, ETOPOSIDE, EXEMESTANE, FADROZOLE, FINASTERIDE, FLOXURIDINE, FLUCYTOSINE, FLUDARABINE, FLUOROURACIL, FLUTAMIDE, FORMESTANE, FOSCARNET, FOSFESTROL, FOTEMUSTINE, FULVESTRANT, GEFITINIB, GENASENSE, GEMCITABINE, GLIVEC, GOSERELIN, GUSPERIMUS, HERCEPTIN, IDARUBICIN, IDOXURIDINE, IFOSFAMIDE, IMATINIB, IMPROSULFAN, INFLIXIMAB, IRINOTECAN, IXABEPILONE, LANREOTIDE, LAPATINIB, LETROZOLE, LEUPRORELIN, LO-BAPLATIN, LOMUSTINE, LUPROLIDE, MELPHALAN, MERCAPTOPURINE, METHOTREXATE, METUREDEPA, MIBOPLATIN, MIFEPRISTONE, MILTEFOSINE, MIRIMOSTIM, MITOGUAZONE, MITOLACTOL, MITOMYCIN, MI-TOXANTRONE, MIZORIBINE, MOTEXAFIN, MYLOTARG, NARTOGRASTIM, NEBAZUMAB, NEDAPLATIN, NILUTAMIDE, NIMUSTINE, OCTREOTIDE, OR-MELOXIFENE, OXALIPLATIN, PACLITAXEL, PALIVIZUMAB, PANITUMUMAB, PATUPILONE, PAZOPANIB, PEGASPARGASE, PEGFILGRASTIM, PE-METREXED, PENTETREOTIDE, PENTOSTATIN, PERFOSFAMIDE, PIPOSUL-FAN, PIRARUBICIN, PLICAMYCIN, PREDNIMUSTINE, PROCARBAZINE, PROPAGERMANIUM, PROSPIDIUM CHLORIDE, RALOXIFEN, RALTITREXED, RANIMUSTINE, RANPIRNASE, RASBURICASE, RAZOXANE, RITUXIMAB, RI-FAMPICIN, RITROSULFAN, ROMURTIDE, RUBOXISTAURIN, SARGRAMO-STIM, SATRAPLATIN, SIROLIMUS, SOBUZOXANE, SORAFENIB, SPIRO-MUSTINE, STREPTOZOCIN, SUNITINIB, TAMOXIFEN, TASONERMIN, TEGA-FUR, TEMOPORFIN, TEMOZOLOMIDE, TENIPOSIDE, TESTOLACTONE, THIOTEPA, THYMALFASIN, TIAMIPRINE, TOPOTECAN, TOREMIFENE, TRAIL, TRASTUZUMAB, TREOSULFAN, TRIAZIQUONE, TRIMETREXATE, TRIPTORELIN, TROFOSFAMIDE, UREDEPA, VALRUBICIN, VATALANIB, VANDETANIB, VERTEPORFIN, VINBLASTINE, VINCRISTINE, VINDESINE, VINORELBINE, VOROZOLE, ZEVALIN and ZOLINZA.

The compounds according to the invention and their salts can exist in the form of tautomers which are included in the embodiments of the invention. In particular, those compounds of the invention which contain a pyrazole moiety for example can exist as a 1 H tautomer, or a 2H tautomer, or even a mixture in any amount of the two tautomers, or a triazole moiety for example can exist as a 1 H tautomer, a 2H tautomer, or a 4H tautomer, or even a mixture in any amount of said 1 H, 2H and 4H tautomers :

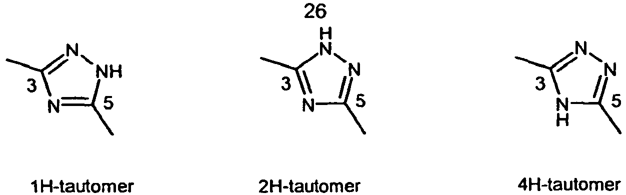

The compounds according to the invention and the salts thereof include stereoisomers. Each of the stereogenic centers present in said stereoisomers may have the absolute configuration R or the absolute configuration S (according to the rules of Cahn, Ingold and Prelog). Accordingly, the stereoisomers (1S) and (1 R) in case of a compound of formula (Ia*) 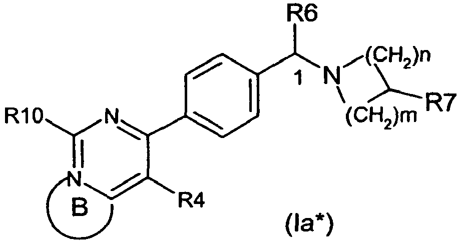 and the salts thereof are part of the invention.

The invention further includes all mixtures of the stereoisomers mentioned above independent of the ratio, including the racemates.

Some of the compounds and salts according to the invention may exist in different crystalline forms (polymorphs) which are within the scope of the invention.

Furthermore, derivatives of the compounds of formula (I) and the salts thereof which are converted into a compound of formula (I) or a salt thereof in a biological system (bioprecursors or pro-drugs) are covered by the invention. Said biological system is e.g. a mammalian organism, particularly a human subject. The bioprecursor is, for example, converted into the compound of formula (I) or a salt thereof by metabolic processes.

The intermediates used for the synthesis of the compounds of claims 1-4 as described below as well as their use for the synthesis of the compounds of claims 1-4 are one further aspect of the present invention.

The compounds according to the invention can be prepared as follows:

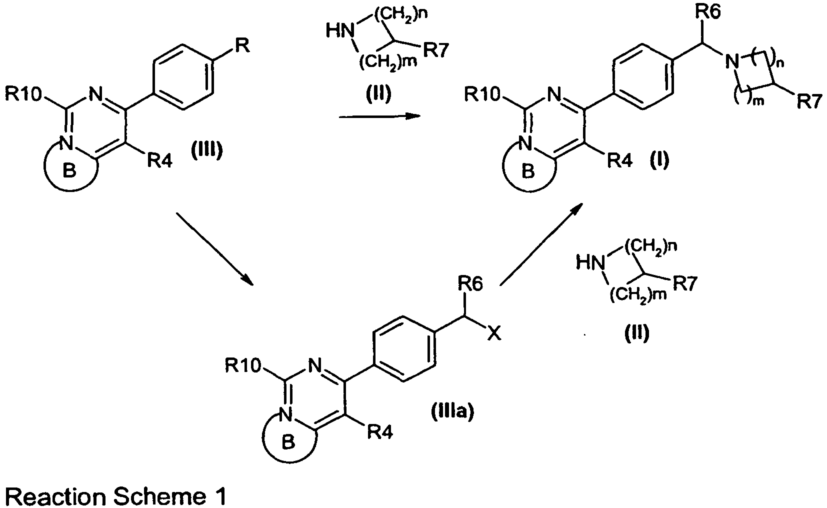

As shown in reaction scheme 1 the compounds of formula (I), wherein B, R4, R7, m, n and R10 have the above mentioned meanings and R6 is hydrogen or 1-4C-alkyl, can be obtained by a reductive amination reaction of a corresponding compound of formula (III), wherein R has the meaning -C(O)R6, with an amine derivative of formula (II), wherein R7 has the above-mentioned meanings. The reductive amination can be carried out according to standard procedures, for example by the use of NaBH(OAc)3 or NaBH3CN in a suitable solvent exemplified by 1,2-dichloroethane (DCE), tetrahydrofuran (THF), N-methylpyrrolidinone (NMP), dimethylformamide (DMF) or methanol or appropriate mixtures of the above solvents.

The amine derivatives of formula (II), wherein R7, m and n have the abovementioned meanings are known or can be prepared according to known procedures (they may contain protecting group(s) in certain cases to protect other functionalities such as but not limited to NH functions). The amine derivatives of formula (II) may be prepared as as a suitable salt, such as, for example a hydrochloride salt, whereby the hydrochloride salt may be a monohydrochloride, or a dihydrochloride. Reactions using salts of amine derivatives of formula (II) require the addition of a suitable base, such as, for example triethylamine. Unless otherwise stated, for the purposes of calculating the amounts of base necessary for reactions with the salts of amine derivatives of formula (II), it is assumed that the salt of the amine derivative of formula (II) is a divalent salt, such as, for example the dihydrochloride salt.

The use of the compounds of formula (II), or the salts thereof, for the synthesis of the compounds of claims 1-4 is one aspect of the present invention.

Compounds of formula (III), wherein R has the meaning -C(O)H can be obtained from corresponding compounds of formula (III), wherein R has the meaning-C(O)O(1-4C-alkyl), in a one or two step procedure. The ester group is selectively reduced to the aldehyde group by methods known to the skilled person, for example by the use of DIBALH under low temperature for example -80 to -60°C in the one step procedure. Alternatively, the ester group is reduced to the alcohol group (-CH2OH) according to known procedures, for example by the use of LiAlH4 or NaBH4, and then, the resulting alcohol is selectively oxidized to the-C(O)H group by methods known to the skilled person, for example with SO3-pyridine complex or Dess-Martin Periodinane, in the two step procedure.

Alternatively to the reaction sequence described above, the compounds of formula (I), wherein B, R4, R7, m, n and R10 have the above mentioned meanings and R6 is hydrogen or 1-4C-alkyl, can be obtained by reaction of a corresponding compound of formula (IIIa), wherein X is a suitable leaving group, such as for example a halogen atom or a sulfonate, with amine derivatives of formula (II), wherein R7, m and n have the above-mentioned meanings. The reaction is preferably carried out in an inert solvent, such as for example DMF, at a temperature of from 60 to 100°C in presence of a base, such as for example triethylamine.

Compounds of formula (IIIa), wherein X is a suitable leaving group, for example a halogen atom can be obtained from corresponding compounds of formula (III), wherein R is -CH(R6)OH and R6 is hydrogen or 1-4C-alkyl, by a halogenation reaction. Such a halogenation reaction can be accomplished, for example, by the use of PBr3 in dichloromethane.

Alternatively, compounds of formula (IIIa), wherein X is a suitable leaving group, for example a halogen atom can be obtained from corresponding compounds of formula (III), wherein R is -CH2R6 and R6 is hydrogen or 1-4C-alkyl, by means of benzylic halogenation. Benzylic halogenation can, for example, be achieved by the use of N-bromosuccinimide (NBS).

Alternatively, compounds of formula (IIIa), wherein X is a suitable leaving group, for example a sulfonate, such as namely methanesulfonate, trifluoromethanesulfonate or para-toluenesulfonate, can be obtained from corresponding compounds of formula (III), wherein R is -CH(R6)OH and R6 is hydrogen or 1-4C-alkyl, by a sulfonylation reaction. Such a sulfonylation reaction can, for example, be achieved by the use of the appropriate sulfonyl anhydride or sulfonyl halide such as, for example, methanesulfonyl chloride, in the presence of a suitable base such as, for example triethylamine, in a suitable solvent such as, for example, dichloromethane or dimethylformamide, or mixtures of the same.

Compounds of formula (III), wherein R is -CH(R6)OH and R6 is hydrogen or 1-4C-alkyl, can, for example, be obtained from corresponding compounds of formula (III), wherein R is -C(O)R6, by methods known to the person skilled in the art, for example by reduction with NaBH4 or LiAlH4.

Alternatively, compounds of formula (III), wherein R is -CH(R6)OH and R6 is hydrogen or 1-4C-alkyl, can be obtained from corresponding compounds of formula (III), wherein R is -CH2R6, by means of benzylic oxidation, which can be achieved, for example, by the use of catalytic or equimolar amounts of SeO2.

In a further alternative, compounds of formula (III), wherein R is -CH(1-4C-alkyl)OH can be obtained from corresponding compounds of formula (III), wherein R is -C(O)H by the addition of a suitable metal organic reagent, such as, but not limited to Gringnard or Lithium reagents.

If necessary for the reactions in reaction scheme 1, for the synthesis of compounds of formula (III), wherein B, R4 and R10 have the above mentioned meanings and R is -C(O)R6 or -CH(R6)OH, these groups can be protected in some or all of the precursors by suitable protecting groups known to the person skilled in the art. Compounds of formula (III), wherein B, R4 and R10 have the above mentioned meanings and R is a protected ketone, aldehyde or alcohol group, can be deprotected by art-known removal of the protecting groups to generate the corresponding deprotected compounds.

Compounds of formula (IIIb), wherein R1, R4 and R10 have the above mentioned meanings and R has the meanings -C(O)O(1-4C-alkyl), -C(O)R6, -CH(R6)OH or -CH2R6 and R6 is hydrogen or 1-4C-alkyl, can be obtained as shown in reaction scheme 2. 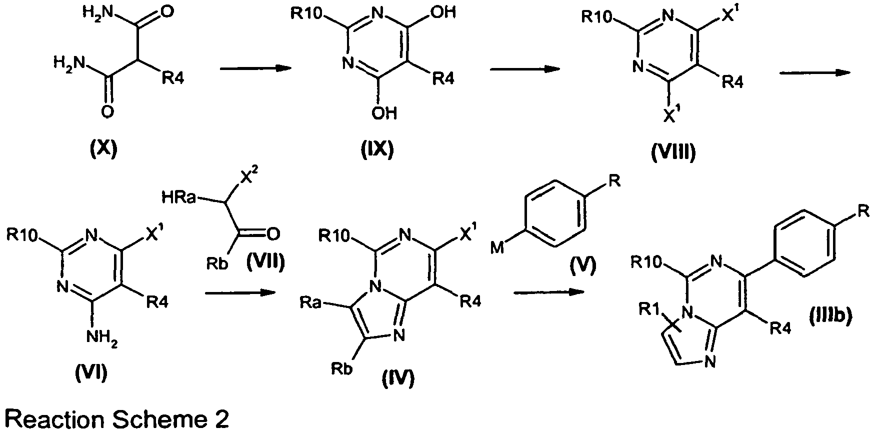 Compounds of formula (III), wherein R1, R4 and R10 have the above mentioned meanings and R has the meanings -C(O)O(1-4C-alkyl), -C(O)R6, -CH(R6)OH or -CH2R6 and R6 is hydrogen or 1-4C-alkyl, can be obtained by a transition metal catalysed C-C bond formation of a corresponding compound of formula (IV), wherein X1 is Cl, Br, I, or -OS(O)2CF3 and Ra and Rb, independently, have the meanings as defined for R1 above, with a corresponding compound of formula (V), wherein M, for example, is -B(OH)2, -Sn(1-4C-alkyl)3, -ZnCl, -ZnBr, -Znl, or,

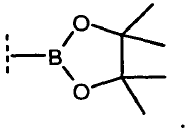

This transition metal catalysed C-C bond formation reaction can, for example, be achieved if M has the meaning of -B(OH)2 in a mixture of dioxane and aqueous Cs2CO3 solution at a temperature between 60 and the boiling point of the solvent, preferably at 115°C and by employing a Pd catalyst such as but not limited to 1,1'-bis(diphenylphosphino)ferrocene]palladium dichloride or Pd(PPh3)4. Compounds of formula (IV), wherein R4, R10, Ra, Rb and X1 have the above mentioned meanings, may be obtained by a condensation reaction of a compound of general formula (VI), wherein R4, R10 and X1 have the above mentioned meanings, with an aldehyde or ketone of formula (VII), Ra and Rb have the above mentioned meanings and X2 is Cl, Br, or I. The condensation reaction may be carried out in a suitable solvent, such as, for example, ethanol, at elevated temperatures, such as, for example, 100 °C. The elevated temperature may be achieved by conventional heating or by the use of microwave irradiation. Aldehydes or ketones of general formula (VII) are either commercial or may be prepared by methods known to those skilled in the art.

Compounds of general formula (VI), wherein R4, R10 and X1 have the above mentioned meanings, may be obtained from compounds of general formula (VIII) by an amination reaction. The amination reaction may be carried out by reacting a compound of general formula (VIII) with a suitable ammonia source, such as, for example a solution of ammonia in ethanol, or aqueous ammonia, at elevated temperatures, such as, for example 100-120 °C, in suitable solvents, such as for example, ethanol, whereby the ammonia source can serve as solvent for the reaction.

Compounds of general formula (VIII), wherein R4, R10 and X1 have the above mentioned meanings, may be obtained from compounds of general formula (IX) by a halogenation reaction, such as, for example, by treatment with POCl3 in the case that X1 has the meaning of Cl, or POBr3 in the case that X1 has the meaning of Br.

Compounds of general formula (IX), wherein R4 and R10 have the above mentioned meanings, may be obtained from compounds of general formula (X) by a condensation reaction with an ester of general formula R10-C(O)ORy, wherein Ry is 1-4C-alkyl, in the presence of a suitable base, such as, for example NaOEt, in a suitable solvent, such as, for example ethanol, at elevated temperatures, such as, for example 50 °C. Compounds of general formula (IX) or R10-C(O)ORy are commercially available, or may be prepared by methods known to those skilled in the art.

Compounds of formula (IIIc), wherein R1, R4 and R10 have the above defined meanings and R has the meanings -C(O)O(1-4C-alkyl), -C(O)R6, -CH(R6)OH or -CH2R6 and R6 is hydrogen or 1-4C-alkyl, can be obtained as shown in reaction scheme 3.

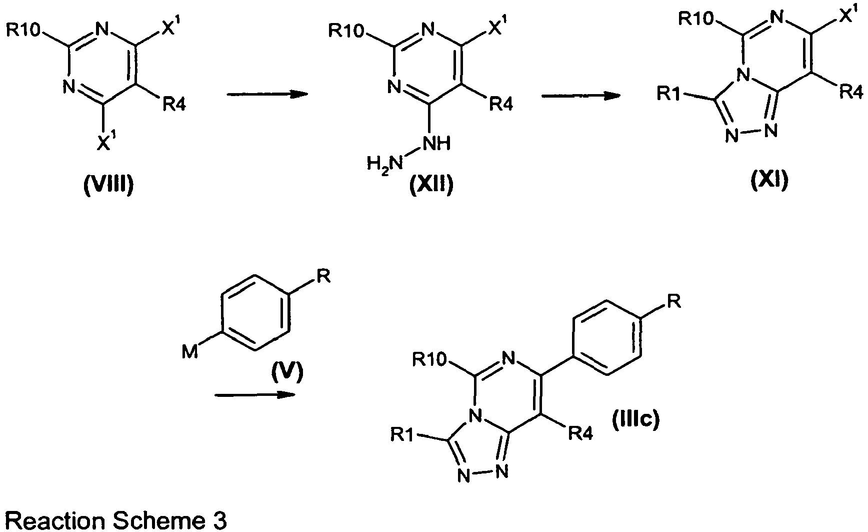

Compounds of formula (III), wherein R1, R4 and R10 have the above mentioned meanings, and R has the meanings -C(O)O(1-4C-alkyl), -C(O)R6, -CH(R6)OH or-CH2R6 and R6 is hydrogen or 1-4C-alkyl, can be obtained by a transition metal catalysed C-C bond formation of a corresponding compound of formula (XI), wherein X1 is Cl, Br, I, or -OS(O)2CF3, with a corresponding compound of formula (V), wherein M, for example, is -B(OH)2, -Sn(1-4C-alkyl)3, -ZnCl,-ZnBr, -Znl, or,

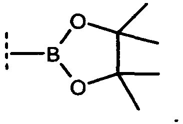

This transition metal catalysed C-C bond formation reaction can, for example, be achieved if M has the meaning of -B(OH)2 in a mixture of dioxane and aqueous Cs2CO3 solution at a temperature between 60 and the boiling point of the solvent, preferably at 115°C and by employing a Pd catalyst such as but not limited to 1,1'-bis(diphenylphosphino)ferrocene]palladium dichloride or Pd(PPh3)4.

Compounds of formula (XI), may be obtained by a condensation reaction of a compound of general formula (XII) with an orthoester of general formula R1(ORy)3, wherein R1 has the above mentioned meanings and Ry is 1-4C-alkyl. The condensation reaction may be carried out in a suitable solvent, whereby the orthoester may serve as the solvent for the reaction, at elevated temperatures, such as, for example the boiling point of the solvent.

Compounds of general formula (XII), wherein R4, R10 and X1 have the above mentioned meanings, may be obtained from compounds of general formula (VIII) by reaction with a suitable hydrazine source, such as, for example hydrazine hydrate. Said reaction may be carried out by reacting a compound of general formula (VIII) with said hydrazine source, in a suitable solvent, such as for example, ethanol, at elevated temperatures, such as, for example, 50 °C.

Alternatively, compounds of general formula (I) wherein R1 = halogen, may be further functionalised to give additional compounds of general formula (I), wherein R1 is 1-4C-alkyl, cyano, 3-7C-cycloalkyl, 2-4C-alkenyl, 2-4C-alkynyl, -C(O)OR2, or a monocyclic 5- or 6-membered heteroarylene. The transformations may be achieved via metal mediated reactions well known to the person skilled in the art, for example those described above.

One preferred aspect of the invention is the process for the preparation of the compounds of claims 1-4 according to the examples.

Optionally, compounds of the formula (I) can be converted into their salts, or, optionally, salts of the compounds of the formula (I) can be converted into the free compounds. Corresponding processes are customary for the skilled person.

It is known to the person skilled in the art that, if there are a number of reactive centers on a starting or intermediate compound, it may be necessary to block one or more reactive centers temporarily by protective groups in order to allow a reaction to proceed specifically at the desired reaction center. A detailed description for the use of a large number of proven protective groups is found, for example, in T. W. Greene, Protective Groups in Organic Synthesis, John Wiley & Sons, 1999, 3rd Ed., or in P. Kocienski, Protecting Groups, Thieme Medical Publishers, 2000.

The compounds according to the invention are isolated and purified in a manner known per se, e.g. by distilling off the solvent in vacuo and recrystallizing the residue obtained from a suitable solvent or subjecting it to one of the customary purification methods, such as column chromatography on a suitable support material.

Salts of the compounds of formula (I) according to the invention can be obtained by dissolving the free compound in a suitable solvent (for example a ketone such as acetone, methylethylketone or methylisobutylketone, an ether such as diethyl ether, tetrahydrofuran or dioxane, a chlorinated hydrocarbon such as methylene chloride or chloroform, or a low molecular weight aliphatic alcohol such as methanol, ethanol or isopropanol) which contains the desired acid or base, or to which the desired acid or base is then added. The acid or base can be employed in salt preparation, depending on whether a mono- or polybasic acid or base is concerned and depending on which salt is desired, in an equimolar quantitative ratio or one differing therefrom. The salts are obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts, which can be obtained, for example, as process products in the manufacturing on an industrial scale, can be converted into pharmaceutically acceptable salts by processes known to the person skilled in the art.

Optionally, compounds of the formula (I) can be converted into their N-oxides. The N-oxide may also be introduced by way of an intermediate. N-oxides may be prepared by treating an appropriate precursor with an oxidizing agent, such as meta-chloroperbenzoic acid, in an appropriate solvent, such as dichloromethane, at suitable temperatures, such as from 0 °C to 40 °C, whereby room temperature is generally preferred. Further corresponding processes for forming N-oxides are customary for the skilled person.

Pure diastereomers and pure enantiomers of the compounds and salts according to the invention can be obtained e.g. by asymmetric synthesis, by using chiral starting compounds in synthesis and by splitting up enantiomeric and diasteriomeric mixtures obtained in synthesis.

Enantiomeric and diastereomeric mixtures can be split up into the pure enantiomers and pure diastereomers by methods known to a person skilled in the art. Preferably, diastereomeric mixtures are separated by crystallization, in particular fractional crystallization, or chromatography. Enantiomeric mixtures can be separated e.g. by forming diastereomers with a chiral auxiliary agent, resolving the diastereomers obtained and removing the chiral auxiliary agent. As chiral auxiliary agents, for example, chiral acids can be used to separate enantiomeric bases such as e.g. mandelic acid and chiral bases can be used to separate enantiomeric acids via formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

As will be appreciated by persons skilled in the art, the invention is not limited to the particular embodiments described herein, but covers all modifications of said embodiments that are within the spirit and scope of the invention as defined by the appended claims.

### Commercial utility

The compounds of formula (I) and the stereoisomers of the compounds of formula (I) according to the invention are hereinafter referred to as the compounds of the invention. In particular, the compounds of the invention are pharmaceutically acceptable. The compounds according to the invention have valuable pharmaceutical properties, which make them commercially utilizable. In particular, they inhibit the Pi3K/Akt pathway and exhibit cellular activity. They are expected to be commercially applicable in the therapy of diseases (e.g. diseases dependent on overactivated Pi3K/Akt.
An abnormal activation of the PI3K/AKT pathway is an essential step towards the initiation and maintenance of human tumors and thus its inhibition, for example with AKT inhibitors, is understood to be a valid approach for treatment of human tumors. For a recent review see Garcia-Echeverria et al (Oncogene, 2008, 27, 551-4526).

Cellular activity and analogous terms in the present invention is used as known to persons skilled in the art, as an example, induction of apoptosis or chemosensitization.

Chemosensitization and analogous terms in the present invention is used as known to persons skilled in the art. These stimuli include, for example, effectors of death receptor and survival pathways as well as cytotoxic / chemotherapeutic and targeted agents and finally radiation therapy. Induction of apoptosis and analogous terms according to the present invention are used to identify a compound which excecutes programmed cell death in cells contacted with that compound or in combination with other compounds routinely used for therapy. Apoptosis in the present invention is used as known to persons skilled in the art. Induction of apoptosis in cells contacted with the compound of this invention might not necessarily be coupled with inhibition of cell proliferation. Preferably, the inhibition of proliferation and/or induction of apoptosis are specific to cells with aberrant cell growth.

Further on, the compounds according to the present invention inhibit protein kinase activity in cells and tissues, causing a shift towards dephosphorylated substrate proteins and as functional consequence, for example the induction of apoptosis, cell cycle arrest and/or sensitization towards chemotherapeutic and target-specific cancer drugs. In a preferred embodiment, inhibition of Pi3K/Akt pathway induces cellular effects as mentioned herein alone or in combination with standard cytotoxic or targeted cancer drugs.

Compounds according to the present invention exhibit anti-proliferative and/or pro-apoptotic and/or chemosensitizing properties. Accordingly, the compounds of the present invention are useful for treatment of hyperproliferative disorders, in particular cancer. Therefore the compounds of the present invention are used in the production of an anti-proliferative and/or pro-apoptotic and/or chemosensitizing effect in mammals such as human being suffering from a hyperproliferative disorders, like cancer.

Compounds according to the present invention exhibit anti-proliferative and/or pro-apoptotic properties in mammals such as humans due to inhibition of metabolic activity of cancer cells which are able to survive despite of unfavourable growth conditions such as glucose depletion, hypoxia or other chemo stress.

Thus, the compounds according to the present invention are for treating, ameliorating or preventing diseases of benign or malignant behaviour as described herein, such as e.g. for inhibiting cellular neoplasia.

Neoplasia in the present invention is used as known to persons skilled in the art. A benign neoplasia is described by hyperproliferation of cells, incapable of forming an aggressive, metastasizing tumor in-vivo. In contrast, a malignant neoplasia is described by cells with multiple cellular and biochemical abnormalities, capable of forming a systemic disease, for example forming tumor metastasis in distant organs.

The compounds according to the present invention can be preferably used for the treatment of malignant neoplasia. Examples of malignant neoplasia treatable with the compounds according to the present invention include solid and hematological tumors. Solid tumors can be exemplified by tumors of the breast, bladder, bone, brain, central and peripheral nervous system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva. Malignant neoplasias include inherited cancers exemplified by Retinoblastoma and Wilms tumor. In addition, malignant neoplasias include primary tumors in said organs and corresponding secondary tumors in distant organs ("tumor metastases"). Hematological tumors can be exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, and cancers of unknown primary site as well as AIDS related malignancies.

It is noted that a malignant neoplasia does not necessarily require the formation of metastases in distant organs. Certain tumors exert devastating effects on the primary organ itself through their aggressive growth properties. These can lead to the destruction of the tissue and organ structure finally resulting in failure of the assigned organ function and death.

Drug resistance is of particular importance for the frequent failure of standard cancer therapeutics. This drug resistance is caused by various cellular and molecular mechanisms. One aspect of drug resistance is caused by constitutive activation of anti-apoptotic survival signals with PKB/Akt as a key signalling kinase. Inhibition of the Pi3K/Akt pathway leads to a resensitization towards standard chemotherapeutic or target specific cancer therapeutics. As a consequence, the commercial applicability of the compounds according to the present invention is not limited to 1^{st} line treatment of cancer patients. In a preferred embodiment, cancer patients with resistance to cancer chemotherapeutics or target specific anti-cancer drugs are also amenable for treatment with these compounds for e.g. 2^{nd} or 3^{rd} line treatment cycles. In particular, the compounds according to the present invention might be used in combination with standard chemotherapeutic or targeted drugs to resensitize tumors towards these agents.

In the context of their properties, functions and utilities mentioned herein, the compounds according to the present invention are distinguished by unexpected valuable and desirable effects related therewith, such as e.g. superior therapeutic window, superior bioavailability (such as e.g. good oral absorption), low toxicity and/or further beneficial effects related with their therapeutic and pharmaceutical qualities.

Compounds according to the present invention are for treatment, prevention or amelioration of the diseases of benign and malignant behavior as described before, such as e.g. benign or malignant neoplasia, particularly cancer, especially a cancer that is sensitive to Pi3K/Akt pathway inhibition.

The present invention further includes the use of the compounds for treating, prevention or amelioration mammals, including humans, which are suffering from one of the abovementioned conditions, illnesses, disorders or diseases. The use is characterized in that a pharmacologically active and therapeutically effective and tolerable amount of one or more of compounds according to the present invention is administered to the subject in need of such treatment.

The present invention further includes the compounds of the invention for use in treating, preventing or ameliorating diseases responsive to inhibition of the Pi3K/Akt pathway, in a mammal, including human, comprising administering a pharmacologically active and therapeutically effective and tolerable amount of one or more of the compounds according to the present invention to said mammal.

The present invention further includes the compounds of the invention for use in treating hyperproliferative diseases of benign or malignant behaviour and/or disorders responsive to induction of apoptosis, such as e.g. cancer, particularly any of those cancer diseases described above, in a mammal, comprising administering a pharmacologically active and therapeutically effective and tolerable amount of one or more of the compounds according to the present invention to said mammal.

The present invention further includes the compounds of the invention for use in inhibiting cellular hyperproliferation or arresting aberrant cell growth in a mammal, comprising administering a pharmacologically active and therapeutically effective and tolerable amount of one or more of the compounds according to the present invention to said mammal.

The present invention further includes the compounds of the invention for use in inducing apoptosis in the therapy of beningn or malignant neoplasia, particularly cancer, comprising administering a pharmacologically active and therapeutically effective and tolerable amount of one or more of the compounds according to the present invention to a subject in need of such therapy.

The present invention further includes the compounds of the invention for use in inhibiting protein kinase activity in cells comprising administering a pharmacologically active and therapeutically effective and tolerable amount of one or more of the compounds according to the present invention to a patient in need of such therapy.

The present invention further includes the compounds of the invention for use in sensitizing towards chemotherapeutic or target-specific anti-cancer agents in a mammal, comprising administering a pharmacologically active and therapeutically effective and tolerable amount of one or more of the compounds according to the present invention to said mammal.

The present invention further includes the compounds of the invention foruse in treating benign and/or malignant neoplasia, particularly cancer, in a mammal, including human, comprising administering a pharmacologically active and therapeutically effective and tolerable amount of one or more of the compounds according to the present invention to said mammal.

The present invention further relates to the use of the compounds for the production of pharmaceutical compositions, which are employed for the treatment, prophylaxis, and/or amelioration of one or more of the illnesses mentioned.

The present invention further relates to the use of the compounds for the manufacture of pharmaceutical compositions for treating, preventing or ameliorating hyperproliferative diseases and/or disorders responsive to the induction of apoptosis, such as e.g. beningn or malignant neoplasia, in particular cancer.

The present invention further relates to the use of the compounds according to this invention for the production of pharmaceutical compositions for treating, preventing or ameliorating benign or malignant neoplasia, particularly cancer, such as e.g. any of those cancer diseases described above.

The invention further relates to a compound according to the invention or a pharmaceutically acceptable salt thereof, for use in the treatment and/or prophylaxis of (hyper)proliferative diseases and/or disorders responsive to induction of apoptosis, which include benign neoplasia and malignant neoplasia, including cancer.

The invention further related to the use of a compound according to the invention or a pharmaceutically acceptable salt thereof, for the production of a pharmaceutical composition for the treatment, prevention or amelioration of a disease mediated by a dysregulated function of a single protein kinase or multiple protein kinases and/or disorders responsive to the induction of apoptosis.

The invention further relates to a pharmaceutical composition, comprising a compound according to the invention or a pharmaceutically acceptable salt thereof, for the treatment and/or prophylaxis of (hyper)proliferative diseases and/or disorders responsive to induction of apoptosis, which include benign neoplasia and malignant neoplasia, including cancer.

The present invention further relates to the use of compounds and pharmaceutically acceptable salts according to the present invention for the manufacture of pharmaceutical compositions, which can be used for sensitizing towards chemotherapeutic and/or target specific anti-cancer agents.

The present invention further relates to the use of compounds according to the present invention for the manufacture of pharmaceutical compositions, which can be used for sensitizing towards radiation therapy of those diseases mentioned herein, particularly cancer.

The present invention further relates to the use of the compounds according to the present invention for the manufacture of pharmaceutical compositions, which can be used in the treatment of diseases sensitive to protein kinase inhibitor therapy and different to cellular neoplasia. These non-malignant diseases include, but are not limited to benign prostate hyperplasia, neurofibromatosis, dermatoses, and myelodysplastic syndromes.

The present invention further relates to pharmaceutical compositions comprising one or more of the compounds according to this invention and a pharmaceutically acceptable carrier or diluent.

The present invention further relates to pharmaceutical compositions comprising one or more of the compounds according to this invention and pharmaceutically acceptable auxiliaries and/or excipients.

The pharmaceutical compositions according to this invention are prepared by processes, which are known per se and familiar to the person skilled in the art. As pharmaceutical compositions, the compounds of the invention (=active compounds) are either employed as such, or preferably in combination with suitable pharmaceutical auxiliaries and/or excipients, e.g. in the form of tablets, coated tablets, dragees, pills, cachets, granules, capsules, caplets, suppositories, patches (e.g. as TTS), emulsions (such as e.g. micro-emulsions or lipid emulsions), suspensions (such as e.g. nano suspensions), gels, solubilisates or solutions (e.g. sterile solutions), or encapsuled in liposomes or as beta-cyclodextrine or beta-cyclodextrin derivative inclusion complexes or the like, the active compound content advantageously being between 0.1 and 95% and where, by the appropriate choice of the auxiliaries and/or excipients, a pharmaceutical administration form (e.g. a delayed release form or an enteric form) exactly suited to the active compound and/or to the desired onset of action can be achieved.

The person skilled in the art is familiar with auxiliaries, vehicles, excipients, diluents, carriers or adjuvants which are suitable for the desired pharmaceutical formulations, preparations or compositions on account of his/her expert knowledge. In addition to solvents, gel formers, ointment bases and other active compound excipients, for example antioxidants, dispersants, emulsifiers, preservatives, solubilizers (such as e.g. polyoxyethylenglyceroltriricinoleat 35, PEG 400, Tween 80, Captisol, Solutol HS15 or the like), colorants, complexing agents, permeation promoters, stabilizers, fillers, binders, thickeners, disintegrating agents, buffers, pH regulators (e.g. to obtain neutral, alkaline or acidic formulations), polymers, lubricants, coating agents, propellants, tonicity adjusting agents, surfactants, flavorings, sweeteners or dyes, can be used.

In particular, auxiliaries and/or excipients of a type appropriate to the desired formulation and the desired mode of administration are used.

The administration of the compounds, pharmaceutical compositions or combinations according to the invention may be performed in any of the generally accepted modes of administration available in the art. Illustrative examples of suitable modes of administration include intravenous, oral, nasal, parenteral, topical, transdermal and rectal delivery. Oral and intravenous deliveries are preferred.

Generally, the pharmaceutical compositions according to the invention can be administered such that the dose of the active compound is in the range customary for Pi3K/Akt pathway inhibitors. In particular, a dose in the range of from 0.01 to 4000 mg of the active compound per day is preferred for an average adult patient having a body weight of 70 kg. In this respect, it is to be noted that the dose is dependent, for example, on the specific compound used, the species treated, age, body weight, general health, sex and diet of the subject treated, mode and time of administration, rate of excretion, severity of the disease to be treated and drug combination.

The pharmaceutical composition can be administered in a single dose per day or in multiple subdoses, for example, 2 to 4 doses per day. A single dose unit of the pharmaceutical composition can contain e.g. from 0.01 mg to 4000 mg, preferably 0.1 mg to 2000 mg, more preferably 0.5 to 1000 mg, most preferably 1 to 500 mg, of the active compound. Furthermore, the pharmaceutical composition can be adapted to weekly, monthly or even more infrequent administration, for example by using an implant, e.g. a subcutaneous or intramuscular implant, by using the active compound in form of a sparingly soluble salt or by using the active compound coupled to a polymer.

The choice of the optimal dosage regime and duration of medication, particularly the optimal dose and manner of administration of the active compounds necessary in each case can be determined by a person skilled in the art.

The present invention further relates to combinations comprising one or more first active ingredients selected from the compounds of the invention and one or more second active ingredients selected from chemotherapeutic anti-cancer agents and target-specific anti-cancer agents e.g. for treating, preventing or ameliorating diseases responsive or sensitive to inhibition of the Pi3K/Akt pathway, such as hyperproliferative diseases of benign or malignant behaviour and/or disorders responsive to the induction of apoptosis, particularly cancer, such as e.g. any of those cancer diseases described above.

The invention further relates to the use of a pharmaceutical composition comprising one or more of the compounds according to this invention as sole active ingredient(s) and a pharmaceutically acceptable carrier or diluent in the manufacture of pharmaceutical products for the treatment and/or prophylaxis of the illnesses mentioned above.

Depending upon the particular disease, to be treated or prevented, additional therapeutic active agents, which are normally administered to treat or prevent that disease, may optionally be coadministered with the compounds according to this invention. As used herein, additional therapeutic agents that are normally administered to treat or prevent a particular disease are known as appropriate for the disease being treated.

The anti-cancer agents mentioned herein above as combination partners of the compounds according to this invention are meant to include pharmaceutically acceptable derivatives thereof, such as e.g. their pharmaceutically acceptable salts.

The person skilled in the art is aware of the total daily dosage(s) and administration form(s) of the additional therapeutic agent(s) coadministered. Said total daily dosage(s) can vary within a wide range.

In practicing the present invention, the compounds according to this invention may be administered in combination therapy separately, sequentially, simultaneously, concurrently or chronologically staggered (such as e.g. as combined unit dosage forms, as separate unit dosage forms, as adjacent discrete unit dosage forms, as fixed or non-fixed combinations, as kit-of-parts or as admixtures) with one or more standard therapeutics (chemotherapeutic and/or target specific anti-cancer agents), in particular art-known anti-cancer agents, such as any of e.g. those mentioned above.

In this context, the present invention further relates to a combination comprising a first active ingredient, which is at least one compound according to this invention, and a second active ingredient, which is at least one art-known anti-cancer agent, such as e.g. one or more of those mentioned herein above, for separate, sequential, simultaneous, concurrent or chronologically staggered use in therapy, such as e.g. in therapy of any of those diseases mentioned herein.

The present invention further relates to a pharmaceutical composition comprising a first active ingredient, which is at least one compound according to this invention, and a second active ingredient, which is at least one art-known anti-cancer agent, such as e.g. one or more of those mentioned herein above, and, optionally, a pharmaceutically acceptable carrier or diluent, for separate, sequential, simultaneous, concurrent or chronologically staggered use in therapy.

The present invention further relates to a combination product comprising
a.) at least one compound according to this invention formulated with a pharmaceutically acceptable carrier or diluent, and
b.) at least one art-known anti-cancer agent, such as e.g. one or more of those mentioned herein above, formulated with a pharmaceutically acceptable carrier or diluent.

The present invention further relates to a kit-of-parts comprising a preparation of a first active ingredient, which is a compound according to this invention, and a pharmaceutically acceptable carrier or diluent; a preparation of a second active ingredient, which is an art-known anti-cancer agent, such as one of those mentioned above, and a pharmaceutically acceptable carrier or diluent; for simultaneous, concurrent, sequential, separate or chronologically staggered use in therapy. Optionally, said kit comprises instructions for its use in therapy, e.g. to treat hyperproliferative diseases and diseases responsive or sensitive to inhibition of the Pi3K/Akt pathway, such as e.g. beningn or malignant neoplasia, particularly cancer, more precisely, any of those cancer diseases described above.

The present invention further relates to a combined preparation comprising at least one compound according to this invention and at least one art-known anti-cancer agent for simultaneous, concurrent, sequential or separate administration.

The present invention further relates to combinations, compositions, formulations, preparations or kits according to the present invention having Pi3K/Akt pathway inhibitory activity.

In addition, the present invention further relates to a method for treating in combination therapy hyperproliferative diseases and/or disorders responsive to the induction of apoptosis, such as e.g. cancer, in a patient comprising administering a combination, composition, formulation, preparation or kit as described herein to said patient in need thereof.

In addition, the present invention further relates to a method for treating hyperproliferative diseases of benign or malignant behaviour and/or disorders responsive to the induction of apoptosis, such as e.g. cancer, in a patient comprising administering in combination therapy separately, simultaneously, concurrently, sequentially or chronologically staggered a pharmaceutically active and therapeutically effective and tolerable amount of a pharmaceutical composition, which comprises a compound according to this invention and a pharmaceutically acceptable carrier or diluent, and a pharmaceutically active and therapeutically effective and tolerable amount of one or more art-known anti-cancer agents, such as e.g. one or more of those mentioned herein, to said patient in need thereof.

In further addition, the present invention relates to a method for treating, preventing or ameliorating hyperproliferative diseases and/or disorders responsive to induction of apoptosis, such as e.g. benign or malignant neoplasia, e.g. cancer, particularly any of those cancer diseases mentioned herein, in a patient comprising administering separately, simultaneously, concurrently, sequentially or chronologically staggered to said patient in need thereof an amount of a first active compound, which is a compound according to the present invention, and an amount of at least one second active compound, said at least one second active compound being a standard therapeutic agent, particularly at least one art-known anti-cancer agent, such as e.g. one or more of those chemotherapeutic and target-specific anti-cancer agents mentioned herein, wherein the amounts of the first active compound and said second active compound result in a therapeutic effect.

In yet further addition, the present invention relates to a method for treating, preventing or ameliorating hyperproliferative diseases and/or disorders responsive to induction of apoptosis, such as e.g. benign or malignant neoplasia, e.g. cancer, particularly any of those cancer diseases mentioned herein, in a patient comprising administering a combination according to the present invention.

In addition, the present invention further relates to the use of a composition, combination, formulation, preparation or kit according to this invention in the manufacture of a pharmaceutical product, such as e.g. a commercial package or a medicament, for treating, preventing or ameliorating hyperproliferative diseases, such as e.g. cancer, and/or disorders responsive to the induction of apoptosis, particularly those diseases mentioned herein, such as e.g. malignant or benign neoplasia.

The present invention further relates to a commercial package comprising one or more compounds of the present invention together with instructions for simultaneous, concurrent, sequential or separate use with one or more chemotherapeutic and/or target specific anti-cancer agents, such as e.g. any of those mentioned herein.

The present invention further relates to a commercial package consisting essentially of one or more compounds of the present invention as sole active ingredient together with instructions for simultaneous, concurrent, sequential or separate use with one or more chemotherapeutic and/or target specific anti-cancer agents, such as e.g. any of those mentioned herein.

The present invention further relates to a commercial package comprising one or more chemotherapeutic and/or target specific anti-cancer agents, such as e.g. any of those mentioned herein, together with instructions for simultaneous, concurrent, sequential or separate use with one or more compounds according to the present invention.

The compositions, combinations, preparations, formulations, kits or packages mentioned in the context of the combination therapy according to this invention may also include more than one of the compounds according to this invention and/or more than one of the art-known anti-cancer agents mentioned.

The first and second active ingredient of a combination or kit-of-parts according to this invention may be provided as separate formulations (i.e. independently of one another), which are subsequently brought together for simultaneous, concurrent, sequential, separate or chronologically staggered use in combination therapy; or packaged and presented together as separate components of a combination pack for simultaneous, concurrent, sequential, separate or chronologically staggered use in combination therapy.

The type of pharmaceutical formulation of the first and second active ingredient of a combination or kit-of-parts according to this invention can be according, i.e. both ingredients are formulated in separate tablets or capsules, or can be different, i.e. suited for different administration forms, such as e.g. one active ingredient is formulated as tablet or capsule and the other is formulated for e.g. intravenous administration.

The amounts of the first and second active ingredients of the combinations, compositions or kits according to this invention may together comprise a therapeutically effective amount for the treatment, prophylaxis or amelioration of a hyperproliferative diseases and/or a disorder responsive to the induction of apoptosis, particularly one of those diseases mentioned herein, such as e.g. malignant or benign neoplasia, especially cancer, like any of those cancer diseases mentioned herein.

In addition, compounds according to the present invention can be used in the pre- or post-surgical treatment of cancer.

In further addition, compounds of the present invention can be used in combination with radiation therapy.

A combination according to this invention can refer to a composition comprising both the compound(s) according to this invention and the other active anti-cancer agent(s) in a fixed combination (fixed unit dosage form), or a medicament pack comprising the two or more active ingredients as discrete separate dosage forms (non-fixed combination). In case of a medicament pack comprising the two or more active ingredients, the active ingredients are preferably packed into blister cards, which are suited for improving compliance.

Each blister card preferably contains the medicaments to be taken on one day of treatment. If the medicaments are to be taken at different times of day, the medicaments can be disposed in different sections on the blister card according to the different ranges of times of day at which the medicaments are to be taken (for example morning and evening or morning, midday and evening). The blister cavities for the medicaments to be taken together at a particular time of day are accommodated in the respective range of times of day. The various times of day are, of course, also put on the blister in a clearly visible way. It is also possible, of course, for example to indicate a period in which the medicaments are to be taken, for example stating the times.

The daily sections may represent one line of the blister card, and the times of day are then identified in chronological sequence in this column.

Medicaments which must be taken together at a particular time of day are placed together at the appropriate time on the blister card, preferably a narrow distance apart, allowing them to be pushed out of the blister easily, and having the effect that removal of the dosage form from the blister is not forgotten.

The following examples illustrate the invention in greater detail, without restricting it. Further compounds according to the invention, of which the preparation is not explicitly described, can be prepared in an analogous way.

The compounds, which are mentioned in the examples and the salts thereof represent preferred embodiments of the invention as well as a claim covering all subcombinations of the residues of the compound of formula (I) as disclosed by the specific examples.

The term "according to" within the experimental section is used in the sense that the procedure referred to is to be used "analogously to".

### Experimental Part

The following table lists the abbreviations used in this paragraph and in the Examples section as far as they are not explained within the text body. NMR peak forms are stated as they appear in the spectra, possible higher order effects have not been considered. Chemical names were generated using AutoNom2000 as implemented in MDL ISIS Draw. In some cases generally accepted names of commercially available reagents were used in place of AutoNom2000 generated names. The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography. In some cases, the compounds may be purified by preparative HPLC. In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the persion skilled in the art, or be used as salts in subsequent biological assays. It is to be understood that the specific form (e.g. salt, free base...) of a compound of the present invention as isolated as described herein is not necessarily the only form in which said compound can be applied to a biological assay in order to quantify the specific biological activity.

| **Abbreviation** | **meaning** |
|---|---|
| Ac | acetyl |
| ACN | acetonitrile |
| br | broad |
| d | doublet |
| DBU | diaza(1,3)bicyclo[5.4.0]undecane |
| dd | doublet of doublet |
| DCM | dichloromethane |
| DIBAL | diisobutylaluminiumhydride |
| DMAP | 4-dimethylaminopyridine |
| DMF | N,N-dimethylformamide |
| DMSO | dimethyl sulfoxide |
| Eq. | equivalent |
| ESI | electrospray ionisation |
| EtOAc | ethylacetate |
| HBTU | 2-(1H-benzotriazol-1-yl)-1,1,3,3-tetramethyluroniumhexafluorophosphate |
| HPLC | high performance liquid chromatography |
| LC-MS | liquid chromatography mass spectrometry |
| m | Multiplet |
| MS | mass spectrometry |
| NBS | N-bromosuccinimide |
| NIS | N-iodosuccinimide |
| NMP | N-methylpyrrolidinone |
| NMR | nuclear magnetic resonance spectroscopy: chemical shifts (δ) are given in ppm. |
| q | quartet |
| qn | quintet |
| rf | at reflux |
| r.t. or rt | room temperature |
| RT | retention time (in minutes), measured by UPLC with a standard procedure, unless stated |
| s | singlet |
| t | triplet |
| TLC | thin layer chromatography |
| THF | Tetrahydrofuran |

Other abbreviations have their meanings customary per se to the skilled person.

The various aspects of the invention described in this application are illustrated by the following examples which are not meant to limit the invention in any way.

### Examples

### UPLC-MS Standard Procedure

Analytical UPLC-MS was performed under the following conditions unless otherwise stated.
Instrument: Waters Acquity UPLC-MS ZQ4000; Column: Acquity UPLC BEH C18 1.7 50x2.1 mm; Eluent A: water + 0.05% formic acid, Eluent B: acetonitrile + 0.05% formic acid; Gradient: 0-1.6 min 1-99% B, 1.6-2.0 min 99% B; Flow rate 0.8 ml/min; Temperature: 60 °C; Injection: 2 µl; DAD scan: 210-400 nm.
The masses (m/z) are reported from the positive mode electrospray ionisation unless the negative mode is indicated (ES-).

### Intermediate Examples

### Intermediate Example 1.0: 4-(5-methyl-8-phenyl-imidazo[1,2-c]pyrimidin-7-yl)-benzaldehyde

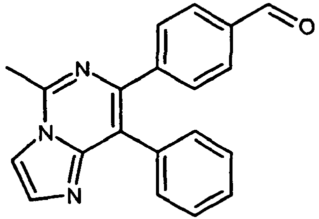

### Step 1: 2-methyl-5-phenyl-pyrimidine-4,6-diol

To a mixture of NaOEt (19.7 g, 0.275 mol) in EtOH (140 mL) was added 2-phenyl-malonamide (10 g, 55 mmol). To the resulting thick suspension was added EtOAc (90.4 mL, 110 mmol) dropwise. The mixture was diluted with EtOH (60 mL) and heated under strong stirring for 2 h at 50 °C. The reaction was cooled to 0 °C and quenched with ice cold water (175 mL). The resulting solution was treated with concentrated aqueous hydrochloric acid (90 mL) and the resulting precipitate was filtered off, washed with 0.5 M aqueous hydrochloric acid (40 mL) and dried to give the crude title compound which was used without further purification in the next step.

### Step 2: 4,6-dibromo-2-methyl-5-phenyl-pyrimidine

A mixture of 2-methyl-5-phenyl-pyrimidine-4,6-diol (3.69 g) and POBr₃ (10.4 g, 2 Eq.) was heated at 180 °C for 30 minutes. On cooling to 0 °C, the reaction was quenched with ice cold water and stirred vigorously before the resulting suspension was filtered. The residue was co-evaporated with toluene and dried to give the crude title compound which was used without further purification in the next step.

### Step 3: 6-bromo-2-methyl-5-phenyl-pyrimidin-4-ylamine

A mixture of 4,6-dibromo-2-methyl-5-phenyl-pyrimidine (3.2 g) and a solution of ammonia in EtOH (2M, 50 mL) was heated at 100 °C overnight. Reaction control (LC-MS) indicated incomplete conversion, so aqueous ammonia (25%, 10 Eq.) was added and the mixture heated at 120 °C. On cooling, the volatiles were removed in vacuo, the residue co-evaporated with toluene and dried to give the crude title compound which was used without further purification in the next step.

### Step 4: 4-(6-amino-2-methyl-5-phenyl-pyrimidin-4-yl)-benzaldehyde

6-bromo-2-methyl-5-phenyl-pyrimidin-4-ylamine (2.59 g) was suspended in dioxane (65 mL), and 4-formylphenylboronic acid (1.90 g, 9.8 mmol), an aqueous solution of Cs₂CO₃ (2M, 39.2 mmol) and Pd(dppf)Cl₂ (0.417 g, 0.5 mmol) were added successively under a nitrogen stream. The reaction was heated under nitrogen at 115 °C for 6 days. On cooling the raction was stirred at rt for a further 2 days before the reaction was filtered through a silica gel pad and the filtrate partitioned between aqueous NH₄Cl and EtOAc. The aqueous phase was extracted with CH₂Cl₂ and the organic phases washed with brine, dried (MgSO₄) and concentrated in vacuo. Purification was achieved by chromatography on silica gel to give the title compound.

### Step 5: 4-(5-methyl-8-phenyl-imidazo[1,2-c]pyrimidin-7-yl)-benzaldehyde

4-(6-amino-2-methyl-5-phenyl-pyrimidin-4-yl)-benzaldehyde (500 mg, 1.7 mmol) was suspended in EtOH (4 mL), treated with chloroacetaldehyde (50% in water, 10 Eq.) and the mixture heated at 100 °C under microwave irradiation for 45 minutes. On cooling, the mixture was concentrated in vacuo and co-evaporated with toluene. Purification was achieved by chromatography on silica gel (eluant: toluene:dioxane 7:3) to give the title compound.

### Intermediate Example 2.0: 4-(8-phenyl-imidazo[1,2-c]pyrimidin-7-yl)-benzaldehyde

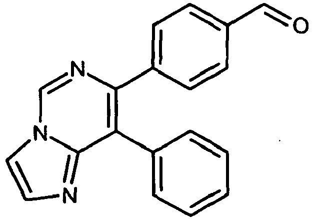

### Step 1: 5-phenyl-pyrimidine-4,6-diol

To a mixture of NaOEt (79 g, 1.1 mol) in EtOH (650 mL) was added 2-phenyl-malonamide (40 g, 0.22 mol). To the resulting suspension was added ethyl formate (36 mL, 0.44 mol) dropwise. The mixture was heated for 2 h at 50 °C before the reaction was cooled to 0 °C and quenched with water (700 mL) and aqueous hydrochloric acid (6N, 360 mL). The resulting precipitate was filtered off, washed with 0.5 N aqueous hydrochloric acid and dried to give the crude title compound which was used without further purification in the next step.
¹H NMR (300MHz, d6-DMSO): δ 11.95 (br s, 2H), 8.07 (s, 1 H), 7.2-7.5 (m, 5H) ppm.

### Step 2: 4,6-dibromo-5-phenyl-pyrimidine

A mixture of 5-phenyl-pyrimidine-4,6-diol (34.8 g) and POBr₃ (106 g, 2 Eq.) was heated at 180 °C for 40 minutes. On cooling to rt, the reaction was quenched with water (500 mL) carefully. The precipitate was filtered and dried to give the crude title compound which was used without further purification in the next step.

### Step 3: 6-bromo-5-phenyl-pyrimidin-4-ylamine

A mixture of 4,6-dibromo-5-phenyl-pyrimidine (52.5 g) and a solution of ammonia in EtOH (2M, 835 mL) was heated at reflux for 30 hours. Additional portions of 15 Eq. of ammonia in MeOH (7M) were added after 2, 6 and 20 hours. On cooling, the volatiles were removed *in vacuo,* and the residue suspended in water (300 mL). The solid was filtered off and dried to give the crude title compound which was used without further purification in the next step.

### Step 4: 4-(6-amino-5-phenyl-pyrimidin-4-yl)-benzaldehyde

To a mixture of 6-bromo-5-phenyl-pyrimidin-4-ylamine (15 g, 60 mmol) and 4-formylphenylboronic acid (11.7 g, 78 mmol), in dioxane (400 mL), was added a solution of Cs₂CO₃ (2M, 120 mL) and Pd(dppf)Cl₂ (2.4 g, 3 mmol). The reaction was heated at reflux under an inert gas atmosphere for 18 hours. On cooling, the reaction was quenched by partitioning between water and EtOAc. The aqueous phase was extracted with EtOAc and the combined organic phases dried (MgSO₄) and concentrated *in vacuo.* Purification was achieved by chromatography on silica gel to give the title compound.
¹H NMR (300MHz, d6-DMSO): δ 9.92 (s, 1 H), 8.5 (s, 1 H), 7.71 (d, 2H), 7.32-7.42 (m, 5H), 7.17 (d, 2H) ppm.

**Step 5: 4-(8-phenyl-imidazo[1,2-c]pyrimidin-7-yl)-benzaldehyde**

4-(6-amino-2-methyl-5-phenyl-pyrimidin-4-yl)-benzaldehyde (7.5 g, 27.2 mmol) was suspended in EtOH (55 mL), treated with chloroacetaldehyde (50% in water, 272 mmol) and the mixture heated at 100 °C under microwave irradiation for 20 minutes. On cooling, the mixture was concentrated *in vacuo* and purification was achieved by chromatography on silica gel to give the title compound.
¹H NMR (300MHz, d6-DMSO): δ 9.98 (s, 1 H), 9.57 (s, 1 H), 8.2 (s, 1 H), 7.8 (d, 2H), 7.73 (s, 1 H), 7.55 (d, 2H), 7.4 (m, 5H) ppm.

### Intermediate Example 3.0: 4-(8-phenyl-[1,2,4]triazolo[4,3-c]pyrimidin-7-yl)-benzaldehyde

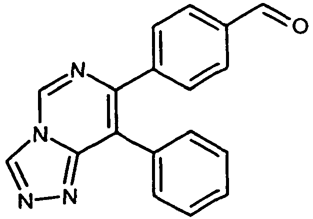

### Step 1: (6-bromo-5-phenyl-pyrimidin-4-yl)-hydrazine

A mixture of 4,6-dibromo-5-phenyl-pyrimidine (20 g) and EtOH (150 mL) was treated with hydrazine hydrate (9.56 g, 191 mmol) and the reaction heated at 50 °C for 45 minutes. The reaction was concentrated in vacuo and the residue co-evaporated with toluene (2x) and dried to give the crude title compound which was used without further purification in the next step.
MS (M+1): 265.0, 267.0 (Br isotopes)

### Step 2: 7-bromo-8-phenyl-[1,2,4]triazolo[4,3-c]pyrimidine

(6-bromo-5-phenyl-pyrimidin-4-yl)-hydrazine (11 g, 41.5 mmol) was suspended in triethylorthoformate (300 mL) and the reaction heated at reflux overnight. The volatiles were almost completely removed *in vacuo,* the resulting suspension was chilled and the solid removed by filtration. The filtrate was concentrated *in vacuo* and the resulting residue recrystallised from EtOH to give the title compound.
¹H NMR (300MHz, d6-DMSO): δ 9.78 (s, 1 H), 8.64 (s, 1 H), 7.51-7.6 (m, 5H).
MS (M+1): 275.1, 277.1 (Br isotopes)

### Step 3: 4-(8-phenyl-[1,2,4]triazolo[4,3-c]pyrimidin-7-yl)-benzaldehyde

A mixture of 7-bromo-8-phenyl-[1,2,4]triazolo[4,3-c]pyrimidine (0.55 g, 2 mmol), 4-formylphenylboronic acid (0.39 g, 2.6 mmol) and Cs₂CO₃ (2.6 g, 8 mmol) in dioxane (20 mL) and water (2 mL) was purged with nitrogen and treated with Pd(dppf)Cl₂ (82 mg, 0.1 mmol). The reaction was heated at reflux overnight. On cooling, the reaction was filtered through Celite, washing through with EtOH (200 mL) and the mixture was diluted with aqueous NH₄Cl and extracted twice with EtOAc. The combined organic phases were washed with brine, dried and concentrated *in vacuo.* Purification was achieved by chromatography to give the title compound.
¹H NMR (300MHz, CDCl₃): δ 10.0 (s, 1H), 9.5 (s, 1H), 7.8 (d, 2H), 7.63 (d, 2H), 7.38-7.45 (m, 5H) ppm.
MS (M+1): 301.2.

### Intermediate Example 4.0: 4-(3-methyl-8-phenyl-[1,2,4]triazolo[4,3-c]pyrimidin-7-yl)-benzaldehyde

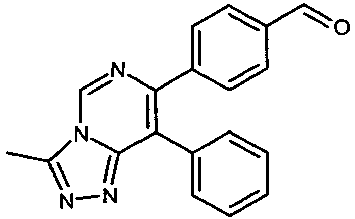

### Step 1: 7-bromo-3-methyl-8-phenyl-[1,2,4]triazolo[4,3-c]pyrimidine

(6-bromo-5-phenyl-pyrimidin-4-yl)-hydrazine (10 g, 37.7 mmol) was suspended in triethylorthoacetate (300 mL) and the reaction heated at reflux overnight. The volatiles were almost completely removed *in vacuo,* the resulting suspension was chilled and the solid removed by filtration. The filtrate was concentrated *in vacuo* and the resulting residue recrystallised from EtOH to give the title compound.
¹H NMR (300MHz, d6-DMSO): δ 9.62 (s, 1H), 7.55 (m, 5H), 2.49 (signal partially obscured by solvent).
MS (M+1): 289.1, 291.1 (Br isotopes)

### Step 2: 4-(3-methyl-8-phenyl-[1,2,4]triazolo(4,3-c]pyrimidin-7-yl)-benzaldehyde

A mixture of 7-bromo-3-methyl-8-phenyl-[1,2,4]triazolo[4,3-c]pyrimidine (0.825 g, 3 mmol), 4-formylphenylboronic acid (0.584 g, 3.9 mmol) and Cs₂CO₃ (3.9 g, 12 mmol) in dioxane (30 mL) and water (3 mL) was purged with nitrogen and treated with Pd(dppf)Cl₂ (123 mg, 0.15 mmol). The reaction was heated at reflux overnight. On cooling, the reaction was filtered through Celite, washing through with EtOH (200 mL) and the mixture was diluted with aqueous NH₄Cl and extracted twice with EtOAc. The combined organic phases were washed with brine, dried and concentrated *in vacuo.* Purification was achieved by chromatography to give the title compound.
¹H NMR (300MHz, d6-DMSO): δ 10.0 (s, 1H), 9.8 (s, 1H), 7.82 (d, 2H), 7.6 (d, 2H), 7.4 (m, 5H), 2.52 (signal partially obscured by solvent) ppm.
MS (M+1): 315.1.

### Intermediate Example 5.0: 2-(5-piperidin-4-yl-2H-[1,2,4]triazol-3-yl)-pyridine dihydrochloride (alternative procedures described in US4011218 or WO02005100344)

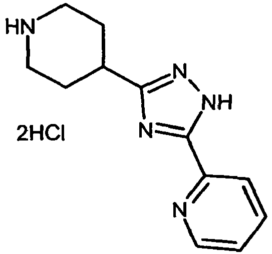

### Step 1: pyridine-2-carbohydrazonamide

A solution of pyridine-2-carbonitrile 20g (192 mmol), hydrazine hydrate (3 eq.) in ethanol (50 ml) was stirred at room temperature for 18 hrs. Reaction mass was then diluted with water, extracted with ethyl acetate, dried over anhydrous sodium sulphate and concentrated under vacuum to yield desired compound.
MS (M+1): 137.28
¹H NMR (300MHZ, CDCl₃): δ 8.53 (d, 1H, J=8 & 2.3 Hz), 8.02 (d, 1H, J=7.8& 2.1 Hz), 7.72 (t, 1H, J=8.2 & 2Hz), 7.29 (t, 1H, J=8.4 & 2.1 Hz), 5.42 (bs, 2H), 4.60 (bs, 2H) ppm.

### Step 2: tert-butyl 4-({(2Z)-2-[amino (pyridin-2-yl) methylidene] hydrazinyl} carbonyl) piperidine-1-carboxylate

To as solution of 1-(*tert*-butoxycarbonyl) piperidine-4-carboxylic acid 37g (167 mM) in dichloromethane (300ml) was added carbonyl diimidazole (1 eq.) in small portions over a period of 30 min. Pyridine-2-carbohydrazonamide was then added to the reaction mixture and stirred at room temperature for 3 hrs. Dichloromethane was evaporated and reaction mass was then stirred in water for 30 min. The precipitated solid was filtered and dried to afford the desired compound.
MS (M+1): 348.07
¹H NMR (300MHZ, CDCl3): δ10.75 (s, 1H), 8.56 (d, 1H, J=4.5Hz), 8.10 (d, 1H, J=8.3Hz), 7.75 (dt, 1H, J=8.2 & 1.3Hz), 7.34 (dt, 1H, J=7.9 & 1.5Hz), 4.18 (bs, 2H), 3.46 (s, 1H), 2.88 (t, 2H), 1.91 (m, 2H), 1.72 (m, 4H), 1.47 (s, 9H) ppm.

### Step 3: tert-butyl 4-[5-(pyridin-2-yl)-1H-1, 2,4-triazol-3-yl] piperidine-1-carboxylate

*Tert*-butyl 4-({(2Z)-2-[amino (pyridin-2-yl) methylidene] hydrazinyl} carbonyl) piperidine-1-carboxylate 45g (129 mmol) obtained in step 2 was melted at 220°C under nitrogen atmosphere for 1 hr. Reaction was then cooled to 150°C and ethanol added till the solid dissolved. The ethanol was then evaporated to get the desired crude compound, contaminated with 2-(5-piperidin-4-yl-2H-[1,2,4]triazol-3-yl)-pyridine.
MS (M+1): 330.5
¹H NMR (300MHZ, DMSO): δ 9.11 (s, 1 H), 8.74 (dd, 1 H, J= 4.8 & 1.3 Hz), 8.17 (dt,2H, J=8.2 & 2.1 Hz), 7.66 (dt, 1H, J=8.0 & 1.3 Hz), 3.34 (m, 2H), 3.18 (m, 1H), 3.06 (m, 2H), 2.20 (m, 2H), 1.99 (m, 2H), 1.28 (s, 9H) ppm.

### Step 4: 2-(5-piperidin-4-yl-2H-[1,2,4]triazol-3-yl)-pyridine dihydrochloride

To a solution of the crude *tert*-butyl 4-[5-(pyridin-2-yl)-1*H*-1,2,4-triazol-3-yl] piperidine-1-carboxylate 39g (111 mmol) in 50 ml methanol was added 100 ml solution of HCl in dioxane and stirred at room temperature for 3 hrs. The precipitated solid was then filtered and washed with cold acetonitrile to obtain 2-(5-piperidin-4-yl-2H-[1,2,4]triazol-3-yl)-pyridine dihydrochloride as white solid.
¹H NMR (300MHZ, DMSO): δ 9.11 (s, 1 H), 8.97 (s, 1H), 8.74 (dd, 1H, J= 4.8 & 1.3 Hz), 8.17 (dt,2H, J=8.2 & 2.1 Hz), 7.66 (dt, 1H, J=8.0 & 1.3 Hz), 3.34 (m, 2H), 3.18 (m, 1H), 3.06 (m, 2H), 2.20 (m, 2H), 1.99 (m, 2H) ppm.

### Intermediate Example 6.0: 2-(5-piperidin-4-yl-2H-[1,2,4]triazol-3-yl)-pyridine 1-oxide hydrochloride salt

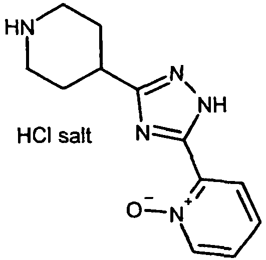

This intermediate was prepared according to 2-(5-piperidin-4-yl-2H-[1,2,4]triazol-3-yl)-pyridine dihydrochloride.
1 H-NMR (300 MHz, d6-DMSO): δ 9.15 (br s, 1 H), 8.93 (br s, 1 H), 8.43-8.46 (m, 1 H), 8.22-8.25 (m, 1 H), 7.5-7.53 (m, 1 H), 3.23-3.28 (m, 2H), 2.95-3.15 (m, 3H), 2.09-2.15 (m, 2H), 1.86-1.99 (m, 2H).

### Intermediate Example 7.0: 4-(5-piperidin-4-yl-2H-[1,2,4]triazol-3-yl)-pyridine hydrochloride salt

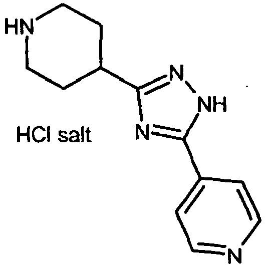

This intermediate was prepared according to 2-(5-piperidin-4-yl-2H-[1,2,4]triazol-3-yl)-pyridine dihydrochloride (intermediate example 5.0).

### Intermediate Example 8.0: 2-methyl-6-(5-piperidin-4-yl-2H-[1,2,4]triazol-3-yl)-pyridine hydrochloride salt

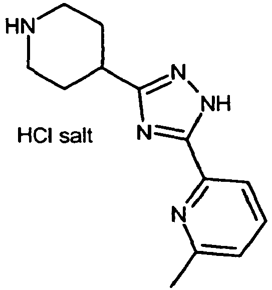

This intermediate was prepared according to 2-(5-piperidin-4-yl-2H-[1,2,4]triazol-3-yl)-pyridine dihydrochloride (intermediate example 5.0).
1H-NMR (300 MHz, d6-DMSO): δ 9.16 - 9.24 (m, 2H), 8.04 - 8.15 (m, 2H), 7.59 (d, 1 H), 3.15 - 3.30 (m, 3H), 2.96 - 3.06 (m, 2H), 2.64 (s, 3H), 2.14 - 2.18 (m, 2H), 1.93 - 2.04 (m, 2H) ppm.

### Intermediate Example 9.0: 5-methyl-2-(5-piperidin-4-yl-2H-[1,2,4]triazol-3-yl)-pyridine hydrochloride salt

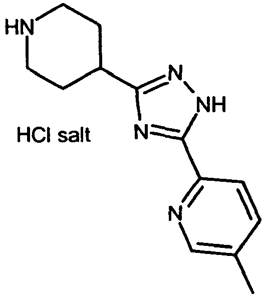

This intermediate was prepared according to 2-(5-piperidin-4-yl-2H-[1,2,4]triazol-3-yl)-pyridine dihydrochloride (intermediate example 5.0).
MS (M+1): 244
1 H-NMR (300 MHz, d6-DMSO): δ 8.70 - 8.66 (m, 2H), 8.54 (d, 1 H), 7.99 (s, 1 H), 7.46 (d, 1 H), 3.27 - 3.32 (m, 2H), 2.97 - 3.16 (m, 3H), [3H obscured by solvent], 2.12 - 2.16 (m, 2H), 1.86 -1.99 (m, 2H) ppm.

### Intermediate Example 10.0: 2,4-dimethyl-6-(5-piperidin-4-yl-2H-[1,2,4]triazol-3-yl)-pyridine hydrochloride salt

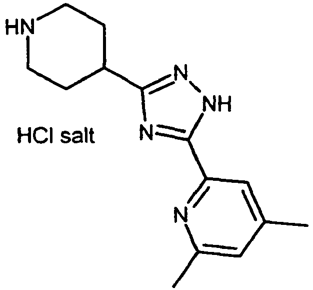

This intermediate was prepared according to 2-(5-piperidin-4-yl-2H-[1,2,4]triazol-3-yl)-pyridine dihydrochloride (intermediate example 5.0).
MS (M+1): 258.33;
UPLC-MS: RT = 0.47 min; m/z = 258.33.

### Intermediate Example 11.0: 2,3-Dimethyl-6-(5-piperidin-4-yl-2H-[1,2,4]triazol-3-yl)-pyridine hydrochloride salt

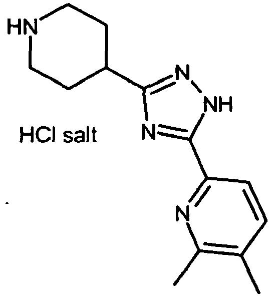

This intermediate was prepared according to 2-(5-piperidin-4-yl-2H-[1,2,4]triazol-3-yl)-pyridine dihydrochloride (intermediate example 5.0).
UPLC-MS: RT = 0.50 min; m/z = 258.27.

### Intermediate Example 12.0: 5-fluoro-2-(5-piperidin-4-yl-2H-[1, 2,4]triazol-3-yl)-pyridine hydrochloride salt

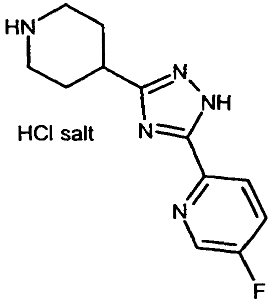

This intermediate was prepared according to 2-(5-piperidin-4-yl-2H-[1,2,4]triazol-3-yl)-pyridine dihydrochloride (intermediate example 5.0).
UPLC-MS: RT = 0.49 min; m/z = 248.22.

### Intermediate Example 13.0: 5-chloro-2-(5-piperidin-4-yl-2H-[1, 2,4]triazol-3-yl)-pyridine hydrochloride salt

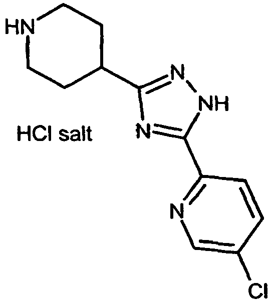

This intermediate was prepared according to 2-(5-piperidin-4-yl-2H-[1,2,4]triazol-3-yl)-pyridine dihydrochloride (intermediate example 5.0).

### Intermediate Example 14.0: 4-chloro-2-(5-piperidin-4-yl-2H-[1,2,4]triazol-3-yl)-pyridine hydrochloride salt

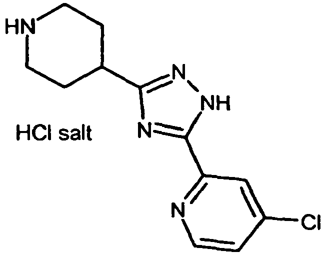

This intermediate was prepared according to 2-(5-piperidin-4-yl-2H-[1,2,4]triazol-3-yl)-pyridine dihydrochloride (intermediate example 5.0).
MS (M+1): 264.23;
UPLC-MS: RT = 0.58 min; m/z = 264.23.

### Intermediate Example 15.0: 2-Chloro-6-(5-piperidin-4-yl-2H-[1,2,4]triazol-3-yl)-pyridine hydrochloride salt

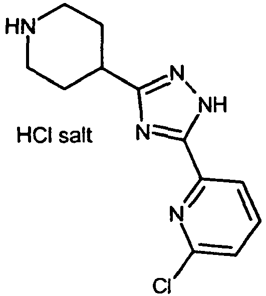

This intermediate was prepared according to 2-(5-piperidin-4-yl-2H-[1,2,4]triazol-3-yl)-pyridine dihydrochloride (intermediate example 5.0).
UPLC-MS: RT = 0.50 min; m/z = 264.21.

### Intermediate Example 16.0: 4-methoxy-2-(5-piperidin-4-yl-2H-[1,2,4]triazol-3-yl)-pyridine hydrochloride salt

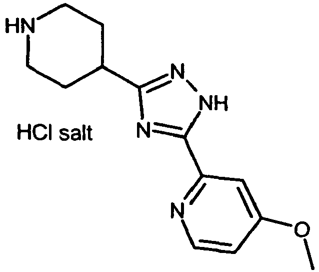

This intermediate was prepared according to 2-(5-piperidin-4-yl-2H-[1,2,4]triazol-3-yl)-pyridine dihydrochloride (intermediate example 5.0).

### Intermediate Example 17.0: 2-methoxy-5-(5-piperidin-4-yl-2H-[1,2,4]triazol-3-yl)-pyridine hydrochloride salt

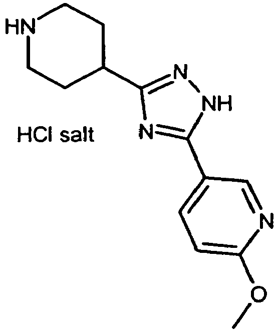

This intermediate was prepared according to 2-(5-piperidin-4-yl-2H-[1,2,4]triazol-3-yl)-pyridine dihydrochloride (intermediate example 5.0).
MS (M+1): 260.26;
UPLC-MS: RT = 0.55 min; m/z = 260.26.

### Intermediate Example 18.0: 4-ethyl-2-(5-piperidin-4-yl-2H-[1,2,4]triazol-3-yl)-pyridinehydrochloride salt

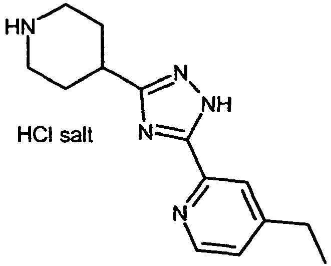

This intermediate was prepared according to 2-(5-piperidin-4-yl-2H-[1,2,4]triazol-3-yl)-pyridine dihydrochloride (intermediate example 5.0).
MS (M+1): 258.29;
UPLC-MS: RT = 0.60 min; m/z = 258.29.

### Intermediate Example 19.0: 2-(5-piperidin-4-yl-2H-[1,2,4]triazol-3-yl)-6-trifluoromethyl-pyridine hydrochloride salt

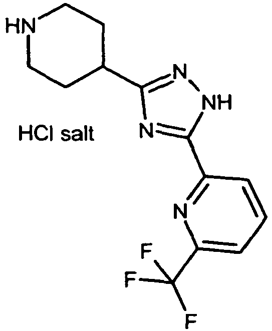

This intermediate was prepared according to 2-(5-piperidin-4-yl-2H-[1,2,4]triazol-3-yl)-pyridine dihydrochloride (intermediate example 5.0).
MS (M+1): 298;
1 H-NMR (300 MHz, d6-DMSO, characteristic signals): δ 9.16 (br s, 1 H), 8.93 (br s, 1 H), 8.28 (d, 1 H), 8.19 (t, 1 H), 7.93 (dd, 1 H) ppm.

### Intermediate Example 20.0: 2-(5-piperidin-4-yl-2H-[1,2,4]triazol-3-yl)-4-trifluoromethyl-pyridine hydrochloride salt

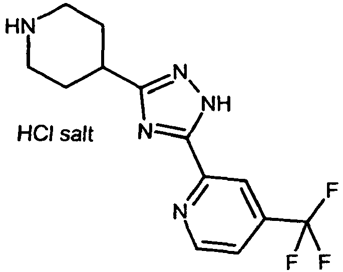

This intermediate was prepared according to 2-(5-piperidin-4-yl-2H-[1,2,4]triazol-3-yl)-pyridine dihydrochloride (intermediate example 5.0).
¹H NMR (300MHz, 400 MHz): δ 9.22 (m, 1H), 9.0 (m, 1 H), 8.93 (d, 1 H), 8.21 (s, 1 H), 7.86 (d, 1H), 3.29 (m, 2H), 3.15 (m, 1H), 3.02 (m, 2H), 2.13-2.17 (m, 2H), 1.91-2.01 (m, 2H) ppm.

### Intermediate Example 21.0: 2-(5-piperidin-4-yl-2H-[1,2,4]triazol-3-yl)-pyrimidine hydrochloride salt

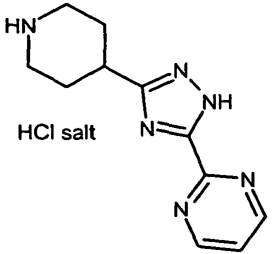

Prepared according to 2-(5-piperidin-4-yl-2H-[1,2,4]triazol-3-yl)-pyridine dihydrochloride (intermediate example 5.0).
1 H-NMR (300 MHz, d6-DMSO): δ 9.17 - 8.98 (m, 2H), 8.93 (d, 2H), 7.57 (t, 1 H), 3.24 - 3.29 (m, 2H), 2.95 - 3.18 (m, 3H), 2.11 - 2.16 (m, 2H), 1.88 - 2.00 (m, 2H) ppm.

### Intermediate Example 22.0: 2-(5-piperidin-4-yl-2H-[1,2,4]triazol-3-yl)-pyrazine hydrochloride salt

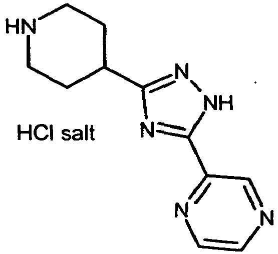

Was prepared according to 2-(5-piperidin-4-yl-2H-[1,2,4]triazol-3-yl)-pyridine dihydrochloride (intermediate example 5.0).
MS (M+1): 231.21;
UPLC-MS: RT = 0.51 min; m/z = 231.21.

### Intermediate Example 23.0: 4-(5-furan-2-yl-1H-[1,2,4]triazol-3-yl)-piperidine hydrochloride salt

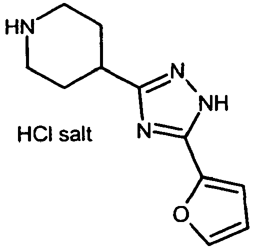

Prepared according to 2-(5-piperidin-4-yl-2H-[1,2,4]triazol-3-yl)-pyridine dihydrochloride (intermediate example 5.0).

### Intermediate Example 24.0: 4-(5-thiophen-2-yl-1H-[1,2,4]triazol-3-yl)-piperidine hydrochloride salt

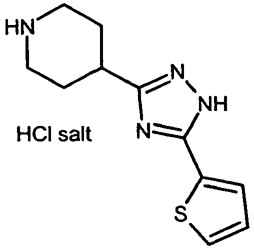

Prepared according to 2-(5-piperidin-4-yl-2H-[1,2,4]triazol-3-yl)-pyridine dihydrochloride (intermediate example 5.0).

### Intermediate Example 25.0 : 4-[5-(1H-pyrrol-2-yl)-1H-[1,2,4]triazol-3-yl]-piperidine hydrochloride salt

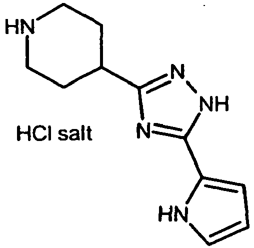

### Step 1: 1H-pyrrole-2-carbohydrazonamide

A solution of 10g 1*H*-pyrrole-2-carbonitrile and 1 eq sodium methoxide in 20ml ethanol was stirred for 10 min. Hydrazine hydrate (3 eq.) was then added and resulting reaction mixture was stirred at room temperature for 18h. The reaction mixture was then diluted with water, extracted with ethyl acetate, dried over Na₂SO₄ and concentrated under vacuum to yield desired compound.
**Step 2:** 4-[5-(1*H*-pyrrol-2-yl)-1*H*-1,2,4-triazol-3-yl]piperidine (3 step process) The further synthesis was performed in analogy to the synthesis of 2-(5-piperidin-4-yl-2H-[1,2,4]triazol-3-yl)-pyridine dihydrochloride (intermediate example 5.0, Steps 2 to 4) except that 1*H*-pyrrole-2-carbohydrazonamide was substituted for pyridine-2-carbohydrazonamide in Step 2.

### Intermediate Example 26.0: 4-(5-phenyl-1H-[1,2,4]triazol-3-yl)-piperidine hydrochloride salt

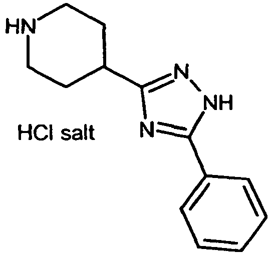

Prepared according to 4-[5-(1H-pyrrol-2-yl)-1H-1,2,4-triazol-3-yl]piperidine hydrochloride salt (intermediate example 25.0)

### Intermediate Example 27.0: 4-(5-thiazol-2-yl-1H-[1,2,4]triazol-3-yl)-piperidine hydrochloride salt

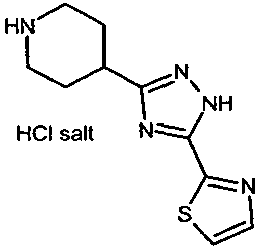

### Step1: 2-Trimethylsilanyl-thiazole

To a mixture of 40.6ml n-butyl lithium (1,6M in hexane) and 18ml diethylether was added dropwise at -70°C a solution of 5.03g thiazole dissolved in 59ml diethylether. After 30min 6.41g trimethylsilylchloride dissolved in 59ml diethylether was added at -70°C. The reaktion mixture was stirred at -70°C for 1 h and allowed to warm up to room temperature. The mixture was washed with saturated NaHCO₃ solution, dried over Na₂SO₄ and the solvent was everporated. The residue was distiled, to yield the desired product.

### Step2: Thiazole-2-yl-iminocarbonylhydrazine

10.0g 2-trimethylsilanyl-thiazole and 11.5g tolylsulfonylcyanid are stirred at 70°C for 5h. The mixture was diluted with THF and 9.83g hydrazinhydrate was added at 10°C. The reaction mixture was stirred at room temperature over night. The solvent was removed by evaporation and the residue was purified by chromatography on silica gel (dichloromethane / methanol) to yield the desired product. **Step3:** 4-[N'-(imino-thiazol-2-yl-methyl)-hydrazinocarbonyl]-piperidine-1-carboxylic acid tert-butyl ester

8.65g piperidine-1 ,4-dicarboxylic acid mono-tert-butyl ester was dissolved in dichloromethane, 6.12g 1,1-carbonyl-diimidazole was added portionwise. 5.45g thiazole-2-yl-iminocarbonylhydrazine are added slowly and the mixture was stirred at room temperature for 18 hours. The solvent was removed by evaporation and the residue was washed with water. The crude product was dried and used without further purification.

### Step4: 4-(5-Thiazol-2-yl-1H-[1,2,4]triazol-3-yl)-piperidine-1-carboxylic acid tert-butyl ester

8.00g 4-[N'-(imino-thiazol-2-yl-methyl)-hydrazinocarbonyl]-piperidine-1-carboxylic acid tert-butyl ester was heated to 220°C. The clear melting was stirred at this temperature for 15min. The melting was cooled to 80°C and 42ml ethanol are added carefully. The solvent was removed to obtain the crude product, a mixture of the desired product and 4-(5-thiazol-2-yl-1*H*-[1,2,4]triazol-3-yl)-piperidine. This mixture was used for the next reaction without further purification.

### Step5: 4-(5-Thiazol-2-yl-1H-[1,2,4]triazol-3-yl)-piperidine hydrochloride

The mixture of 7.59g of the crude product optained in step 4 was dissolved in dioxane and 68ml hydrogen chloride 4M sol. in dioxane was added slowly. The product appears as an oil. After addition of 542ml methanol the oil was dissolved. The solution was stirred over night until precipitation of the crystilline product.

### Intermediate Example 28.0: 2-(3-piperidin-4-yl-[1,2,4]oxadiazol-5-yl)-pyridine hydrochloride salt

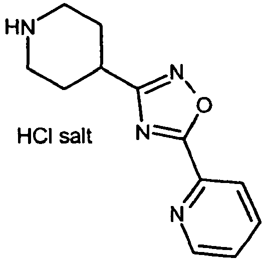

May be prepared according to procedures given in WO2006065601.

### Intermediate Example 29.0: 2-(5-piperidin-4-yl-2H-pyrazol-3-yl)-pyridine hydrochloride salt

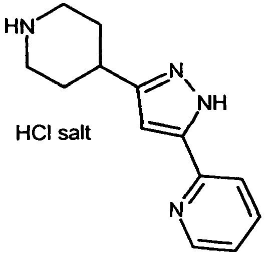

May be prepared according to procedures given in WO2004096131.

### Intermediate Example 30.0: 2-(5-azetidin-3-yl-2H-[1,2,4]triazol-3-yl)-pyridine hydrochloride salt

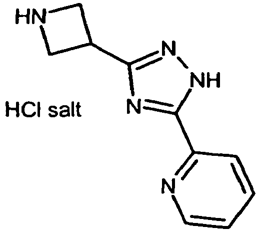

### Procedure A

### Step 1: pyridine-2-carbohydrazonamide

A solution of pyridine-2-carbonitrile 20g (192 mmol) and hydrazine hydrate (3 Eq.) in ethanol (50 mL) was stirred at room temperature for 18 hrs. The reaction mass was then diluted with water, extracted with ethyl acetate, and the organic portion dried (Na2SO4) and concentrated in vacuo to yield the desired compound.
MS (M+1): 137.07
¹H NMR (300MHz, CDCl₃): δ 8.53 (d, 1 H), 8.02 (d, 1 H), 7.72 (t, 1 H), 7.29 (t, 1H), 5.42 (bs, 2H), 4.60 (bs, 2H) ppm.

### Step 2: 3-[1-amino-1-pyridin-2-yl-meth-(Z)-ylidene-hydrazinocarbonyl]-azetidine-1-carboxylic acid tert-butyl ester

To a solution of 1-(*tert*-butoxycarbonyl) azetidine-3-carboxylic acid in dichloromethane (0.56 mL per mmol 1-(*tert*-butoxycarbonyl) azetidine-3-carboxylic acid) was added carbonyl diimidazole (1 Eq.) in small portions over a period of 30 min. Pyridine-2-carbohydrazonamide was then added to the reaction mixture and stirred at room temperature for 3 hrs. The mixture was concentrated *in vacuo* and the reaction mass was then stirred in water for 30 min. The precipitated solid was filtered and dried to afford the desired compound.
MS (M+1): 319.93
¹H NMR (300MHz, CDCl3): δ 10.90 (s, 1 H), 8.53 (d, 1H), 8.04 (d, 1H), 7.75-7.70 (m, 1 H), 7.24 (d, 1 H), 6.44 (s, 2H), 4.24-4.17 (m, 4H), 4.09-4.03 (m, 1 H), 1.45 (s, 9H) ppm.

### Step 3: 3-(5-pyridin-2-yl-1H-[1,2,4]triazol-3-yl)-azetidine-1-carboxylic acid tert-butyl ester

The 3-[1-amino-1-pyridin-2-yl-meth-(Z)-ylidene-hydrazinocarbonyl]-azetidine-1-carboxylic acid tert-butyl ester obtained in step 2 was melted at 220°C under nitrogen atmosphere for 1 hr. The reaction was then cooled until ethanol could be safely added to the still warm melt. Enough ethanol was added till the solid dissolved. The ethanol was evaporated to get the desired crude compound, which was used without further purification in the next step.
MS (M+1): 302.35
¹H NMR (300MHz, CDCl3): δ 12.97 (bs, 1 H), 8.76 (d, 1 H), 8.24 (d, 1H), 7.89 (t, 1 H), 7.45 (d, 1 H), 4.3-4.27 (m, 4H), 4.03-4.0 (m, 1 H), 1.46 (s, 9H) ppm.

### Step 4: 2-(5-azetidin-3-yl-2H-[1,2,4]triazol-3-yl)-pyridine hydrochloride salt

3-(5-pyridin-2-yl-1H-[1,2,4]triazol-3-yl)-azetidine-1-carboxylic acid tert-butyl ester (3.13 g, 10.39 mmol) was suspended in a solution of HCl in dioxane (4M, 80 mL) and stirred at rt overnight. The reaction mixture was diluted with diethyl ether, filtered and the residue suspended in acetonitrile and stirred for 45 min at rt. The solid (hydroscopic) was isolated by filtration, partially dissolved in warm methanol and addition of diethyl ether resulted in precipitation of a yellow sticky solid which could not be filtered. The mixture was concentrated in vacuo and dried in a vacuum oven (40 °C) to obtain the desired compound as a light-yellow solid.
MS (M+1): 202.13
¹H NMR (300MHz, d6-DMSO): δ 9.61 (bs, 1 H), 9.25 (bs, 1 H), 8.76 (d, 1H), 8.16 (m, 2H), 7.75 (d, 1H), 4.10-4.27 (m, 5H) ppm.

### Procedure B

### Step 1: 3-hydrazinocarbonyl-azetidine-1-carboxylic acid tert-butyl ester

1-(*tert*-butoxycarbonyl) azetidine-3-carboxylic acid (5 g, 24.8 mmol) was suspended in dichloromethane (15 mL) and 1,1'-carbonyldiimidazole (4.56 g, 28.1 mmol) was added in portions. The resulting mixture was stirred at rt for 30 minutes and then added dropwise to a solution of hydrazine hydrate (1.94 mL, 39.9 mmol) in dichloromethane (5 mL). After the addition was complete, the mixture was stirred for 30 min at rt. The reaction mixture was washed with saturated aqueous Na2CO3 solution (2x), brine, dried (Na2SO4) and concentrated under vacuum to give a white crystalline solid, which was triturated with diethyl ether overnight, filtered and air-dried for 5 h to give a white solid.

### Step 2: 3-(5-pyridin-2-yl-1 H-[1,2,4]triazol-3-yl)-azetidine-1-carboxylic acid tert-butyl ester

3-hydrazinocarbonyl-azetidine-1-carboxylic acid tert-butyl ester (2.74 g, 12.73 mmol) and 2-cyano-pyridine (1.45 g, 13.95 mmol) were dissolved in 2-ethoxyethanol (30 mL) amd a 30 wt% solution of NaOMe in MeOH (1.19 mL, 6.38 mmol) was added. The resulting mixture was heated to 130 °C and stirred overnight. On cooling the mixture was neutralised by the addition of acetic acid and partitioned between EtOAc and saturated aqueous NaHCO3 solution. The organic phase was dried (Na2SO4) and concentrated in vacuo to give a yellow solid. Further purification was achieved by trituration with diethyl ether followed by recrystallisation from MeOH.

### Step 3: 2-(5-azetidin-3-yl-2H-[1,2,4]triazol-3-yl)-pyridine hydrochloride salt Prepared as described above for Procedure A Step 4.

### Intermediate Example 31: 2-[5-(azetidine-3-yl)-1H-1,2,4-triazole-3-yl]-6-methylpyridine dihydrochloride

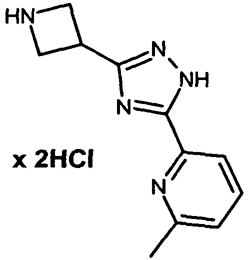

This intermediate has been prepared in analogy to example 44.0, procedure A.

### Step 1: 6-methylpyridine-2-carbohydrazonamide

11.97 g (101.4 mmol) 6-methylpyridine-2-carbonitrile are dissolved in 25 mL ethanol. After addition of 36.3 mL (304.05 mmol) hydrazine hydrate (w = 30%) the reaction mixture is stirred for 24 hours at room temperature. The precipitated product (K1 = 1.45 g) has been filtered off and the filtrate is evaporated to 1/3 of its volume. After dilution with water the reaction mixture is extracted three times with ethyl acetate. The combined organic extracts are washed with brine and dried (Na₂SO₄). The solvent has been removed yielding a K2 (11.11 g) of the desired product. The overall yield is 78.9 %.
MS (ES+, M+1): 151
¹H-NMR (300 MHz, d6-DMSO): 7.65 (d, 1H), 7.90 (dd, 1 H), 7.12 (d, 1H), 5.65 (br., 2H), 5.19 (br., 2H), 2.51 (s, 3H, under the signal of the solvent) ppm.

### Step 2: tert-Butyl 3-({2-[amino(6-methylpyridine-2-yl)methylene]hydrazino}carbonyl)azetidine-1-carboxylate

To a solution of 8.21 g (54.7 mmol) 1-(*tert*-butoxycarbonyl) azetidine-3-carboxylic acid in 80 mL dichloromethane are added 8.86 g (54.7 mmol) carbonyl diimidazole within 30 min. After stirring for five minutes 11 g (54.7 mmol) 6-methylpyridine-2-carbohydrazonamide are added and the reaction mixture is stirred at room temperature for 3 hours. The solvent has been evaporated and the residue is treated with water. The formed precipitate has been filtered off and dried yielding 16.47 g (81.3%) of the desired compound as a mixture of tautomers.
MS (Cl, M+1): 334
¹H-NMR (300 MHz, d6-DMSO): 10.09, 9.87 (s, combined 1H), 7.64-7.89 (m, 2H), 7.22-7.31 (m, 1 H), 6.59 (br., 2H), 3.80-4.10 (m, 4H), 3.25-3.45 (m, 1H, under the water signal of the solvent, 2.52 (s, 3H), 1.35 ("s", 9H) ppm.

### Step 3: tert-Butyl 3-[3-(6-methylpyridine-2-yl)-1H-1,2,4-triazole-5-yl]azetidine-1-carboxylate

16.4 g (49.3 mmol) *tert*-Butyl 3-({2-[amino(6-methylpyridine-2-yl)methylene]hydrazino}carbonyl)azetidine-1-carboxylate are heated under a nitrogen atmosphere to the melting point (220 °C) and kept there for 90 minutes. Ethanol is cautiously added to the reaction mixture during the cooling down phase (at around 135 °C). The reaction mixture is stirred over night at room temperature and the ethanol is evaporated. Due to an incomplete reaction the residue is heated once more to 220 °C for one hour and the work up is repeated yielding 12.91 g (74.58%) of the desired crude product (the byproduct is the cyclised compound which has lost the Boc group).
¹H-NMR (300 MHz, d6-DMSO): 14.30 (br., 1 H), 7.75-7.89 (m, 2H), 7.31 (d, 1 H), 3.82-4.49 (m, 4H), 3.32-3.48 (m, 1 H, partly under the water signal of the solvent), 2.52 (s, 3H), 1.39 (s, 9H) ppm.

### Step 4: 2-[5-(Azetidine-3-yl)-1H-1,2,4-triazole-3-yl]-6-methylpyridine dihydrochloride

11.6 g (36.8 mmol) *tert*-Butyl 3-[3-(6-methylpyridine-2-yl)-1*H*-1,2,4-triazole-5-yl]azetidine-1-carboxylate are dissolved in 150 mL dioxane. 27.6 mL HCl in dioxane (4M) are added dropwise and the reaction mixture is stirred over night at room temperature. The reaction mixture is evaporated to dryness yielding 13.1 g (76.6%) of the desired salt which is 60% pure and is used without further purification.

### Intermediate Example 32.0: 2-(5-pyrrolidin-3-yl-2H-[1,2,4]triazol-3-yl)-pyridine hydrochloride salt

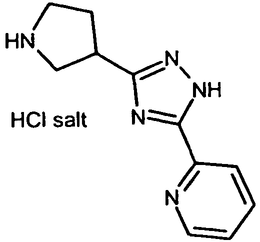

This intermediate was prepared according to the procedures for 2-(5-azetidin-3-yl-2H-[1,2,4]triazol-3-yl)-pyridine hydrochloride salt (intermediate example 30.0).
MS (M+1): 216

### Intermediate Example 33.0: 5-fluoro-2-piperidin-4-yl-1H-benzoimidazole hydrochloride salt

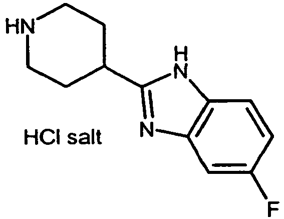

To a mixture of piperidine-4-carboxylic acid (18.33 g, 0.14 mol) and 4-fluorobenzene-1,2-diamine (18.01 g, 0.14 mol) was added polyphosphoric acid (138.39 g) and the mixture heated at 180 °C (internal temperature) for 2 h 45 minutes. The reaction mixture was cooled, reheated to 80 °C and the reaction was quenched by cautious addition to water (300 mL). The mixture was made basic (pH 8) by the addition of concentrated aqueous NaOH. The aqueous phase was extracted sequentially with 3:7 isopropanol:CH2Cl2 (2 x 200 mL) and CH2Cl2 (150 mL) and the combined organic phase dried (Na2SO4) and concentrated. The aqueous phase was reextracted with n-butanol (2 x 200 mL), the organic layer dried (Na2SO4) and concentrated. The crude product was stirred in diethyl ether, filtered and dried to give crude 5-fluoro-2-piperidin-4-yl-1 H-benzoimidazole. Further purification was achieved by preparing the hydrochloride salt. Thus, 10 g of the crude 5-fluoro-2-piperidin-4-yl-1 H-benzoimidazole was dissolved in MeOH (85 mL) and a solution of HCl in dioxane (20 mL) was added dropwise, and the title compound was obtained by filtration.
MS (M+1): 220.1
1H NMR (d6-DMSO + D₂O): δ 7.78 (m, 1 H), 7.6 (m, 1 H), 7.38 (m, 1 H), 3.55 (m, 1H), 3.4 (m, 2H), 3.08 (m, 1H), 2.3 (m, 2H), 2.08 (m, 2H) ppm.

The following intermediates were prepared in analogy to 5-fluoro-2-piperidin-4-yl-1H-benzoimidazole dihydrochloride above by replacing 4-fluoro-benzene-1,2-diamine with the appropriate diamine.

| Intermediate Example | Structure / Name | Analytical Data |
|---|---|---|
| 33.1 | 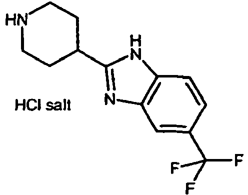 | 1 H NMR (d6-DMSO + D₂O): δ 8.1 (s, 1 H), 7.9 (d, 1 H), 7.78 (d, 1 H), 3.56 (m, 1 H), 3.4 (m, 2H), 3.1 (m, 2H), 2.32 (m,. 2H), 2.1 (m, 2H) ppm |
| | 2-piperidin-4-yl-5-trifluoromethyl-1 H-benzoimidazole hydrochloride salt | |
| 33.2 | 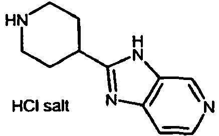 | MS (M+1): 203.1 1H NMR (d6-DMSO + D₂O): δ 9.28 (s, 1H), 8.5 (d, 1 H), 8.1 (d, 1 H), 3.42 (m, 1 H), 3.27 (m, 2H), 2.28 (m, 2H), 2.04 (m, 2H) ppm |
| | 2-piperidin-4-yl-3H-imidazo[4,5-c]pyridine hydrochloride salt | |
| 33.3 | 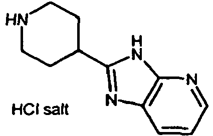 | MS (M+1): 203.1 1 H NMR (d6-DMSO + D₂O): δ 8.58 (d, 1 H), 8.4 (d, 1H), 7.6 (m, 1H), 3.36-3.5 (m, 3H), 3.08 (m, 2H), 2.28 (m, 2H), 2.04 (m, 2H) ppm |
| | 2-piperidin-4-yl-3H-imidazo[4,5-b]pyridine hydrochloride salt | |

### Intermediate Example 34.0: 2-piperidin-4-yl-1H-benzoimidazole-5-carbonitrile hydrochloride salt

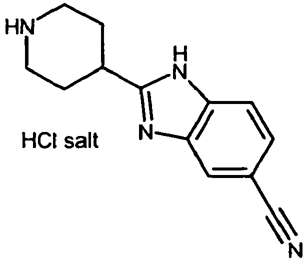

### Step 1: 4-(2-amino-4-cyano-phenylcarbamoyl)-piperidine-1-carboxylic acid tert-butyl ester

To a solution of piperidine-1,4-dicarboxylic acid mono-tert-butyl ester (14.1 g, 0.061 mol) in DMF (282 mL) was added HBTU (27.76 g, 0.073 mol), DMAP (10.2 g, 0.084 mol) and diisopropylethyl amine (24.2 mL). The reaction mixture was stirred for 30 minutes at rt before 3,4-diaminobenzonitrile (8 g, 0.059 mol) was added. The mixture was stirred overnight at rt before the reaction was quenched by pouring into water (2 L). The mixture was extracted with CH2Cl2 and the organic phase washed successively with 1 M aq. HCl solution and 10% aq. Na2CO3 solution, dried (Na2SO4) and concentrated in vacuo. Purification by chromatography on silica gel afforded the title compound.

### Step 2: 4-(5-cyano-1H-benzoimidazol-2-yl)-piperidine-1-carboxylic acid tert-butyl ester

A solution of 4-(2-amino-4-cyano-phenylcarbamoyl)-piperidine-1-carboxylic acid tert-butyl ester (6 g) in EtOH (61 mL) and 2M aq. NaOH solution (61 mL) was heated at 75 °C (bath temperature) overnight. The heating was discontinued, the reaction was chilled (ice water bath) and quenched by pouring into saturated aq. citric acid solution (250 mL). The mixture was extracted with CH2Cl2 (5 x) and the combined organic extract was dried (Na2SO4), filtered and concentrated in vacuo. Purification by chromatography on silica gel afforded the title compound.

### Step 3: 2-piperidin-4-yl-1H-benzoimidazole-5-carbonitrile hydrochloride salt

To a solution of 4-(5-cyano-1 H-benzoimidazol-2-yl)-piperidine-1-carboxylic acid tert-butyl ester (3.2 g, 10 mmol) in dioxane (13 mL) was added a solution of HCl in dioxane (25%, 14.3 mL). The resulting precipitate was filtered to give the title compound.
MS (M+1): 227.1
1 H NMR (400 MHz, d6-DMSO + D₂O) δ 8.22 (s, 1 H), 7.85 (d, 1 H), 7.77 (d, 1 H), 3.42-3.5 (m, 3H), 3.12 (m, 2H), 2.34 (m, 2H), 2.09 (m, 2H) ppm.

### Intermediate Example 35.0: 9-piperidin-4-yl-9H-purin-6-ylamine hydrochloride salt

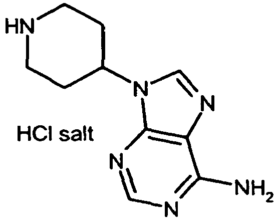

Was prepared according to procedures given in WO2006065601.
MS (M+1): 219.2

### Intermediate Example 36.0: 2-piperidin-4-yl-quinoxaline hydrochloride salt

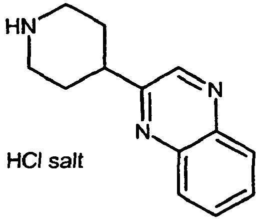

To a stirred solution of 4-quinoxalin-2-yl-piperidine-1-carboxylic acid tert-butyl ester (200 mg, 0.64 mmol, obtained commercially) in 0.5 mL dioxane/MeOH (2:3), at rt was added a solution of HCl in dioxane (1.6 mL, 10 Eq.). The mixture was stirred for 2 h before the solid was filtered, washed and dried to give the title compound.
MS (M+1): 214.2
1 H NMR (300 MHz, d6-DMSO + D₂O): δ 9.45 (br s, 1 H), 9.15 (br s, 1H), 8.95 (s, 1 H), 8.08 (m, 2H), 7.85 (m, 2H), 3.35-3.45 (m, 3H), 3.06 (m, 2H), 2.1-2.2 (m, 4H) ppm.

### Intermediate Example 37.0: 4-methyl-2-(5-piperidin-4-yl-2H-[1,2,4]triazol-3-yl)-pyridine hydrochloride salt

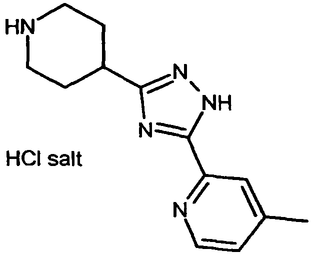

This intermediate was prepared according to 2-(5-piperidin-4-y1-2H-[1,2,4]triazol-3-yl)-pyridine dihydrochloride (intermediate example 5.0).
UPLC-MS: RT = 0.47 min; m/z = 244.27;
1 H NMR (300 MHz, d6-DMSO): δ 9.01 - 9.23 (2 x br s, 2H), 8.49 (d, 1H), 7.86 (s, 1 H), 7.28 (d, 1 H), 3.24 - 3.28 (m, 2H), 2.95 - 3.13 (m, 3H), 2.37 (s, 3H), 2.09 - 2.15 (m, 2H), 1.87 - 2.01 (m, 2H) ppm.

Compounds of General Formula (I) may typically be prepared according to the following General Procedures, or their preparation is illustrated by specific examples below. The preparation of further examples not listed here may be accomplished by analogy to, modification of, or adaptation to, these or known procedures.

### General Procedure 1: Reductive amination (use of amine salt)

To a solution of 0.75 mmol of the aldehyde intermediate in THF (6 mL) is added triethylamine (2 Eq.). The reaction mixture is stirred for 5 minutes before the amine salt (1.5 Eq.) and acetic acid (2.5 Eq.) are added. The reaction mixture is stirred for 10 minutes before NaBH(OAc)₃ (6 Eq.) is added portionwise over 40 minutes. The reaction mixture is stirred overnight at room temperature, before quenching with methanol and concentration *in vacuo.* The residue is taken up in chloroform and washed with water, dried and concentrated *in vacuo.* Purification according to standard techniques affords the desired compound.

In the case that the free base of the amine is employed, the general procedures above may be modified by omitting the triethylamine.

### General Procedure 2: Amination via a methanesulfonate intermediate (use of amine salt)

To the stirred solution of the benzyl alcohol intermediate (0.52 mmol) in 15mL of dichloromethane is added methanesulfonyl chloride (1.1 eq) at 0°C followed by triethylamine (1.5 eq). The reaction mixture is allowed to stir at room temperature for 3h. The reaction is quenched with water and extracted with DCM. The organic layer is dried and concentrated. It is then taken up in the next reaction without further purification. The crude is dissolved in 5mL of DMF. To this solution the amine hydrochloride salt (1 eq) and triethylamine (4 eq) are added. The reaction mixture is heated at 80°C for 3h. The reaction mixture is quenched with water and extracted with ethyl acetate. The organic layer is dried and concentrated. Purification by standard techniques obtains the desired compound.

In the case that the free base of the amine is employed, the general procedure above may be modified by reducing the number of equivalents of triethylamine from 4 to 2.

### Example 1.0: 5-methyl-8-phenyl-7-{4-[4-(5-pyridin-2-yl-1H-[1,2,4]triazol-3-yl)-piperidin-1-ylmethyl]-phenyl}-imidazo[1,2-c]pyrimidine

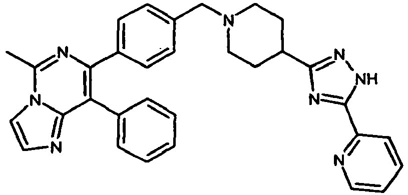

A mixture of 0.350 g (1.12 mmol) 4-(5-methyl-8-phenyl-imidazo[1,2-c]pyrimidin-7-yl)-benzaldehyde, 406 mg (1.33 mmol) 2-(5-piperidin-4-yl-2H-[1,2,4]triazol-3-yl)-pyridine dihydrochloride, 392 µL triethylamine and 92 µL AcOH, in 35 mL DCE was stirred at 40 °C. On cooling to rt, 283 mg NaBH(OAC)₃, was added. The reaction mixture was stirred for 2 h before heating to 45 °C. After 1 hour, an additional 283 mg NaBH(OAc)₃ was added and heating continued at 45 °C. On cooling to rt, the reaction was concentrated *in vacuo* and purified by chromatography followed by preparative HPLC to afford the title compound.
MS (M+1): 527.1;
1 H NMR (400MHz, d6-DMSO): δ 13.8 & 14.3 (br s, br s, 1 H), 8.65 (br s, 1 H), 7.9-8.05 (m, 3H), 7.67 (s, 1 H), 7.3-7.55 (m, 8H), 7.2 (d, 2H), 3.46 (s, 2H), 2.88 (s, 3H), 2.75-2.88 (m, 3H), 2.08 (m, 2H), 1.95 (m, 2H), 1.78 (m, 2H) ppm.

### Example 2.0: 8-phenyl-7-{4-[4-(5-pyridin-2-yl-1H-[1,2,4]triazol-3-yl)-piperidin-1-ylmethyl]-phenyl}-imidazo[1,2-c]pyrimidine

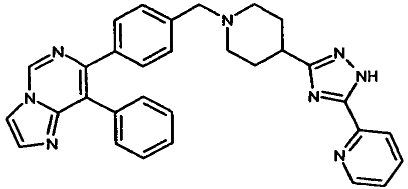

2.93 mL (20.9 mmol) triethylamine was added to a solution of 3.02 g 2-(5-piperidin-4-yl-2H-[1,2,4]triazol-3-yl)-pyridine dihydrochloride in 80 mL MeOH. To this solution a solution of 2.50 g (8.35 mmol) 4-(8-phenyl-imidazo[1,2-c]pyrimidin-7-yl)-benzaldehyde in 80 mL DMF was added, followed by 1.2 mL glacial acetic acid and 7.45 g NaBH(OAc)₃: The resulting mixture was stirred at rt. Additional 2 Eq. portions of NaBH(OAc)₃ were added after 1, 2 and 3 hours respectively. The reaction was stirred for 3 days before the volatiles were removed in vacuo and the residue partitioned between CH₂Cl₂ and water. The organic phase was separated and the water phase extracted with CH₂Cl₂. The combined organic portions were dried and concentrated and the residue was purified by chromotagraphy to yield the title compound (762 mg).
MS (M+1): 513.1;
1 H NMR (300MHz, d6-DMSO): δ 13.9, 14.3 (br s, br s, 1 H), 9.5 (s, 1 H), 8.67 (m, 1H), 8.10 (s, 1 H), 8.03 (d, 1 H), 7.95 (m, 1 H), 7.65 (m, 1H), 7.46 (m, 1 H), 7.3-7.4 (m, 10H), 7.2 (d, 2H), 3.47 (s, 2H), 2.7-2.85 (m, 3H), 2.09 (m, 2H), 1.95 (m, 2H), 1.8 (m, 2H) ppm.

### Example 3.0: 8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine

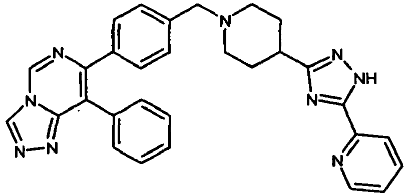

This example was prepared by reacting 4-(8-phenyl-[1,2,4]triazolo[4,3-c]pyrimidin-7-yl)-benzaldehyde with 2-(5-piperidin-4-yl-2H-[1,2,4]triazol-3-yl)-pyridine dihydrochloride in analogy to Example 1.
MS (M+1): 514.2;
1 H NMR (300MHz, CDCl₃): δ 9.45 (s, 1 H), 8.67 (m, 1 H), 8.42 (s, 1 H), 8.15 (d, 1 H), 7.83 (t, 1 H), 7.35-7.45 (m, 8H), 7.25-7.3 (m, 2H), 3.58 (s, 2H), 3.0 (m, 2H), 2.9 (m, 1 H), 2.0-2.2 (m, 6H) ppm.

### Example 4.0: 3-methyl-8-phenyl-7-{4-[4-(5-pyridin-2-yl-1H-[1,2,4]triazol-3-yl)-piperidin-1-ylmethyl]-phenyl}-[1,2,4]triazolo[4,3-c]pyrimidine

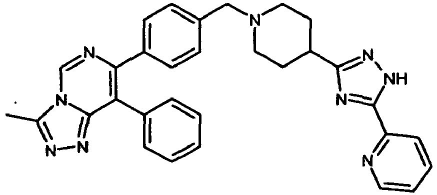

This example was prepared by reacting 4-(3-methyl-8-phenyl-[1,2,4]triazolo[4,3-c]pyrimidin-7-yl)-benzaldehyde with 2-(5-piperidin-4-yl-2H-[1,2,4]triazol-3-yl)-pyridine dihydrochloride in analogy to Example 1.
MS (M+1): 528.2;
1 H NMR (300MHz, CDCl₃): δ 9.30 (s, 1H), 8.62 (m, 1H), 8.15 (d, 1H), 7.82 (t, 1H), 7.32-7.42 (m, 8H), 7.25 (m, 2H), 3.52 (s, 2H), 2.95 (m, 2H), 2.85 (m, 1H), 2.52 (s, 3H), 1.9-2.18 (m, 6H) ppm.

### Example 5.0: 7-(4-{4-[5-(5,6-dimethyl-pyridin-2-yl)-1H-[1,2,4]triazol-3-yl]-piperidin-1-ylmethyl}-phenyl)-5-methyl-8-phenyl-imidazo[1,2-c]pyrimidine

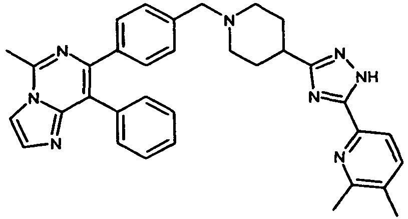

A mixture of 4-(5-methyl-8-phenyl-imidazo[1,2-c]pyrimidin-7-yl)-benzaldehyde (150 mg, 0.479 mmol) and 2,3-dimethyl-6-[3-(piperidin-4-yl)-1H-1,2,4-triazol-5-yl]pyridine dihydrochloride (174 mg) in NMP (2 mL) was treated with triethylamine (0.147 mL) and AcOH (0.066 mL) and stirred overnight at rt. NaBH(OAc)₃ (0.264 g) was added and the mixture stirred for an additional 5 hours. The reaction was partitioned between DCM and saturated aqueous sodium hydrogencarbonate solution and the organic layer dried and concentrated in vacuo. The residue was purified by preparative reverse phase HPLC to give the title compound, contamined with formic acid.
UPLC-MS: RT = 0.69 min; m/z = 553.5 (ES-);
1H NMR (300MHz, d6-DMSO): δ 14.05 & 13.71 (br s), 8.03 (s, 1 H), 7.71 (d, 1 H), 7.50 - 7.63 (m, 2H), 7.26 - 7.32 (m, 7H), 7.16 (d, 2H), 3.49 (s, 2H), 2.80 - 2.84 (m, 6H), [3H obscured by solvent], 2.26 (s, 3H), 2.00 - 2.18 (m, 2H), 1.90 -1.94 (m, 2H), 1.68 -1.79 (m, 2H) ppm.

The following Examples were prepared in analogy by using the appropriate aldehyde and amine intermediate.

| Example | Structure/ Name | Analytical data |
|---|---|---|
| 6.0 | 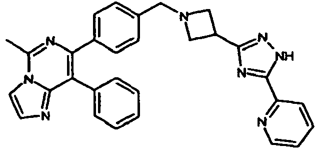 | UPLC-MS: RT = 0.66 min; m/z = 499.4; |
| | | ¹H-NMR (300 MHz, d6-DMSO): δ 14.36 (br s), 8.63 (d, 1 H), 8.01 - 8.03 (m, 2H), 7.91 (t, 1 H), 7.63 (d, 1 H), 7.44 (t, 1 H), 7.24 - 7.30 (m, 7H), 7.13 (m, 2H) ppm. |
| | 5-methyl-8-phenyl-7-{4-[3-(5-pyridin-2-yl-1H-[1,2,4]triazol-3-yl)-azetidin-1-ylmethyl]-phenyl}-imidazo[1,2-c]pyrimidine | |
| 7.0 | 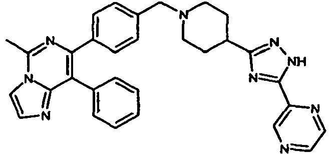 | UPLC-MS: RT = 0.64 min; m/z = 528.5; |
| | 5-methyl-8-phenyl-7-{4-[4-(5-pyrazin-2-yl-1H-[1,2,4]triazol-3-yl)-piperidin-1-ylmethyl]-phenyl}-imidazo[1,2-c]pyrimidine | ¹H-NMR (300 MHz, d6-DMSO): δ 9.17 (m, 1H), 8.68 - 8.70 (m, 2H), 8.04 (d, 1 H), 7.65 (m, 1 H), 7.24 - 7.34 (m, 9H), 3.82 (br s, 2H), 2.84 - 3.15 (m, 6H), [2H obscured by solvent], 1.73 - 2.13 (m, 4H) ppm. |
| 8.0 | 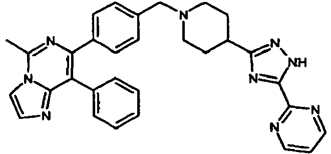 | UPLC-MS: RT = 0.57 min; m/z = 528.5; |
| | 5-methyl-8-phenyl-7-{4-[4-(5-pyrimidin-2-yl-1H-[1,2,4]triazol-3-yl)-piperidin-1-ylmethyl]-phenyl}-imidazo[1,2-c]pyrimidine | ¹H-NMR (300 MHz, d6-DMSO): δ 8.88 (d, 2H), 8.02 (m, 1 H), 7.63 (m, 1 H), 7.51 (t, 1 H), 7.25 - 7.30 (m, 7H), 7.15 (d, 2H), 3.43 (s, 2H), 2.71 - 2.84 (m, 6H), 2.01 - 2.08 (m, 2H), 1.90 - 1.94 (m, 2H), 1.67 - 1.78 (m, 2H) ppm. |
| 9.0 | 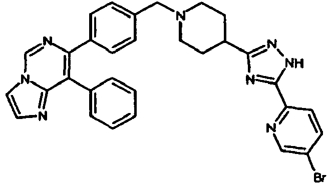 | UPLC-MS: RT = 0.77 min; m/z = 591.61 (ES-); |
| | 7-(4-{4-[5-(5-bromo-pyridin-2-yl)-1H-[1,2,4]triazol-3-yl]-piperidin-1-ylmethyl}-phenyl)-8-phenyl-imidazo[1,2-c]pyrimidine | ¹H-NMR (300 MHz, d6-DMSO): δ 14.15 (br s), 9.45 (s, 1H), 8.74 (m, 1 H), 8.14 (m, 1 H), 8.07(m, 1 H), 7.93 (d, 1 H), 7.61 (m, 1 H), 7.26 - 7.32 (m, 7H), 7.15 (m, 2H), 3.42 (s, 2H), 2.69 - 2.84 (m, 3H), 2.00 - 2.07 (m, 2H), 1.89 - 1.92 (m, 2H), 1.66 -1.77 (m, 2H) ppm. |
| 10.0 | 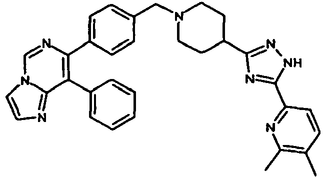 | UPLC-MS: RT = 0.65 min; m/z = 541.5; |
| | 7-(4-{4-[5-(5,6-dimethyl-pyridin-2-yl)-1H-[1,2,4]triazol-3-yl]-piperidin-1-ylmethyl}-phenyl)-8-phenyl-imidazo[1,2-c]pyrimidine | ¹H-NMR (300 MHz, d6-DMSO): δ 14.1 & 13.7 (2 x br s), 9.46 (s, 1 H), 8.08 (d, 1H), 7.71 (d, 1 H), 7.60 - 7.65 (m, 2H), 7.27 - 7.35 (m, 7H), 7.17 (d, 2H), 3.52 (br s), 2.66 - 2.86 (m, 3H), [3H obscured by solvent], 2.26 (s, 3H), 2.14 (br s, 2H), 1.91 -1.94 (m, 2H), 1.69 -1.80 (m, 2H) ppm. |
| 11.0 | 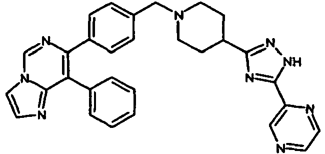 | UPLC-MS: RT = 0.57 min; m/z = 514.5; |
| | 8-phenyl-7-{4-[4-(5-pyrazin-2-yl-1 H-[1,2,4]triazol-3-yl)-piperidin-1-ylmethyl]-phenyl}-imidazo[1,2-c]pyrimidine | ¹H-NMR (300 MHz, d6-DMSO): δ 14.20 (br s), 9.45 (s, 1 H), 9.17 (m, 1 H), 8.64 - 8.69 (m, 2H), 8.08 (d, 1 H), 7.61 (d, 1 H), 7.26 (m, 7H), 7.15 (d, 2H), 3.44 (s, 2H), 2.75 - 2.83 (m, 3H), 2.02 - 2.09 (m, 2H), 1.91-1.94 (m, 2H), 1.69 -1.80 (m, 2H) ppm. |
| 11.1 | 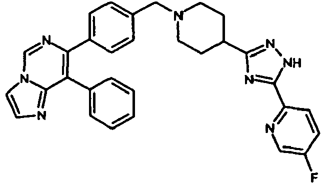 | UPLC-MS: RT = 0.68 min; m/z = 531.62; |
| | 7-(4-{4-[5-(5-fluoro-pyridin-2-yl)-1H-[1,2,4]triazol-3-yl]-piperidin-1-ylmethyl}-phenyl)-8-phenyl-imidazo[1,2-c]pyrimidine | ¹H-NMR (300 MHz, d6-DMSO): δ 14.11 (br s), 9.45 (s, 1H), 8.62 (s, 1 H), 8.03 - 8.08 (m, 2H), 7.81 (m, 1 H), 7.61 (d, 1 H), 7.26 - 7.34 (m, 7H), 7.15 (d, 2H), 3.43 (s, 2H), 2.69 - 2.84 (m, |
| | | 3H), 2.01 - 2.08 (m, 2H), 1.89-1.92 (m, 2H), 1.67 -1.78 (m, 2H) ppm. |
| 12.0 | 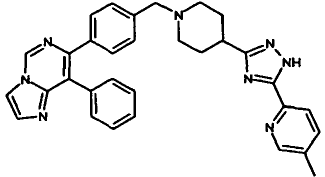 | UPLC-MS: RT = 0.67 min; m/z = 525.54 (ES-); |
| | 7-(4-{4-[5-(5-methyl-pyridin-2-yl)-1H-[1,2,4]triazol-3-yl]-piperidin-1-ylmethyl}-phenyl)-8-phenyl-imidazo[1,2-c]pyrimidine | ¹H-NMR (300 MHz, d6-DMSO): δ 14.35 (br s), 9.46 (s, 1 H), 8.47 (m, 1H), 8.09 (m, 1H), 7.84 (s, 1 H), 7.62 (m, 1H), 7.34 - 7.21 (m, 10H), 3.72 (br s, 2H), 2.80-2.96 (m, 3H), [2H obscured by solvent], 2.36 (s, 3H), 1.97-2.01 (2H, m), 1.75 -1.87 (m, 2H) ppm. |
| 13.0 | 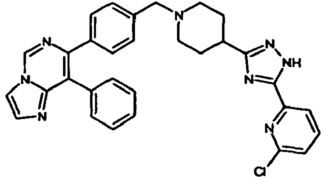 | UPLC-MS: RT = 0.74 min; m/z = 547.57; |
| | 7-(4-{4-[5-(6-chloro-pyridin-2-yl)-1H-[1,2,4]triazol-3-yi]-piperidin-1-ylmethyl}-phenyl)-8-phenyl-imidazo[1,2-c]pyrimidine | ¹H-NMR (300 MHz, d6-DMSO): δ 14.06 (br s), 9.45 (s, 1 H), 8.08 (m, 1H), 7.90 - 7.99 (m, 2H), 7.61 (m, 1H), 7.52 (d, 1 H), 7.26 - 7.34 (m, 7H), 7.15 (d, 2H), 3.43 (s, 2H), 2.69 - 2.82 (m, 3H), 2.00 - 2.08 (m, 2H), 1.89-1.93 (m, 2H), 1.67 -1.78 (m, 2H) ppm. |
| 14.0 | 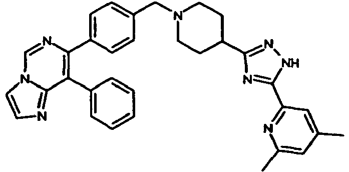 | UPLC-MS: RT = 0.68 min; m/z = 541.67; |
| | 7-(4-{4-[5-(4,6-dimethyl-pyridin-2-yl)-1H-[1,2,4]triazol-3-yl]-piperidin-1-ylmethyl}-phenyl)-8-phenyl-imidazo[1,2-c]pyrimidine | ¹H-NMR (300 MHz, d6-DMSO): δ 14.07 (br s), 9.45 (s, 1 H), 8.07 (m, 1 H), 7.61 - 7.65 (m, 2H), 7.26 - 7.34 (m, 7H), 7.10 - 7.17 (m, 3H), 3.43 (s, 2H), 2.69-2.81 (m, 3H), [3H obscured by solvent], 2.30 (s, 3H), 2.00-2.07 (m, 2H), 1.88 -1.91 (m, 2H), 1.67 -1.78 (m, 2H) ppm. |
| 15.0 | 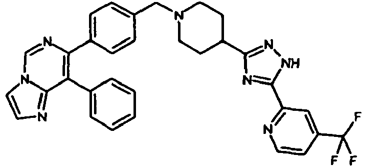 | UPLC-MS: RT = 0.79 min; m/z = 581.59; |
| | 8-phenyl-7-(4-{4-[5-(4-trifluoromethyl-pyridin-2-yl)-1H-[1,2,4]triazol-3-yl]-piperidin-1-ylmethyl}-phenyl)-imidazo[1,2-c]pyrimidine | ¹H-NMR (300 MHz, d6-DMSO): δ 14.29 (br s), 9.47 (s, 1 H), 8.92 (d, 1 H), 8.18 (s, 1 H), 8.09 (m, 1 H), 7.83 (m, 1 H), 7.62 (m, 1 H), 7.29 - 7.34 (m, 7H), 7.24 (d, 2H), 3.78 (br s, 2H), 2.91 - 3.02 (m, 3H), [2H obscured by solvent], 2.02 - 2.06 (m, 2H), 1.87 (m, 2H) ppm. |
| 16.0 | 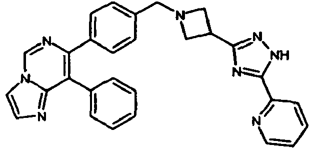 | UPLC-MS: RT = 0.62 min; m/z = 485.61; |
| | 8-phenyl-7-{4-[3-(5-pyridin-2-yl-1H-[1,2,4]triazol-3-yl)-azetidin-1-ylmethyl]-phenyl}-imidazo[1,2-c]pyrimidine | ¹H-NMR (300 MHz, d6-DMSO): δ 14.43 (br s), 9.45 (s, 1 H), 8.63 (d, 1 H), 8.07 (m, 1H), 8.02 (d, 1 H), 7.91 (m, 1H), 7.61 (m, 1 H), 7.44 (m, 1 H), 7.25 - 7.34 (m, 7H), 7.13 (d, 2H), 3.67 - 3.77 (m, 1 H), 3.55 - 3.60 (m, 4H), 3.30 (t, 2H) ppm. |

### Biological investigations

The following assays can be used to illustrate the commercial utility of the compounds according to the present invention.

### Biological Assay 1.0: Akt1 kinase assay

Akt1 inhibitory activity of compounds of the present invention may be quantified employing the Akt1 TR-FRET assay as described in the following paragraphs.

His-tagged human recombinant kinase full-length Akt1 expressed in insect cells was purchased form Invitrogen (part number PV 3599). As substrate for the kinase reaction the biotinylated peptide biotin-Ahx-KKLNRTLSFAEPG (C-terminus in amide form) was used which can be purchased e.g. from the company Biosynthan GmbH (Berlin-Buch, Germany).
For the assay 50 nl of a 100fold concentrated solution of the test compound in DMSO was pipetted into a black low volume 384well microtiter plate (Greiner Bio-One, Frickenhausen, Germany), 2 µl of a solution of Akt1 in assay buffer [50 mM TRIS/HCl pH 7.5, 5 mM MgCl₂, 1 mM dithiothreitol, 0.02% (v/v) Triton X-100 (Sigma)] were added and the mixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 16.7 µM => final conc. in the 5 µl assay volume is 10 µM) and substrate (1.67 µM => final conc. in the 5 µl assay volume is 1 µM) in assay buffer and the resulting mixture was incubated for a reaction time of 60 min at 22°C. The concentration of Akt1 in the assay was adjusted depending of the activity of the enzyme lot and was chosen appropriate to have the assay in the linear range, typical enzyme concentrations were in the range of about 0.05 ng/µl (final conc. in the 5 µl assay volume).
The reaction was stopped by the addition of 5 µl of a solution of HTRF detection reagents (200 nM streptavidine-XL665 [Cisbio] and 1.5 nM anti-phosho-Serine antibody [Millipore, cat. # 35-001] and 0.75 nM LANCE Eu-W 1024 labeled anti-mouse IgG antibody [Perkin Elmer]) in an aqueous EDTA-solution (100 mM EDTA, 0.1 % (w/v) bovine serum albumin in 50 mM HEPES/NaOH pH 7.5).
The resulting mixture was incubated 1 h at 22°C to allow the binding of the biotinylated phosphorylated peptide to the streptavidine-XL665 and the antibodies. Subsequently the amount of phosphorylated substrate was evaluated by measurement of the resonance energy transfer from the anti-mouse-IgG-Eu-Chelate to the streptavidine-XL665. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Normally test compound were tested on the same microtiter plate at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM, dilution series prepared before the assay at the level of the 100fold conc. stock solutions by serial 1:3 dilutions) in duplicate values for each concentration and IC₅₀ values were calculated by a 4 parameter fit using an inhouse software.

### Biological Assay 2.0: Akt2 kinase assay

Akt2 inhibitory activity of compounds of the present invention was quantified employing the Akt2 TR-FRET assay as described in the following paragraphs.

His-tagged human recombinant kinase full-length Akt2 expressed in insect cells and activated by PDK1 was purchased form Invitrogen (part number PV 3975). As substrate for the kinase reaction the biotinylated peptide biotin-Ahx-KKLNRTLSFAEPG (C-terminus in amide form) was used which can be purchased e.g. from the company Biosynthan GmbH (Berlin-Buch, Germany).
For the assay 50 nl of a 100fold concentrated solution of the test compound in DMSO was pipetted into a black low volume 384well microtiter plate (Greiner Bio-One, Frickenhausen, Germany), 2 µl of a solution of Akt2 in assay buffer [50 mM TRIS/HCl pH 7.5, 5 mM MgCl₂, 1 mM dithiothreitol, 0.02% (v/v) Triton X-100 (Sigma)] were added and the mixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 16.7 µM => final conc. in the 5 µl assay volume is 10 µM) and substrate (1.67 µM => final conc. in the 5 µl assay volume is 1 µM) in assay buffer and the resulting mixture was incubated for a reaction time of 60 min at 22°C. The concentration of Akt2 in the assay was adjusted depending of the activity of the enzyme lot and was chosen appropriate to have the assay in the linear range, typical enzyme concentrations were in the range of about 0.2 ng/µl (final conc. in the 5 µl assay volume).
The reaction was stopped by the addition of 5 µl of a solution of HTRF detection reagents (200 nM streptavidine-XL665 [Cisbio] and 1.5 nM anti-phosho-Serine antibody [Millipore, cat. # 35-001] and 0.75 nM LANCE Eu-W 1024 labeled anti-mouse IgG antibody [Perkin Elmer]) in an aqueous EDTA-solution (100 mM EDTA, 0.1 % (w/v) bovine serum albumin in 50 mM HEPES/NaOH pH 7.5).
The resulting mixture was incubated 1 h at 22°C to allow the binding of the biotinylated phosphorylated peptide to the streptavidine-XL665 and the antibodies. Subsequently the amount of phosphorylated substrate was evaluated by measurement of the resonance energy transfer from the anti-mouse-IgG-Eu-Chelate to the streptavidine-XL665. Therefore, the fluorescence emissions at 620 nm and 665 nm after excitation at 350 nm was measured in a TR-FRET reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Normally test compound were tested on the same microtiter plate at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM, dilution series prepared before the assay at the level of the 100fold conc. stock solutions by serial 1:3 dilutions) in duplicate values for each concentration and IC₅₀ values were calculated by a 4 parameter fit using an inhouse software.

Preferred compounds of the present invention show in either the Akt1 or Akt2 kinase assay: IC₅₀ < 5 µM, more preferably, IC₅₀ < 0.5 µM, even more preferably, IC₅₀ < 0.05 µM.

### Cellular Assays: p-PRAS40 and p-AKT Assay

The assessment of cellular AKT activities was conducted with HEK293-AKT and HEK293-PRAS40 cell lines. The cell lines express AKT or PRAS40 as fusions with green fluorescent protein (GFP, a suitable TR-FRET acceptor for the excited-state Tb fluorophore) respectively. The effects of AKT inhibitors on the phosphorylation state of the GFP-PRAS40 or the GFP-AKT fusion proteins were detected in cell lysates using LanthaScreen™ Tb-anti-AKT(S473) and Tb-anti-pPRAS40 [pThr246] antibodies.

### Biological Assay 3.1: p-PRAS40 Assay

HEK293-PRAS40 cells (PerkinElmer # 6007688) were plated at 20000 cells/well in 384 well MTP. Following overnight incubation at 37°C, testing compounds diluted into growth medium were added to the cells. After 1 hour treatment, cells were stimulated with insulin (Insulin #12585-014 Invitrogen) with a final concentration of 500 pM for 40 min. Thereafter, cells were lysed with a buffer containing 20mM Tris, pH 7.4, 5mM EDTA, 150mM NaCl, 1% NP-40, phosphatase/protease inhibitors, and 5 nM of Tb-anti-AKT. After 2 hours incubation at room temperature, the TR-FRET value was detected using a PHERAstar plate reader (BMG LABTECH) and 520/490nm emission ratio is used for IC50 calculation.

### Biological Assay 3.2: p-AKT Assay

The phospho-AKT assay was conducted in analogy to the p-PRAS40 protocol, except that the cell line is HEK293-AKT, and the stimulation is 5 ng/mL IGF-1.

Preferred compounds of the present invention show, in either the p-PRAS40 or p-AKT assay: IC₅₀ < 10µM, more preferably, IC₅₀ < 1 µM.

### Biological Assay 4.0: Tumor cell proliferation assay

Compounds were tested in a cell-based assay that measures the capacity of the compounds to inhibit tumor cell proliferation following a 72 h drug exposure. Cell viability is determined using the Cell Titer-Glo luminescent cell viability kit from Promega (Cat. #G7573). Cells were plated at 1000-5000 cells/well (depending on cell lines) in 100 mL growth medium on black/clear bottom plates (Fisher #07-200-565). For each cell line assayed, cells were plated onto a separate plate for determination of luminescence at the t = 0 hours and t = 72 hour time points. Following overnight incubation at 37 °C, luminescence values for the t = 0 samples were determined by adding 100 µL of Cell Titer-Glo solution per well, transferring the plates to an orbital shaker for 10 minutes at room temperature, and then reading the plates on a Wallac Victor2 1420 Multi-label HTS Counter using the luminometry window (maximum light detection is measured at 428 nM). Dose plates for t = 72 hour time points were treated with compounds diluted into growth medium in a final volume of 50 µL. Cells were then incubated for 72 hours at 37 oC. Luminescence values for the t = 72 hour samples were determined by adding 150 µL of Promega CellTiter-Glo solution, placing the cells on a shaker for 10 minutes at room temperature, and then reading the luminescence using a Victor luminometer. Data were processed using a template specific for the luciferase assay. Briefly, t = 0 values were subtracted from those determined for the t = 72 hour time points, for both the treated and untreated samples. Percent differences in luminescence between drug treated and controls were used to determine the percent inhibition of growth.

The following further cellular assays can be used to further illustrate the commercial utility of the compounds according to the present invention.

### Biological Assay 5.0: Cellular PI3K / Akt pathway assay

In order to study the cellular activity of the compounds according to the present invention, an Enzyme Linked Immunosorbent Assay (ELISA)-based assay may be used to investigate the inhibitory effect on Akt phosphorylation. The assay is based on a Sandwich ELISA kit (PathScan^{™} Phospho-Akt1 (Ser473); Cell Signaling, USA; #7160).
The ELISA Kit detects endogenous levels of phosphorylated Akt protein. A phospho-Akt (Ser473) antibody (Cell Signaling, USA; #9271) has been coated onto the microwells. After incubation with cell lysates, the coated antibody captures the phosphorylated Akt protein. Following extensive washing, Akt1 monoclonal antibody (Cell Signaling, USA; #2967) is added to detect the captured phospho-Akt1 protein. HRP-linked anti-mouse antibody (HRP: horseradish peroxidase; Cell Signaling, USA; #7076) is then used to recognize the bound detection antibody. HRP substrate (= 3,3',5,5'-tetramethylbenzidine (TMB); Cell Signaling, USA; #7160) is added to develop colour. The magnitude of optical density for this developed color is proportional to the quantity of phosphorylated Akt protein. MCF7 cells (ATCC HTB-22) are seeded into 96 well fate bottom plates at a density of 10000 cells/well. 24 hours after seeding, the cells are serum starved using low-serum medium (IMEM media including 0,1% charcoal treated FCS (FCS: fetal calf serum)). After 24 hours 1 µl each of the compound dilutions (test compounds were dissolved as 10 mM solutions in dimethylsulfoxide (DMSO) and subsequently diluted) are added into each well of the 96 well plates and incubated for 48h at 37°C in a humidified athmosphere containing 5% CO₂. To stimulate Akt phosphorylation, β-Heregulin (20ng/mL β-HRG) is added in parallel to the compounds. Wells containing unstimulated control cells (no β-Heregulin stimulation) are incubated with or without the diluted compound. Wells containing untreated control cells (no compound) are filled with medium containing 0.5% v:v DMSO and are or are not stimulated with β-Heregulin.
Cells are harvested and lysed with brief sonification in 1 x cell lysis buffer (20mM Tris (pH7.5), 150 mM NaCl, 1 mM ethylene diaminetetraacetate (EDTA), 1 mM ethylene glycolbis(2-aminoethyl)-N,N,N',N'-tetraacetic acid (EGTA), 1vol% Triton X-100, 2.5mM sodium pyrophosphate, 1 mM β-glycerolphosphate, 1 mM Na₃VO₄, 1 µg/mL leupeptin). The lysate is centrifuged for 10 min. at 4°C and the supernatant is transferred to a new tube. 100 µl of sample diluent (0.1vol% Tween-20, 0.1vol% sodium azide in phosphate buffered saline (PBS)) are added to a microcentrifuge tube and 100 µl of cell lysate are transferred into the tube and vortexed. 100 µl of each diluted cell lysate are added to the appropriate ELISA well, and incubated overnight at 4°C. The plates are washed 4 times with 1 x wash buffer (1vol% tween-20, 0.33vol% thymol, in PBS). Next 100 µl of detection antibody (Akt1 (2H10) monoclonal detection antibody; Cell Signaling, USA; #2967) are added to each well and incubation continued for 1 h at 37°C. The washing procedure is repeated between each step. 100µl of secondary antibody (anti-mouse IgG HRP-linked antibody; Cell Signaling, USA; #7076) are added to each well and incubated for 30 min. at 37°C. Than, 100µl of TMB substrate (0.05% 3,3',5,5' tetramethylbenzidine, 0.1% hydrogen peroxide, complex polypeptides in a buffered solution; Cell Signaling, USA; #7160) are added to each well and incubated for 30 min. at 25°C. Finally 100 µl of STOP solution (0.05vol% α and β unsaturated carbonyl compound) are added to each well and the plate are shaked gently. The absorbance is measured at λ =450 nm (Wallac Victor2; Perkin Elmer, USA) within 30 min. after adding the STOP solution. The analysis of the data is performed using a statistical program (Excel; Microsoft, USA).

### Biological Assay 6.0: Cellular pGSK3 assay:

In order to study the cellular activity of the compounds according to the present invention, an ELISA-based assay may be used for the phosphorylated protein glycogen synthetase kinase 3 (GSK3). The assay is based on a solid phase sandwich ELISA that detects endogenous levels of phosphorylated GSK3 using a phospho-GSK3 (Ser9) specific antibody (BioSource International, Inc.; Catalog #KHO0461). After incubation with cell lysates, the coated antibody captures the phosphorylated GSK3 protein. Following extensive washing, GSK3 polyclonal antibody is added to detect the captured phospho-GSK3 protein. Secondary antibody (anti-rabbit IgG-HRP) is then used to recognize the bound detection antibody. After the second incubation and washing to remove all the excess anti-rabbit IgG-HRP, a substrate solution is added, which is acted upon by the bound enzyme to produce color. The intensity of this colored product is directly proportional to the concentration of GSK-3β [pS9] present in the original specimen. MCF7 cells (ATCC HTB-22) were seeded into 96 well fate bottom plates at a density of 10000 cells/well. After 24 h 1 µl each of the compound dilutions (test compounds were dissolved as 10 mM solutions in dimethylsulfoxide (DMSO) and subsequently diluted) were added into each well of the 96 well plates and incubated for 48h at 37°C in a humidified athmosphere containing 5% CO₂. Cells were harvested and lysed in cell extraction buffer (10 mM Tris, pH 7.4, 100 mM NaCl, 1 mM EDTA, 1 mM EGTA, 1 mM NaF, 20 mM Na₄P₂O₇, 2 mM Na₃VO₄, 1% Triton X-100, 10vol% glycerol, 0.1vol% SDS, 0.5vol% deoxycholate, 1 mM phenylmethylsulfonylfluorid (PMSF)). The lysate were centrifuged for 10 min. at 4°C and the supernatant were transferred to a new tube. 50 µl of sample diluent (standard diluent buffer, Biosource) were added and 100 µl of cell lysate transferred into the tube and vortexed. 100 µl of each diluted cell lysate were added to the appropriate ELISA well plate and incubated for 3h at room temperature. The plates were washed 4 times with 1 x wash buffer (Biosource). 50 µl of detection antibody (GSK3 (Ser9) detection antibody; BioSource) were added to each well and incubated for 30 min. at room temperature. The washing procedure was repeated between each step. 100µl of HRP-linked secondary antibody (anti-mouse IgG HRP-linked antibody) were added to each well and incubated for 30 min. at room temperature. 100µl of TMB substrate (0.05vol% 3,3',5,5' tetramethylbenzidine, 0.1vol% hydrogen peroxide, complex polypeptides in a buffered solution; Biosource) were added to each well and incubated for 30 min. at room temperature. Finally 100 µl of Stop solution (0.05vol% α and β unsaturated carbonyl compound) were added to each well and the plate were shaked gently for a few seconds. The absorbance was measured at λ = 450 nm (Wallac Victor2; Perkin Elmer, USA) within 30 min. after adding the stop solution.
The analysis of the data was performed using a statistical program (Excel; Microsoft, USA) and the IC50 of pGSK3 inhibition was determined.

### Biological Assay 7.0: Cellular proliferation / Cytotoxicity assay:

The anti-proliferative activity of the compounds as described herein, may be evaluated using the OvCAR3, HCT116 and A549 cell lines and the Alamar Blue (Resazurin) cell viability assay (O'Brien et al. Eur J Biochem 267, 5421-5426, 2000). Resazurin is reduced to the fluorescent resorufin by cellular dehydrogenase activity, correlating with viable, proliferating cells. Test compounds are dissolved as 10 mM solutions in DMSO and subsequently diluted. Cells like HCT116 or A549 cells were seeded into 96 well flat bottom plates at a density of 10000 cells/well (OvCAR3 cells), 1000 cells/well (HCT116 cells) or 2000 cells/well (A549 cells) in a volume of 200 µl/well. 24 hours after seeding, 1 µl each of the compound dilutions are added into each well of the 96 well plates. Each compound dilution is tested as at least as duplicates. Wells containing untreated control cells were filled with 200 µl DMEM (Dulbecco's Modified Eagle Medium) containing 0.5vol% v:v DMSO. The cells are then incubated with the substances for 72h at 37°C in a humidified atmosphere containing 5vol% CO2. To determine the viability of the cells, 20 µl of a Resazurin solution (90mg /l) are added. After 4h incubation at 37°C, the fluorescence is measured by extinction at λ= 544 nm and an emission of λ= 590 nm (Wallac Victor2; Perkin Elmer, USA). For the calculation of the cell viability, the emission value from untreated cells is set as 100% viability and the fluorescence intensity of treated cells are set in relation to the values of untreated cells. Viabilities are expressed as % values. The corresponding IC50 values of the compounds for cytotoxic activity are determined from the concentration-effect curves by means of non-linear regression. The analysis of the data is performed using a biostatistical program (GraphPad Prism, USA).

### Biological Assay 8.0: Chemosensitization assay

The herein disclosed compounds may be evaluated for the ability to sensitize cancer cells towards apoptotic stimuli. Inhibitors of Akt are tested alone and in combination with chemotherapeutic and targeted cancer therapeutics to determine the effect on apoptosis induction.
Cancer cells are seeded in 96 well plates at concentrations ranging from 2x10³ to 1 x 10⁴ cells per well in their respective growth media. 48-72 hours later, the apoptosis assay are set up as follows:
For combination assays with a chemotherapeutic agent especially preferred topoisomerase inhibitors (such as doxorubicin, etoposide, camptothecin or mitoxantrone) or antimitotic agents / tubulin inhibitors (such as vincristine), compounds are added at respective concentrations indicated and plates incubated at 37°C in a CO₂ incubator for 18 hours. For standard combination assays utilizing treatment with chemotherapeutic agent are added at the same time at the respective concentrations indicated.
For combinations assays involving addition of targeted pro-apoptotic agents like the death receptor ligand TRAIL/Apo2L (Research Diagnostics) compounds are added for 1,5 hours prior to addition of TRAIL and plates incubated an additional 3 to 4 hours post TRAIL addition. In the case of the time course, plates are incubated for 2, 3, 4 and 6 hours with TRAIL ligand before ending the assay.
For both procedures, total final volumes do not exceed 250µl. At the end of the incubation time, the cells are pelleted by centrifugation (200 x g; 10 min. at rt) and the supernatant is discarded. The cells are resuspended and incubated using lysis buffer for 30 min. at rt (Cell Death Detection ELISA^{PLUS}, Roche, Cat. No.11774425001). After the centrifugation is repeated (200 x g; 10 min. at rt) an aliquot of the supernatant is transferred to a streptavidin-coated well of a microplate. Followed by the incubation (2h, rt) and binding of nucleosomes in the supernatant with, anti-histone antibody (biotin-labeled) and anti-DNA antibody (peroxidase-conjugated; Cell Death Detection ELISA^{PLUS} , Roche, Cat. No.11774425 001). The antibody-nucleosome complexes are bound to the microplate. The immobilized antibody-histone complexes are washed three times at rt to remove cell components that are not immunoreactive. The substrate solution (2,2'-AZINO-bis [3-ethylbenziazoline-6-sulfonic acid (ABTS); Cell Death Detection ELISA^{PLUS} , Roche, Cat. No. 11 774 425 001) is added and the samples were incubated for 15 min., rt. The amount of colored product is determined spectrophotometrically (absorbance at λ= 405 nm). Data are expressed as percent activity of control with cisplatin used as a positive control. Apoptosis induction by 50µM cisplatin is arbitrarily defined as 100 cisplatin units (100 CPU).

The following Tables give selected data for selected Examples of the present invention.

**Table 1**

| Example | IC₅₀ p-Akt (Biological Assay 5.0), µM |
|---|---|
| 1.0 | <0.05 |
| 2.0 | 0.008 |
| 3.0 | < 0.5 |
| 4.0 | < 0.5 |

**Table 2**

| Example | IC₅₀ Akt1 (Biological Assay 1.0), µM | IC₅₀ Akt2 (Biological Assay 2.0), µM |
|---|---|---|
| 1.0 | 0.004 | 0.053 |
| 2.0 | 0.008 | 0.042 |
| 5.0 | 0.003 | 0.027 |
| 6.0 | 0.003 | 0.081 |
| 7.0 | 0.012 | 0.334 |
| 8.0 | 0.014 | 0.414 |
| 9.0 | 0.018 | 0.185 |
| 10.0 | 0.007 | 0.038 |
| 11.0 | 0.029 | 0.445 |
| 11.1 | 0.019 | 0.098 |
| 12.0 | 0.008 | 0.050 |
| 13.0 | 0.016 | 0.132 |
| 14.0 | 0.007 | 0.016 |
| 15.0 | 0.022 | 0.222 |
| 16.0 | 0.012 | 0.052 |

## Claims

1. A compound of formula (I) 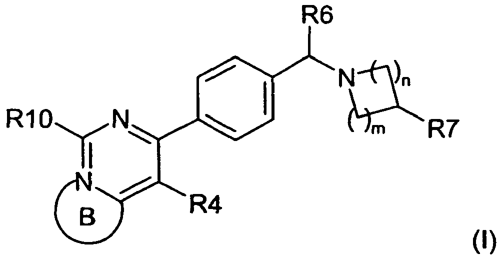 wherein ring B fused with the pyrimidine moiety is selected from 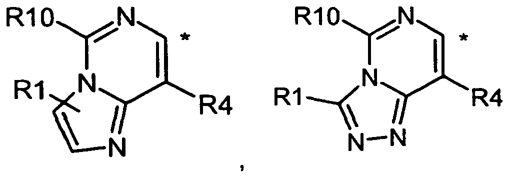
* marks the point of attachment
R1 is hydrogen, 1-4C-alkyl (optionally substituted by halogen, hydroxy, amino, mono-or di1-4C-alkylamino), halogen, trifluoromethyl, cyano, 3-7C-cycloalkyl, 2-4C-alkenyl, 2-4C-alkynyl, C(O)NR11R12, -C(O)OR2, or a monocyclic 5- or 6-membered heteroarylene comprising 1 nitrogen atom and optionally 1, 2 or 3 further heteroatoms independently selected from oxygen, nitrogen and sulfur,
R2 is hydrogen, 1-4C-alkyl (optionally substituted by halogen, hydroxyl, amino, mono- or di-1-4C-alkylamino) or 3-7C-cycloalkyl,
R4 is phenyl, thienyl, pyridinyl, oxazolyl or thiazolyl and wherein R4 is optionally substituted by R5,
R5 is 1-4C-alkyl, halogen or 1-4C-alkoxy or NR11R12,
R6 is hydrogen or 1-4C-alkyl,
n is 1 or 2,
m is 1 or 2,
R7 is -W-Y,
W is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1 , 2 or 3 further heteroatoms independently selected from oxygen, nitrogen and sulphur and wherein the bicyclic heteroarylene is optionally substituted by R8,
R8 hydrogen, is 1-4C-alkyl, 1-4C-haloalkyl, 3-7C-cycloalkyl, 1-4Calkoxy, halogen, cyano or NR11R12,
Y is hydrogen, aryl or a monocyclic 5- or 6-membered heteroaryl comprising 1 nitrogen atom and optionally 1,2 or 3 further heteroatoms independently selected from oxygen, nitrogen, sulphur and wherein the heteroaryl is optionally substituted by R9 and optionally further substituted by R9A,
R9 is 1-4C-alkyl, 1-4C-alkoxy, halogen, hydroxy, 1-4C-haloalkyl, NR11R12, cyano or C(O)NH2,
R9A is 1-4Calkyl or halogen
R10 is hydrogen or 1-4C-alkyl (optionally substituted by halogen, hydroxy, amino, mono-or di1-4C-alkylamino),
R11, R12 which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

2. A compound according to claim 1,
wherein
R1 is hydrogen, 1-4C-alkyl (optionally substituted by halogen, hydroxy, amino, mono-or di1-4C-alkylamino), halogen, trifluoromethyl, cyano, 3-7Ccycloalkyl, C(O)NR11R12, -C(O)OR2,
R2 is hydrogen or 1-4C-alkyl,
R4 is phenyl, thienyl, pyridinyl, oxazolyl or thiazolyl and wherein R4 is optionally substituted by R5,
R5 is 1-4C-alkyl, halogen or 1-4C-alkoxy or NR11R12,
R6 is hydrogen or 1-4C-alkyl,
n is 1 or 2,
m is 1 or 2,
R7 is -W-Y,
W is a monocyclic 5- or 6 membered heteroarylene comprising 1 nitrogen atom and optionally 1, 2 or 3 further heteroatoms independently selected from oxygen, nitrogen and sulphur, or a bicyclic 9-membered heteroarylene comprising 1 nitrogen atom and optionally 1, 2 or 3 further heteroatoms independently selected from oxygen, nitrogen and sulphur and wherein the bicyclic heteroarylene is optionally substituted by R8,
R8 hydrogen, is 1-4C-alkyl, 1-4C-haloalkyl, 3-7C-cycloalkyl, 1-4Calkoxy, halogen, cyano or NR11R12,
Y is hydrogen, aryl or a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9 and optionally further substituted by R9A,
R9 is 1-4C-alkyl, 1-4C-alkoxy, halogen, hydroxy, 1-4C-haloalkyl, NR11R12, cyano or C(O)NH2,
R9A is 1-4Calkyl or halogen
R10 is hydrogen or 1-4C-alkyl (optionally substituted by halogen, hydroxy, amino, mono-or di1-4C-alkylamino),
R11, R12 which can be same or different is hydrogen or 1-4C-alkyl,
or an N-oxide or a salt, a tautomer, or a stereoisomer of said compound, or a salt, of said N-oxide, tautomer or stereoisomer.

3. A compound according to claim1
wherein
R1 is hydrogen, 1-4C-alkyl (optionally substituted by halogen, hydroxy, amino, mono-or di1-4C-alkylamino), halogen, trifluoromethyl, cyano, 3-7C-cycloalkyl, C(O)NR11R12, -C(O)OR2,
R2 is hydrogen or 1-4C-alkyl,
R4 is phenyl, thienyl, pyridinyl, oxazolyl or thiazolyl,
R6 is hydrogen or 1-4C-alkyl,
n is 1 or 2,
m is 1 or 2,
R7 is -W-Y,
W is 1,2,4-triazolylene,
Y is a monocyclic 5- or 6-membered heteroaryl comprising 1 nitrogen atom and optionally 1, 2 or 3 further heteroatoms independently selected from oxygen, nitrogen, sulphur and wherein the heteroaryl is optionally substituted by R9 and optionally further substituted by R9A,
R9 is 1-4C-alkyl, 1-4C-alkoxy or halogen, hydroxy, 1-4C-haloalkyl, NR11R12, cyano or C(O)NH2,
R9A is 1-4Calkyl
R10 is hydrogen or 1-4C-alkyl,
R11, R12 which can be same or different is hydrogen or 1-4C-alkyl, (optionally substituted by halogen, hydroxyl, amino, mono- or di-1-4C-alkylamino) or 3-7C-cycloalkyl,
or an N-oxide or a salt, a tautomer, or a stereoisomer of said compound, or a salt of said N-oxide, tautomer or stereoisomer.

4. A compound according to claim 1, wherein
R1 is hydrogen or 1-4C-alkyl
R4 is phenyl,
R6 is hydrogen,
n is 1 or 2,
m is 1 or 2,
R7 is -W-Y,
W is 1,2,4-triazolylene,
Y is pyridin-2-yl, 2-pyrazinyl or 2-pyrimidinyl which is optionally substituted by R9 and optionally further substituted by R9A,
R9 is hydrogen, 1-4C-alkyl, halogen, 1-4C-haloalkyl
R9A is 1-4Calkyl
R10 is hydrogen or 1-4C-alkyl,
or an N-oxide or a salt, a tautomer, or a stereoisomer of said compound, or a salt, of said N-oxide, tautomer or stereoisomer.

5. A process for the manufacture of compounds of general formula (I), **characterized in that** an aldehyde or ketone of formula (III) can be reacted with an amine (II) or a salt thereof, to yield compounds of formula (I) 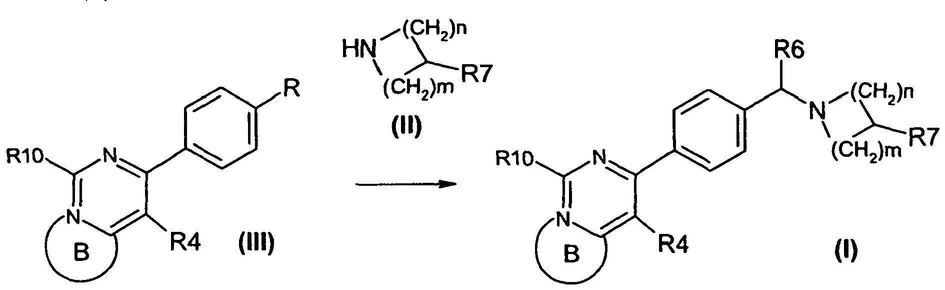 wherein B, R4, R6, R7, m, n and R10 have the meanings that are indicated in claim 1 and R has the meaning -C(O)R6.

6. A process for the manufacture of compounds of general formula (I), **characterized in that** a compound of formula (IIIa) can be reacted with an amine (II) or a salt thereof, to yield compounds of formula (I) 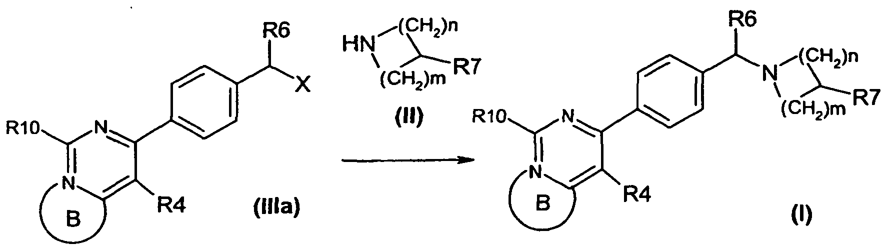 wherein B, R4, R6, R7, m, n and R10 have the meanings that are indicated in claim 1 and X is a suitable leaving group.

7. Compounds of general formula (III) and (IIIa), 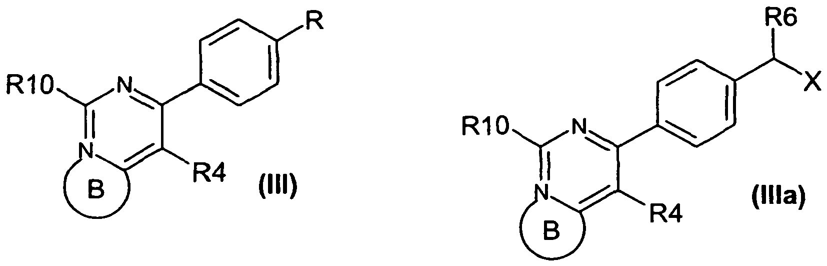 wherein B, R4, R6, and R10 have the meanings that are indicated in claim 1, R has the meanings -C(O)O(1-4C-alkyl), -C(O)R6, -CH(R6)OH or -CH2R6 and X is a suitable leaving group.

8. Use of the compounds of general formula (III) and (IIIa), according to one of the claims 5 or 6 for the manufacture of a compound of general formula (I), 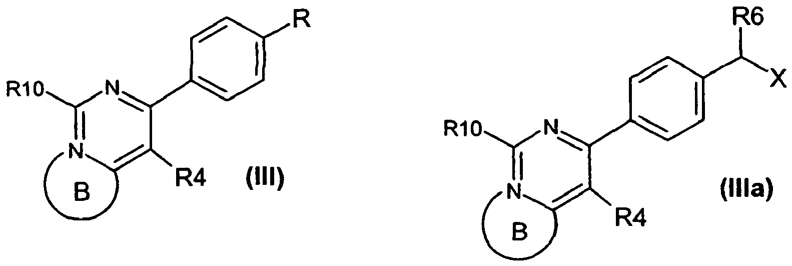 wherein B, R4 and R6 have the meanings that are indicated in claim 1 and R has the meanings -C(O)O(1-4C-alkyl), -C(O)R6, -CH(R6)OH or -CH2R6 and X is a suitable leaving group.

9. Use of the compounds of general formula (II) or a salt thereof, according to one of the claims 5 or 6 for the manufacture of a compound of general formula (I) 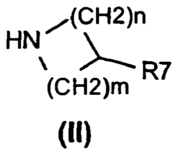 wherein R7, m and n have the meanings that are indicated in claim 1.

10. Compound of general formula (I) according to any of claims 1 to 4 or a pharmaceutical composition comprising a compound of general formula (I) according to any of claims 1 to 4, for use in the treatment or prophylaxis of diseases.

11. Compound for use according to claim 10 whereby the diseases are hyperproliferative diseases and/or disorders responsive to induction of apoptosis.

12. Compound for use according to claim 11 whereby the hyperproliferative diseases and/or disorders responsive to induction of apoptosis are cancer.

13. A pharmaceutical composition comprising at least one compound of general formula (I) according to any of claims 1 to 4, together with at least one pharmaceutically acceptable auxiliary.

14. A combination comprising one or more first active ingredients selected from a compound of general formula (I) according to any of claims 1 to 4, and one or more second active ingredients selected from chemotherapeutic anti-cancer agents and target-specific anti-cancer agents.

15. The use of a compound of general formula (I) according to any of claims 1 to 4 for the production of a pharmaceutical composition for the treatment of benign and/or malignant neoplasia.

16. The use of a compound of general formula (I) according to any of claims 1 to 4 for the production of a pharmaceutical composition for the treatment of cancer.

## Patentansprüche

1. Verbindungen der Formel (I) 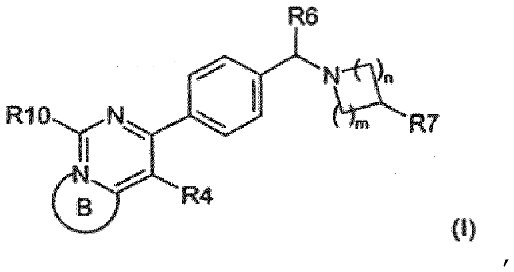 wobei Ring B mit der Pyrimidingruppierung ausgewählt aus 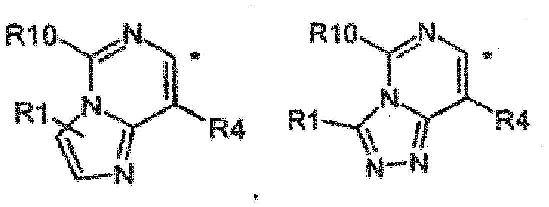 kondensiert ist,
* die Verknüpfungsstelle markiert,
R1 für Wasserstoff, 1-4C-Alkyl (gegebenenfalls substituiert durch Halogen, Hydroxy, Amino, Mono- oder Di-1-4C-alkylamino), Halogen, Trifluormethyl, Cyano, 3-7C-Cycloalkyl, 2-4C-Alkenyl, 2-4C-Alkinyl, C(O)NR11R12, -C(O)OR2 oder monocyclisches, 5- oder 6-gliedriges Heteroarylen mit einem Stickstoffatom und gegebenenfalls 1, 2 oder 3 weiteren, unabhängig voneinander aus Sauerstoff, Stickstoff und Schwefel ausgewählten Heteroatomen steht,
R2 für Wasserstoff, 1-4C-Alkyl (gegebenenfalls substituiert durch Halogen, Hydroxy, Amino, Mono- oder Di-1-4C-alkylamino) oder 3-7C-Cycloalkyl steht,
R4 für Phenyl, Thienyl, Pyridinyl, Oxazolyl oder Thiazolyl steht, wobei R4 gegebenenfalls durch R5 substituiert ist,
R5 für 1-4C-Alkyl, Halogen oder 1-4C-Alkoxy oder NR11R12 steht,
R6 für Wasserstoff oder 1-4C-Alkyl steht,
n für 1 oder 2 steht,
m für 1 oder 2 steht,
R7 für -W-Y steht,
W für monocyclisches, 5- oder 6-gliedriges Heteroarylen mit 1 Stickstoffatom und gegebenenfalls 1, 2 oder 3 weiteren, unabhängig voneinander aus Sauerstoff, Stickstoff und Schwefel ausgewählten Heteroatomen oder bicyclisches, 9-gliedriges Heteroarylen mit 1 Stickstoffatom und gegebenenfalls 1, 2 oder 3 weiteren, unabhängig voneinander aus Sauerstoff, Stickstoff und Schwefel ausgewählten Heteroatomen steht, wobei das bicyclische Heteroarylen gegebenenfalls durch R8 substituiert ist,
R8 für Wasserstoff, 1-4C-Alkyl, 1-4C-Halogenalkyl, 3-7C-Cycloalkyl, 1-4C-Alkoxy, Halogen, Cyano oder NR11R12 steht,
Y für Wasserstoff, Aryl oder monocyclisches, 5- oder 6-gliedriges Heteroaryl mit 1 Stickstoffatom und gegebenenfalls 1, 2 oder 3 weiteren, unabhängig voneinander aus Sauerstoff, Stickstoff und Schwefel ausgewählten Heteroatomen steht, wobei das Heteroaryl gegebenenfalls durch R9 substituiert ist und gegebenenfalls weiter durch R9A substituiert ist,
R9 für 1-4C-Alkyl, 1-4C-Alkoxy, Halogen, Hydroxy, 1-4C-Halogenalkyl, NR11R12, Cyano oder C(0)NH2 steht,
R9A für 1-4C-Alkyl oder Halogen steht,
R10 für Wasserstoff oder 1-4C-Alkyl (gegebenenfalls substituiert durch Halogen, Hydroxy, Amino, Mono- oder Di-1-4C-Alkylamino) steht,
R11, R12, die gleich oder verschieden sein können, für Wasserstoff oder 1-4C-Alkyl (gegebenenfalls substituiert durch Halogen, Hydroxy, Amino, Mono- oder Di-1-4C-alkylamino) oder 3-7C-Cycloalkyl stehen,
und die N-Oxide und die Salze, die Tautomere und die Stereoisomere dieser Verbindungen, und die Salze dieser N-Oxide, Tautomere und Stereoisomere.

2. Verbindungen nach Anspruch 1,
wobei
R1 für Wasserstoff, 1-4C-Alkyl (gegebenenfalls substituiert durch Halogen, Hydroxy, Amino, Mono- oder Di-1-4C-alkylamino), Halogen, Trifluormethyl, Cyano, 3-7C-Cycloalkyl, C(O)NR11R12 oder -C(O)OR2 steht,
R2 für Wasserstoff oder 1-4C-Alkyl steht,
R4 für Phenyl, Thienyl, Pyridinyl, Oxazolyl oder Thiazolyl steht, wobei R4 gegebenenfalls durch R5 substituiert ist,
R5 für 1-4C-Alkyl, Halogen oder 1-4C-Alkoxy oder NR11R12 steht,
R6 für Wasserstoff oder 1-4C-Alkyl steht,
n für 1 oder 2 steht,
m für 1 oder 2 steht,
R7 für -W-Y steht,
W für monocyclisches, 5- oder 6-gliedriges Hetero-arylen mit 1 Stickstoffatom und gegebenenfalls 1, 2 oder 3 weiteren, unabhängig voneinander aus Sauerstoff, Stickstoff und Schwefel ausgewählten Heteroatomen oder bicyclisches, 9-gliedriges Heteroarylen mit 1 Stickstoffatom und gegebenenfalls 1, 2 oder 3 weiteren, unabhängig voneinander aus Sauerstoff, Stickstoff und Schwefel ausgewählten Heteroatomen steht, wobei das bicyclische Heteroarylen gegebenenfalls durch R8 substituiert ist,
R8 für Wasserstoff, 1-4C-Alkyl, 1-4C-Halogenalkyl, 3-7C-Cycloalkyl, 1-4C-Alkoxy, Halogen, Cyano oder NR11R12 steht,
Y für Wasserstoff, Aryl oder monocyclisches, 5- oder 6-gliedriges Heteroaryl mit 1 Stickstoffatom und gegebenenfalls 1, 2 oder 3 weiteren, unabhängig voneinander aus Sauerstoff, Stickstoff und Schwefel ausgewählten Heteroatomen steht, wobei das Heteroaryl gegebenenfalls durch R9 substituiert ist und gegebenenfalls weiter durch R9A substituiert ist,
R9 für 1-4C-Alkyl, 1-4C-Alkoxy, Halogen, Hydroxy, 1-4C-Halogenalkyl, NR11R12, Cyano oder C(0)NH2 steht,
R9A für 1-4C-Alkyl oder Halogen steht,
R10 für Wasserstoff oder 1-4C-Alkyl (gegebenenfalls substituiert durch Halogen, Hydroxy, Amino, Mono- oder Di-1-4C-alkylamino) steht,
R11, R12, die gleich oder verschieden sein können, für Wasserstoff oder 1-4C-Alkyl stehen,
und die N-Oxide und die Salze, die Tautomere und die Stereoisomere dieser Verbindungen, und die Salze dieser N-Oxide, Tautomere und Stereoisomere.

3. Verbindungen nach Anspruch 1,
wobei
R1 für Wasserstoff, 1-4C-Alkyl (gegebenenfalls substituiert durch Halogen, Hydroxy, Amino, Mono- oder Di-1-4C-alkylamino), Halogen, Trifluormethyl, Cyano, 3-7C-Cycloalkyl, C(O)NR11R12 oder -C(O)OR2 steht,
R2 für Wasserstoff oder 1-4C-Alkyl steht,
R4 für Phenyl, Thienyl, Pyridinyl, Oxazolyl oder Thiazolyl steht,
R6 für Wasserstoff oder 1-4C-Alkyl steht,
n für 1 oder 2 steht,
m für 1 oder 2 steht,
R7 für -W-Y steht,
W für 1,2,4-Triazolylen steht,
Y für monocyclisches, 5- oder 6-gliedriges Heteroaryl mit 1 Stickstoffatom und gegebenenfalls 1, 2 oder 3 weiteren, unabhängig voneinander aus Sauerstoff, Stickstoff und Schwefel ausgewählten Heteroatomen steht, wobei das Heteroaryl gegebenenfalls durch R9 substituiert ist und gegebenenfalls weiter durch R9A substituiert ist,
R9 für 1-4C-Alkyl, 1-4C-Alkoxy, Halogen, Hydroxy, 1-4C-Halogenalkyl, NR11R12, Cyano oder C(0)NH2 steht,
R9A für 1-4C-Alkyl steht,
R10 für Wasserstoff oder 1-4C-Alkyl steht,
R11, R12, die gleich oder verschieden sein können, für Wasserstoff oder 1-4C-Alkyl (gegebenenfalls substituiert durch Halogen, Hydroxy, Amino, Mono- oder Di-1-4C-alkylamino) oder 3-7C-Cycloalkyl stehen,
und die N-Oxide und die Salze, die Tautomere und die Stereoisomere dieser Verbindungen, und die Salze dieser N-Oxide, Tautomere und Stereoisomere.

4. Verbindungen nach Anspruch 1, wobei
R1 für Wasserstoff oder 1-4C-Alkyl steht,
R4 für Phenyl steht,
R6 für Wasserstoff steht,
n für 1 oder 2 steht,
m für 1 oder 2 steht,
R7 für -W-Y steht,
W für 1,2,4-Triazolylen steht,
Y für Pyridin-2-yl, 2-Pyrazinyl oder 2-Pyrimidinyl, welches gegebenenfalls durch R9 substituiert ist und gegebenenfalls durch R9A substituiert ist, steht,
R9 für Wasserstoff, 1-4C-Alkyl, Halogen oder 1-4C-Halogenalkyl steht;
R9A für 1-4C-Alkyl steht,
R10 für Wasserstoff oder 1-4C-Alkyl steht,
und die N-Oxide und die Salze, die Tautomere und die Stereoisomere dieser Verbindungen, und die Salze dieser N-Oxide, Tautomere und Stereoisomere.

5. Verfahren zur Herstellung von Verbindungen der allgemeinen Formel (I), **dadurch gekennzeichnet, dass** man ein Aldehyd oder Keton der Formel (III) mit einem Amin (II) oder einem Salz davon zu Verbindungen der Formel (I) umsetzen kann 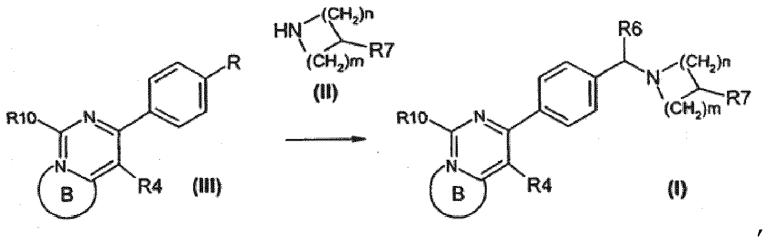 wobei B, R4, R6, R7, m, n und R10 die in Anspruch 1 angegebenen Bedeutungen haben und R für -C(O)R6 steht.

6. Verfahren zur Herstellung von Verbindungen der allgemeinen Formel (I), **dadurch gekennzeichnet, dass** man eine Verbindung der Formel (IIIa) mit einem Amin (II) oder einem Salz davon zu Verbindungen der Formel (I) umsetzen kann 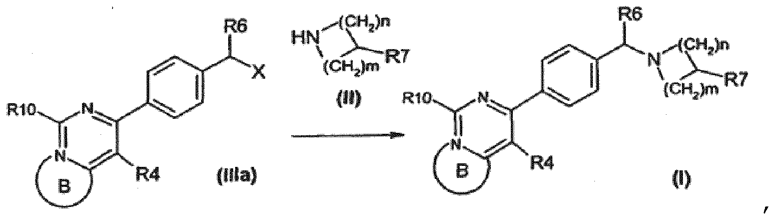 wobei B, R4, R6, R7, m, n und R10 die in Anspruch 1 angegebenen Bedeutungen haben und X für eine geeignete Abgangsgruppe steht.

7. Verbindungen der allgemeinen Formeln (III) und (IIIa) 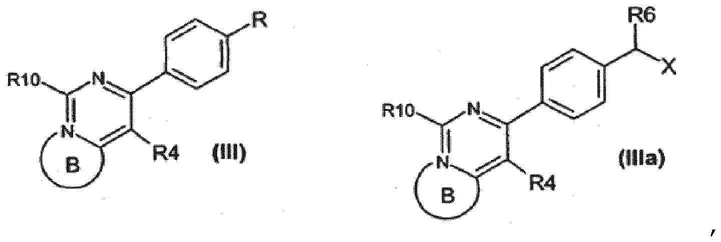 wobei B, R4, R6 und R10 die in Anspruch 1 angegebenen Bedeutungen haben, R für -C(O)O(1-4C-Alkyl), -C(O)R6, -CH(R6)OH oder -CH₂R6 steht und X für eine geeignete Abgangsgruppe steht.

8. Verwendung von Verbindungen der allgemeinen Formeln (III) und (IIIa) nach Anspruch 5 oder 6 zur Herstellung einer Verbindung der allgemeinen Formel (I) 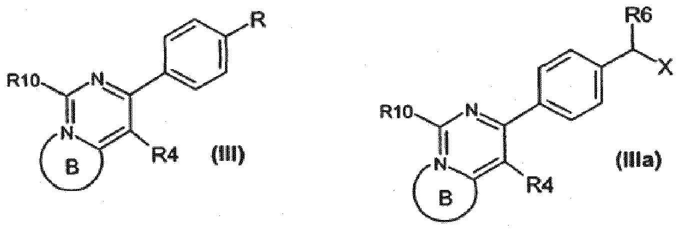 wobei B, R4 und R6 die in Anspruch 1 angegebenen Bedeutungen haben, R für -C(O)O(1-4C-Alkyl), -C(O)R6, -CH(R6)OH oder -CH₂R6 steht und X für eine geeignete Abgangsgruppe steht.

9. Verwendung der Verbindungen der allgemeinen Formel (II) und deren Salzen nach Anspruch 5 oder 6 zur Herstellung einer Verbindung der allgemeinen Formel (I) 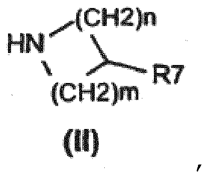 wobei R7, m und n die in Anspruch 1 angegebenen Bedeutungen haben.

10. Verbindungen der allgemeinen Formel (I) nach einem der Ansprüche 1 bis 4 oder eine eine Verbindung der allgemeinen Formel (I) nach einem der Ansprüche 1 bis 4 umfassende pharmazeutische Zusammensetzung zur Verwendung bei der Behandlung oder Prophylaxe von Krankheiten.

11. Verbindungen zur Verwendung nach Anspruch 10, wobei es sich bei den Krankheiten um hyperproliferative Krankheiten und/oder Erkrankungen, die auf die Induktion von Apoptose ansprechen, handelt.

12. Verbindungen zur Verwendung nach Anspruch 11, wobei es sich bei den hyperproliferativen Krankheiten und/oder Erkrankungen, die auf die Induktion von Apoptose ansprechen, um Krebs handelt.

13. Pharmazeutische Zusammensetzung, umfassend mindestens eine Verbindung der allgemeinen Formel (I) nach einem der Ansprüche 1 bis 4 zusammen mit mindestens einem pharmazeutisch unbedenklichen Hilfsmittel.

14. Kombination, umfassend einen oder mehrere erste Wirkstoffe ausgewählt aus Verbindungen der allgemeinen Formel (I) nach einem der Ansprüche 1 bis 4 sowie einen oder mehrere zweite Wirkstoffe ausgewählt aus chemotherapeutischen Antikrebsmitteln und zielspezifischen Antikrebsmitteln.

15. Verwendung einer Verbindung der allgemeinen Formel (I) nach einem der Ansprüche 1 bis 4 zur Herstellung einer pharmazeutischen Zusammensetzung zur Behandlung einer benignen und/oder malignen Neoplasie.

16. Verwendung einer Verbindung der allgemeinen Formel (I) nach einem der Ansprüche 1 bis 4 zur Herstellung einer pharmazeutischen Zusammensetzung zur Behandlung von Krebs.

## Revendications

1. Composé de formule (I) 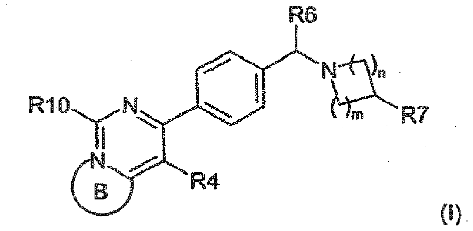 dans laquelle le cycle B condensé avec le motif pyrimidine est choisi parmi 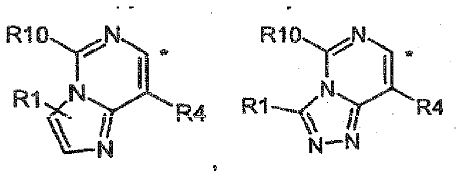
* indique le point de fixation
R1 est hydrogène, 1-4C-alkyle (éventuellement substitué par halogène, hydroxy, amino, mono- ou di-1-4C-alkylamino), halogène, trifluorométhyle, cyano, 3-7C-cycloalkyle, 2-4C-alcényle, 2-4C-alcynyle, C(O)NR11R12, -C(O)OR2, ou un hétéroarylène monocyclique à 5 ou 6 chaînons comprenant 1 atome d'azote et éventuellement 1, 2 ou 3 autres hétéroatomes choisis indépendamment parmi oxygène, azote et soufre,
R2 est hydrogène, 1-4C-alkyle (éventuellement substitué par halogène, hydroxy, amino, mono- ou di-1-4C-alkylamino) ou 3-7C-cycloalkyle,
R4 est phényle, thiényle, pyridinyle, oxazolyle ou thiazolyle et où R4 est éventuellement substitué par R5,
R5 est 1-4C-alkyle, halogène ou 1-4C-alcoxy ou NR11R12,
R6 est hydrogène ou 1-4C-alkyle,
n est 1 ou 2,
m est 1 ou 2,
R7 est -W-Y,
W est un hétéroarylène monocyclique à 5 ou 6 chaînons comprenant 1 atome d'azote et éventuellement 1, 2 ou 3 autres hétéroatomes choisis indépendamment parmi oxygène, azote et soufre, ou un hétéroarylène bicyclique à 9 chaînons comprenant 1 atome d'azote et éventuellement 1, 2 ou 3 autres hétéroatomes choisis indépendamment parmi oxygène, azote et soufre et où l'hétéroarylène bicyclique est éventuellement substitué par R8,
R8 est hydrogène, 1-4C-alkyle, 1-4C-halogénoalkyle, 3-7C-cycloalkyle, 1-4C-alcoxy, halogène, cyano ou NR11R12,
Y est hydrogène, aryle ou un hétéroaryle monocyclique à 5 ou 6 chaînons comprenant 1 atome d'azote et éventuellement 1, 2 ou 3 autres hétéroatomes choisis indépendamment parmi oxygène, azote, soufre et où l'hétéroaryle est éventuellement substitué par R9 et éventuellement substitué en outre par R9A,
R9 est 1-4C-alkyle, 1-4C-alcoxy, halogène, hydroxy, 1-4C-halogénoalkyle, NR11R12, cyano ou C(O)NH2,
R9A est 1-4C-alkyle ou halogène
R10 est hydrogène ou 1-4C-alkyle (éventuellement substitué par halogène, hydroxy, amino, mono- ou di-1-4C-akylamino),
R11, R12 qui peuvent être identiques ou différents sont hydrogène ou 1-4C-alkyle, (éventuellement substitué par halogène, hydroxy, amino, mono- ou di-1-4C-alkylamino) ou 3-7C-cycloalkyle,
ou un N-oxyde ou un sel, un tautomère, ou un stéréoisomère dudit composé, ou un sel dudit N-oxyde, tautomère ou stéréoisomère.

2. Composé selon la revendication 1, **caractérisé en ce que**
R1 est hydrogène, 1-4C-alkyle (éventuellement substitué par halogène, hydroxy, amino, mono- ou di-1-4C-alkylamino), halogène, trifluorométhyle, cyano, 3-7C-cycloalkyle, C(O)NR11R12, -C(O)OR2,
R2 est hydrogène ou 1-4C-alkyle,
R4 est phényle, thiényle, pyridinyle, oxazolyle ou thiazolyle et où R4 est éventuellement substitué par R5,
R5 est 1-4C-alkyle, halogène ou 1-4C-alcoxy ou NR11R12,
R6 est hydrogène ou 1-4C-alkyle,
n est 1 ou 2,
m est 1 ou 2,
R7 est -W-Y,
W est un hétéroarylène monocyclique à 5 ou 6 chaînons comprenant 1 atome d'azote et éventuellement 1, 2 ou 3 autres hétéroatomes choisis indépendamment parmi oxygène, azote et soufre, ou un hétéroarylène bicyclique à 9 chaînons comprenant 1 atome d'azote et éventuellement 1, 2 ou 3 autres hétéroatomes choisis indépendamment parmi oxygène, azote et soufre et où l'hétéroarylène bicyclique est éventuellement substitué par R8,
R8 est hydrogène, 1-4C-alkyle, 1-4C-halogénoalkyle, 3-7C-cycloalkyle, 1-4C-alcoxy, halogène, cyano ou NR11R12,
Y est hydrogène, aryle ou un hétéroaryle monocyclique à 5 ou 6 chaînons comprenant 1 atome d'azote et éventuellement 1, 2 ou 3 autres hétéroatomes choisis indépendamment parmi oxygène, azote, soufre et où l'hétéroaryle est éventuellement substitué par R9 et éventuellement substitué en outre par R9A,
R9 est 1-4C-alkyle, 1-4C-alcoxy, halogène, hydroxy, 1-4C-halogénoalkyle, NR11R12, cyano ou C(O)NH2,
R9A est 1-4C-alkyle ou halogène
R10 est hydrogène ou 1-4C-alkyle (éventuellement substitué par halogène, hydroxy, amino, mono- ou di-1-4C-akylamino),
R11, R12 qui peuvent être identiques ou différents sont hydrogène ou 1-4C-alkyle,
ou un N-oxyde ou un sel, un tautomère, ou un stéréoisomère dudit composé, ou un sel dudit N-oxyde, tautomère ou stéréoisomère.

3. Composé selon la revendication 1, **caractérisé en ce que**
R1 est hydrogène, 1-4C-alkyle (éventuellement substitué par halogène, hydroxy, amino, mono- ou di-1-4C-alkylamino), halogène, trifluorométhyle, cyano, 3-7C-cycloalkyle, C(O)NR11R12, -C(O)OR2,
R2 est hydrogène ou 1-4C-alkyle,
R4 est phényle, thiényle, pyridinyle, oxazolyle ou thiazolyle,
R6 est hydrogène ou 1-4C-alkyle,
n est 1 ou 2,
m est 1 ou 2,
R7 est -W-Y,
W est 1,2,4-triazolylène,
Y est un hétéroaryle monocyclique à 5 ou 6 chaînons comprenant 1 atome d'azote et éventuellement 1, 2 ou 3 autres hétéroatomes choisis indépendamment parmi oxygène, azote, soufre et où l'hétéroaryle est éventuellement substitué par R9 et éventuellement substitué en outre par R9A,
R9 est 1-4C-alkyle, 1-4C-alcoxy ou halogène, hydroxy, 1-4C-halogénoalkyle, NR11R12, cyano ou C(O)NH2,
R9A est 1-4C-alkyle
R10 est hydrogène ou 1-4C-alkyle,
R11, R12 qui peuvent être identiques ou différents sont hydrogène ou 1-4C-alkyle, (éventuellement substitué par halogène, hydroxy, amino, mono- ou di-1-4C-alkylamino) ou 3-7Ccycloalkyle,
ou un N-oxyde ou un sel, un tautomère, ou un stéréoisomère dudit composé, ou un sel dudit N-oxyde, tautomère ou stéréoisomère.

4. Composé selon la revendication 1, **caractérisé en ce que**
R1 est hydrogène ou 1-4C-alkyle,
R2 est hydrogène ou 1-4C-alkyle,
R4 est phényle,
R6 est hydrogène,
n est 1 ou 2,
m est 1 ou 2,
R7 est -W-Y,
W est 1,2,4-triazolylène,
Y est pyridin-2-yle, 2-pyrazinyle ou 2-pyrimidinyle qui est éventuellement substitué par R9 et éventuellement substitué en outre par R9A,
R9 est hydrogène, 1-4C-alkyle, 1-4C-halogénoalkyle,
R9A est 1-4C-alkyle
R10 est hydrogène ou 1-4C-alkyle,
ou un N-oxyde ou un sel, un tautomère, ou un stéréoisomère dudit composé, ou un sel dudit N-oxyde, tautomère ou stéréoisomère.

5. Procédé de fabrication de composés de formule générale (I), **caractérisé en ce qu'**un aldéhyde ou une cétone de formule (III) peut être mis(e) en réaction avec une amine (II) ou un sel de celle-ci, pour donner des composés de formule (I) 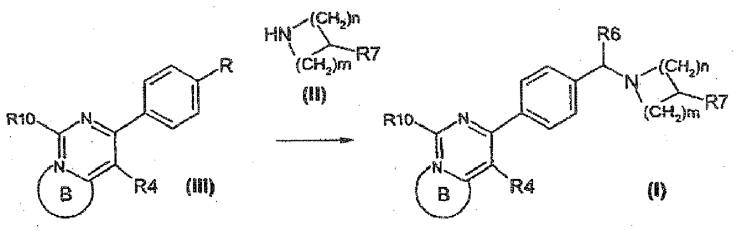 dans lesquelles B, R4, R6, R7, m, n et R10 ont les significations qui sont indiquées dans la revendication 1 et R a la signification -C(O)R6.

6. Procédé de fabrication de composés de formule générale (I), **caractérisé en ce qu'**un composé de formule (IIIa) peut être mis en réaction avec une amine (II) ou un sel de celle-ci, pour donner des composés de formule (I) 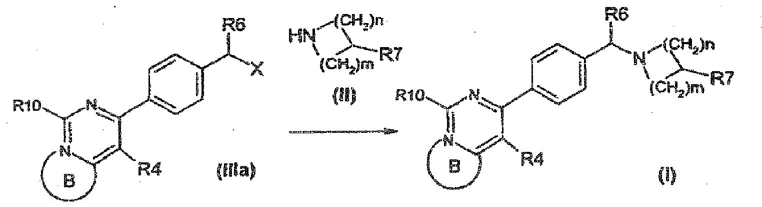 dans lesquelles B, R4, R6, R7, m, n et R10 ont les significations qui sont indiquées dans la revendication 1 et X est un groupement partant convenable.

7. Composés de formules générales (III) et (IIIa), 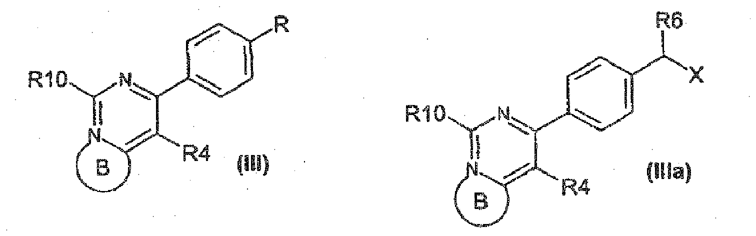 dans lesquelles B, R4, R6 et R10 ont les significations qui sont indiquées dans la revendication 1, R a les significations -C(O)O(1-4C-alkyl), -C(O)R6, -CH(R6)OH ou -CH2R6 et X est un groupement partant convenable.

8. Utilisation des composés de formules générales (III) et (IIIa), selon l'une des revendications 5 ou 6, pour la fabrication d'un composé de formule générale (I), 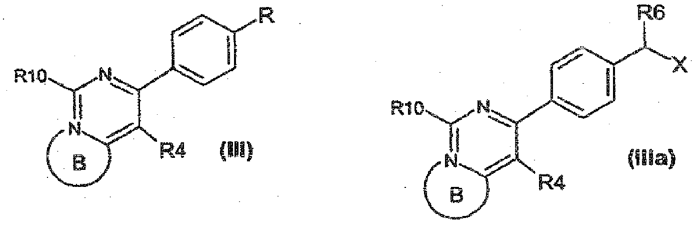 dans lesquelles B, R4, R6 et R10 ont les significations qui sont indiquées dans la revendication 1, R a les significations -C(O)O(1-4C-alkyl), -C(O)R6, -CH(R6)OH ou -CH2R6 et X est un groupement partant convenable.

9. Utilisation des composés de formule générale (II) ou d'un sel de ceux-ci, selon l'une des revendications 5 ou 6, pour la fabrication d'un composé de formule générale (I) 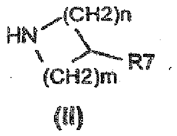 dans laquelle R7, m et n ont les significations qui sont indiquées dans la revendication 1.

10. Composé de formule générale (I) selon l'une quelconque des revendications 1 à 4 ou composition pharmaceutique comprenant un composé de formule générale (I) selon l'une quelconque des revendications 1 à 4, destiné(e) à être utilisé(e) dans le traitement ou la prophylaxie de maladies.

11. Composé destiné à être utilisé selon la revendication 10, les maladies étant des maladies et/ou troubles hyperprolifératifs présentant une réponse à l'induction d'apoptose.

12. Composé destiné à être utilisé selon la revendication 11, les maladies et/ou troubles hyperprolifératifs présentant une réponse à l'induction d'apoptose étant le cancer.

13. Composition pharmaceutique comprenant au moins un composé de formule générale (I) selon l'une quelconque des revendications 1 à 4, conjointement avec au moins un auxiliaire pharmaceutiquement acceptable.

14. Combinaison comprenant un ou plusieurs premiers principes actifs choisis parmi un composé de formule générale (I) selon l'une quelconque des revendications 1 à 4, et un ou plusieurs deuxièmes principes actifs choisis parmi des agents chimiothérapeutiques anti-cancéreux et des agents anti-cancéreux spécifiques à la cible.

15. Utilisation d'un composé de formule générale (I) selon l'une quelconque des revendications 1 à 4 pour la production d'une composition pharmaceutique destinée au traitement de néoplasies bénignes et/ou malignes.

16. Utilisation d'un composé de formule générale (I) selon l'une quelconque des revendications 1 à 4, pour la production d'une composition pharmaceutique destinée au traitement du cancer.
